(19) Europäisches Patentamt
European Patent Office
Office européen des brevets

(11) EP 2 914 248 B2

(12) NEW EUROPEAN PATENT SPECIFICATION
After opposition procedure

(45) Date of publication and mention of the opposition decision:
**18.10.2023 Bulletin 2023/42**

(45) Mention of the grant of the patent:
**05.09.2018 Bulletin 2018/36**

(21) Application number: **13792149.0**

(22) Date of filing: **01.11.2013**

(51) International Patent Classification (IPC):
***A61K 9/20*** (2006.01) ***A61K 45/06*** (2006.01)
***A61K 31/443*** (2006.01) ***A61K 31/47*** (2006.01)

(52) Cooperative Patent Classification (CPC):
(C-Sets available)
**A61K 31/47; A61K 9/2027; A61K 9/2054; A61K 9/2077; A61K 31/443; A61P 1/00; A61P 1/16; A61P 1/18; A61P 3/12; A61P 11/00; A61P 43/00** (Cont.)

(86) International application number:
**PCT/US2013/067952**

(87) International publication number:
**WO 2014/071122 (08.05.2014 Gazette 2014/19)**

(54) **PHARMACEUTICAL COMPOSITIONS FOR THE TREATMENT OF CFTR MEDIATED DISEASES**

PHARMAZEUTISCHE ZUSAMMENSETZUNGEN ZUR BEHANDLUNG VON CFTR-VERMITTELTEN ERKRANKUNGEN

COMPOSITIONS PHARMACEUTIQUES POUR LE TRAITEMENT DE MALADIES MÉDIÉES PAR CFTR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **02.11.2012 US 201261721622 P**
**20.11.2012 US 201261728328 P**
**28.02.2013 US 201361770668 P**
**16.05.2013 US 201361824005 P**
**28.06.2013 US 201361840668 P**

(43) Date of publication of application:
**09.09.2015 Bulletin 2015/37**

(60) Divisional application:
**18192504.1 / 3 470 063**

(73) Proprietor: **Vertex Pharmaceuticals Incorporated**
**Boston, MA 02210 (US)**

(72) Inventors:
- **VERWIJS, Marinus, Jacobus**
  **Framingham, MA 01701 (US)**
- **KARKARE, Radhika**
  **Medford, MA 02155 (US)**
- **MOORE, Michael, Douglas**
  **Charlestown, MA 02129 (US)**

(74) Representative: **Carpmaels & Ransford LLP**
**One Southampton Row**
**London WC1B 5HA (GB)**

(56) References cited:
**WO-A1-2009/073757 WO-A1-2013/112804**
**WO-A2-2010/019239 WO-A2-2010/019239**
**US-A1- 2011 256 220**

- **BOYLE, MP ET AL.: "VX-809, an investigational CFTR corrector, in combination with VX-770, an investigational CFTR potentiator, in subjects with CF and homozygous for the F508del-CFTR mutation", PEDIATRIC PULMONOLOGY, vol. 46, no. S34, 1 October 2011 (2011-10-01), page 287, XP002719045, ISSN: 1099-0496**
- **"Study of VX-809 Alone and in Combination With VX-770 in Cystic Fibrosis (CF) Patients Homozygous for the F508del-CFTR Mutation", INTERNET CITATION, March 2011 (2011-03), pages 1-4, XP002680870, Retrieved from the Internet: URL:http://clinicaltrials.gov/archive/NCT01225211/2011_03_01 [retrieved on 2012-07-26]**
- **ROWE et al.: "Progress in cystic fibrosis and the CF Therapeutics Development Network", Thorax, vol. 67, no. 10, September 2019 (2019-09), pages 889-890,**

EP 2 914 248 B2

- **MERK et al.: "Neue Ansätze bei Mukoviszidose", Pharmazeutische Zeitung, 13 September 2011 (2011-09-13), Retrieved from the Internet: URL:https://www.pharmazeutische-zeitung.de /ausgabe-372011/neue-ansaetze-bei-mukovisz idose/**
- **VERTEX: "Data from Phase 2 Combination Study of VX-809 and Ivacaftor in People with Cystic Fibrosis Who Have the Most Common Genetic Mutation (F508del) Presented at North American Cystic Fibrosis Conference", Press-Release, 11 October 2012 (2012-10-11), pages 1-4,**


Remarks:

The file contains technical information submitted after the application was filed and not included in this specification

(52) Cooperative Patent Classification (CPC): (Cont.)

C-Sets

**A61K 31/443, A61K 2300/00;**
**A61K 31/47, A61K 2300/00**

## Description

TECHNICAL FIELD OF INVENTION

The invention is defined in the claims and relates to tablets comprising 3-(6-(1-(2,2-difluorobenzo[d][1,3]dioxol-5-yl) cyclopropanecarboxamido)-3-methylpyridin-2-yl)benzoic acid (Compound 1) Form I and a solid dispersion comprising substantially amorphous N-(5-hydroxy-2,4-ditert-butyl-phenyl)-4-oxo-1H-quinoline-3-carboxamide (Compound 2). The tablets can be used in methods of treatment. The invention also relates to methods of manufacturing tablets, and to kits comprising the tablets.

BACKGROUND

Cystic fibrosis (CF) is a recessive genetic disease that affects approximately 30,000 children and adults in the United States and approximately 30,000 children and adults in Europe. Despite progress in the treatment of CF, there is no cure.

In patients with CF, mutations in CFTR endogenously expressed in respiratory epithelia leads to reduced apical anion secretion causing an imbalance in ion and fluid transport. The resulting decrease in anion transport contributes to enhanced mucus accumulation in the lung and the accompanying microbial infections that ultimately cause death in CF patients. In addition to respiratory disease, CF patients typically suffer from gastrointestinal problems and pancreatic insufficiency that, if left untreated, results in death. In addition, the majority of males with cystic fibrosis are infertile and fertility is decreased among females with cystic fibrosis. In contrast to the severe effects of two copies of the CF associated gene, individuals with a single copy of the CF associated gene exhibit increased resistance to cholera and to dehydration resulting from diarrhea - perhaps explaining the relatively high frequency of the CF gene within the population.

Sequence analysis of the CFTR gene of CF chromosomes has revealed a variety of disease causing mutations (Cutting, G. R. et al. (1990) Nature 346:366-369; Dean, M. et al. (1990) Cell 61:863:870; and Kerem, B-S. et al. (1989) Science 245:1073-1080; Kerem, B-S et al. (1990) Proc. Natl. Acad. Sci. USA 87:8447-8451). To date, greater than 1000 disease causing mutations in the CF gene have been identified (http://www.genet.sickkids.on.ca/cftr/app). The most prevalent mutation is a deletion of phenylalanine at position 508 of the CFTR amino acid sequence, and is commonly referred to as ΔF508-CFTR. This mutation occurs in approximately 70% of the cases of cystic fibrosis and is associated with a severe disease.

The deletion of residue 508 in ΔF508-CFTR prevents the nascent protein from folding correctly. This results in the inability of the mutant protein to exit the ER, and traffic to the plasma membrane. As a result, the number of channels present in the membrane is far less than observed in cells expressing wild-type CFTR. In addition to impaired trafficking, the mutation results in defective channel gating. Together, the reduced number of channels in the membrane and the defective gating lead to reduced anion transport across epithelia leading to defective ion and fluid transport. (Quinton, P. M. (1990), FASEB J. 4: 2709-2727). Studies have shown, however, that the reduced numbers of ΔF508-CFTR in the membrane are functional, albeit less than wild-type CFTR. (Dalemans et al. (1991), Nature Lond. 354: 526-528; Denning et al., supra; Pasyk and Foskett (1995), J. Cell. Biochem. 270: 12347-50). In addition to ΔF508-CFTR, other disease causing mutations in CFTR that result in defective trafficking, synthesis, and/or channel gating could be up- or down-regulated to alter anion secretion and modify disease progression and/or severity.

Compound 1 in salt form is disclosed in International PCT Publication WO2007056341 and United States Patent No. 7,741,321 as an inducer of CFTR activity and thus as a useful treatment for CFTR-mediated diseases such as cystic fibrosis. Compound 1Form I, which is a substantially crystalline and salt-free form, is disclosed in International PCT Publication WO2009073757 and United States Patent No. 8,507,534. Compound 2 is disclosed in International PCT Publication WO2006002421 and United States Patent No. 7,495,103 as an inducer of CFTR activity and thus as useful treatment for CFTR-mediated diseases such as cystic fibrosis. A solid dispersion comprising substantially amorphous Compound 2 is disclosed in International PCT Publication WO2010019239 and United States Published Patent Application No. US20100074949.

Compounds which are CFTR potentiators, such as Compound 2, and compounds which are CFTR correctors, such as Compound 1, have been shown independently to have utility in the treatment of CFTR related diseases, such as cystic fibrosis.

Accordingly, there is a need for novel treatments of CFTR mediated diseases which involve CFTR corrector and potentiator compounds.

Particularly, there is a need for combination therapies to treat CFTR mediated diseases, such as cystic fibrosis, which include CFTR potentiator and corrector compounds.

More particularly, there is a need for combination therapies to treat CFTR mediated diseases, such as cystic fibrosis, which include CFTR potentiator compounds, such as substantially amorphous Compound 2, in combination with CFTR corrector compounds, such as Compound 1 Form I.

Compound 1 as part of a combination with Compound 2 has been granted a Breakthrough Therapy Designation from the Food and Drug Administration (FDA) for the treatment of cystic fibrosis, one of only two such grants at the time of the filing of this application (the other being for Compound 2). This demonstrates a significant unmet need for the effective treatment of the cause of cystic fibrosis over symptomatic treatments. Additionally, a common challenge for drugs approved by the FDA is the occasional lack of drug availability for patients in need thereof. Accordingly, a significant unmet need exists for the presently disclosed Compound 1 and Compound 2 formulations and processes for preparing them in a continuous and controlled manner.

Additionally, patient compliance with treatment schedules and dosage amounts is largely dependent on ease of drug administration. A pharmaceutical composition comprising fixed dosage amounts of a CFTR corrector and CFTR potentiator, wherein the solid forms of said corrector and potentiator are stable, is a significant breakthrough for the treatment of CFTR mediated diseases such as cystic fibrosis. WO 2010/019239 A2 discloses pharmaceutical compositions comprising a solid dispersion of N-[2,4-Bis(1,1-dimethylethyl)-5-hydroxyphenyl]-1,4-dihydro-4-oxoquinoline-3-carboxamide, methods of manufacturing these pharmaceutical compositions, and methods of administering these pharmaceutical compositions. WO 2009/073757 A1 discloses a substantially crystalline and free solid state form of 3-(6-(1-(2,2-difluorobenzo[D][1,3]dioxol-5-yl) cyclopropanecarboxamido)-3-methylpyridin-2-yl)benzoic acid (Form I), pharmaceutical compositions thereof, and methods of treatment therewith.

## SUMMARY

The invention features a tablet comprising 200 mg of 3-(6-(1-(2,2-difluorobenzo[d][1,3]dioxol-5-yl) cyclopropanecarboxamido)-3-methylpyridin-2-yl)benzoic acid (Compound 1) Form I, which has the structure below:

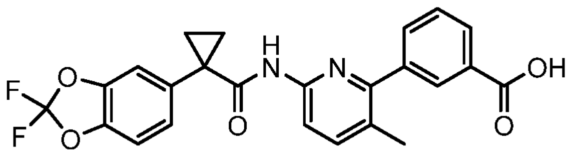

Compound 1; and

a solid dispersion of substantially amorphous N-(5-hydroxy-2,4-ditert-butyl-phenyl)-4-oxo-1H-quinoline-3-carboxamide (Compound 2), which has the structure below:

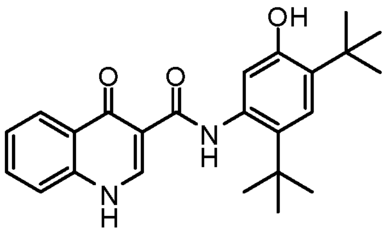

Compound 2;

wherein the substantially amorphous Compound 2 is present in the tablet in an amount of 125 mg, wherein Compound 1 Form I is characterized by one or more peaks at 15.4, 16.3, and 14.5 degrees in an X-ray powder diffraction pattern, wherein substantially amorphous Compound 2 has less than 15% crystallinity, and wherein the tablet comprises 25 to 50 percent by weight Compound 1 Form I, and 15 to 35 percent weight of a solid dispersion comprising Compound 2.

The invention also provides a tablet comprising: 100 mg of 3-(6-(1-(2,2-difluorobenzo[d][1,3]dioxol-5-yl) cyclopropanecarboxamido)-3-methylpyridin-2-yl)benzoic acid (Compound 1) Form I and a solid dispersion comprising substantially amorphous N-(5-hydroxy-2,4-ditert-butyl-phenyl)-4-oxo-1H-quinoline-3-carboxamide (Compound 2) and a polymer, wherein the substantially amorphous Compound 2 is present in the tablet in an amount of 125 mg, wherein Compound 1 Form I is characterized by one or more peaks at 15.4, 16.3, and 14.5 degrees in an X-ray powder diffraction pattern, wherein substantially amorphous Compound 2 has less than 15% crystallinity, and wherein the tablet has the following formulation:

| Component | mg/Tablet |
|---|---|
| Compound 1 Form I | 100 |

(continued)

| Component | mg/Tablet |
|---|---|
| Solid dispersion comprising substantially amorphous Compound 2 | 156 |
| Microcrystalline cellulose | 55 |
| Croscarmellose sodium | 7 |
| Polyvinylpyrrolidone | 11 |
| Sodium lauryl sulfate | 3 |
| Total Granules | 332 |
| Croscarmellose sodium | 18 |
| Microcrystalline cellulose | 53 |
| Magnesium stearate | 4 |
| Total Tablet | 407 |

referred to as **PC-XXIII**

In one embodiment, the tablet comprises:

a. Compound 1 Form I;

b. a solid dispersion comprising substantially amorphous Compound 2;

c. a filler;

d. a disintegrant;

e. a surfactant; and

f. a binder;

referred to as **PC-I.**

Disclosed herein are pharmaceutical compositions comprising 30 to 55 percent by weight Compound 1 Form I, and 10 to 45 percent by weight solid dispersion comprising substantially amorphous Compound 2.

In one embodiment, the filler is selected from cellulose, modified cellulose, sodium carboxymethyl cellulose, ethyl cellulose hydroxymethyl cellulose, hydroxypropylcellulose, cellulose acetate, microcrystalline cellulose, dibasic calcium phosphate, sucrose, lactose, corn starch, potato starch, or any combination thereof. In another embodiment, the filler is microcrystalline cellulose, and is present in an amount ranging from 10 to 20 percent by weight.

In one embodiment, the disintegrant is selected from agar-agar, algins, calcium carbonate, carboxmethylcellulose, cellulose, hydroxypropylcellulose, low substituted hydroxypropylcellulose, clays, croscarmellose sodium, crospovidone, gums, magnesium aluminum silicate, methylcellulose, polacrilin potassium, sodium alginate, sodium starch glycolate, maize starch, potato starch, tapioca starch, or any combination thereof. In another embodiment, the disintegrant is croscarmellose sodium, and is present in an amount ranging from 1 to 3 percent by weight.

In one embodiment, the surfactant is selected from sodium lauryl sulfate, sodium stearyl fumerate, polyoxyethylene 20 sorbitan mono-oleate, or any combination thereof. In another embodiment, the surfactant is sodium lauryl sulfate, and is present in an amount ranging from 0.5 to 2 percent by weight.

In one embodiment, the binder is selected from polyvinylpyrrolidone, dibasic calcium phosphate, sucrose, corn starch, modified cellulose, or any combination thereof. In another embodiment, the binder is polyvinylpyrrolidone, and is present in an amount ranging from 0 to 5 percent by weight.

Disclosed herein is a pharmaceutical composition having the following formulation:

| | % by wgt. |
|---|---|
| Compound 1 Form I | 35-50 |

(continued)

| | % by wgt. |
|---|---|
| Solid dispersion comprising substantially amorphous Compound 2 | 25-40 |
| Microcrystalline cellulose | 10-20 |
| Croscarmellose sodium | 1-3 |
| Sodium lauryl sulfate | 0.5-2 |
| Polyvinylpyrrolidone | 0-5 |

referred to as **PC-II.**

In another embodiment, the tablet comprises:

a. Compound 1 Form I;

b. a solid dispersion comprising substantially amorphous Compound 2;

c. a filler;

d. a disintegrant;

e. a surfactant;

f. a binder; and

g. a lubricant;

referred to as **PC-III.**

In another embodiment, the tablets of the present invention comprise about 200 mg of Compound 1 Form I, and about 125 mg of substantially amorphous Compound 2.

The tablets of the present invention comprise 25 to 50 percent by weight Compound 1 Form I, and 15 to 35 percent by weight a solid dispersion comprising substantially amorphous Compound 2.

In one embodiment, the filler is selected from cellulose, modified cellulose, sodium carboxymethyl cellulose, ethyl cellulose hydroxymethyl cellulose, hydroxypropylcellulose, cellulose acetate, microcrystalline cellulose, dibasic calcium phosphate, sucrose, lactose, corn starch, potato starch, or any combination thereof. In another embodiment, the filler is microcrystalline cellulose, and is present in an amount ranging from 20 to 30 percent by weight.

In one embodiment, the disintegrant is selected from agar-agar, algins, calcium carbonate, carboxmethylcellulose, cellulose, hydroxypropylcellulose, low substituted hydroxypropylcellulose, clays, croscarmellose sodium, crospovidone, gums, magnesium aluminum silicate, methylcellulose, polacrilin potassium, sodium alginate, sodium starch glycolate, maize starch, potato starch, tapioca starch, or any combination thereof. In another embodiment, the disintegrant is croscarmellose sodium, and is present in an amount ranging from 3 to 10 percent by weight.

In one embodiment, the surfactant is selected from sodium lauryl sulfate, sodium stearyl fumerate, polyoxyethylene 20 sorbitan mono-oleate, or any combination thereof. In another embodiment, the surfactant is sodium lauryl sulfate, and is present in an amount ranging from 0.5 to 2 percent by weight.

In one embodiment, the binder is selected from polyvinylpyrrolidone, dibasic calcium phosphate, sucrose, corn starch, modified cellulose, or any combination thereof. In another embodiment, the binder is polyvinylpyrrolidone, and is present in an amount ranging from 0 to 5 percent by weight.

In one embodiment, the lubricant is selected from magnesium stearate, calcium stearate, zinc stearate, sodium stearate, stearic acid, aluminum stearate, leucine, glyceryl behenate, hydrogenated vegetable oil or any combination thereof. In another embodiment, the lubricant is magnesium stearate, and is present in an amount ranging from 0.5 to 2 percent by weight.

In one embodiment, the present invention features a tablet having the following formulation:

| | % by wgt. |
|---|---|
| Compound 1 Form I | 25-50 |
| A solid dispersion comprising substantially amorphous Compound 2 | 15-35 |
| Microcrystalline cellulose | 20-30 |
| Croscarmellose sodium | 3-10 |
| Sodium lauryl sulfate | 0.5-2 |
| Polyvinylpyrrolidone | 0-5 |
| Magnesium stearate | 0.5-2 |

referred to as **PC-IV.**

In one embodiment, the tablets of the present invention further comprise a colorant and optionally a wax. In another embodiment, the colorant is present in an amount ranging from 2 to 4 percent by weight. In another embodiment, the wax is carnauba wax present in an amount ranging from 0 to 0.020 percent by weight.

The pharmaceutical compositions of the present invention are solid oral pharmaceutical compositions. Solid oral pharmaceutical compositions that are a granular pharmaceutical composition are disclosed herein. Solid oral pharmaceutical compositions of the invention are tablets.

Granular pharmaceutical compositions having the following formulation are disclosed herein:

| | % by wgt. |
|---|---|
| Compound 1 Form I | 43 |
| Solid dispersion comprising substantially amorphous Compound 2 | 34 |
| Microcrystalline cellulose | 17 |
| Croscarmellose sodium | 2 |
| Sodium lauryl sulfate | 1 |
| Polyvinylpyrrolidone | 3 |

referred to as **PC-V.**

Granular pharmaceutical compositions having the following formulation are disclosed herein:

| | % by wgt. |
|---|---|
| Compound 1 Form I | 38 |
| Solid dispersion comprising substantially amorphous Compound 2 | 40 |
| Microcrystalline cellulose | 16 |
| Croscarmellose sodium | 2 |
| Sodium lauryl sulfate | 1 |
| Polyvinylpyrrolidone | 3 |

referred to as **PC-VI.**

Granular pharmaceutical compositions having the following formulation are disclosed herein:

| | % by wgt. |
|---|---|
| Compound 1 Form I | 51 |
| Solid dispersion comprising substantially amorphous Compound 2 | 27 |
| Microcrystalline cellulose | 16 |

(continued)

| | % by wgt. |
|---|---|
| Croscarmellose sodium | 2 |
| Sodium lauryl sulfate | 1 |
| Polyvinylpyrrolidone | 3 |

referred to as **PC-VII.**

In one embodiment, the tablets of the present invention have the following formulation:

| | % by wgt. |
|---|---|
| Compound 1 Form I | 35 |
| Solid dispersion comprising substantially amorphous Compound 2 | 28 |
| Microcrystalline cellulose | 26 |
| Croscarmellose sodium | 6 |
| Sodium lauryl sulfate | 1 |
| Polyvinylpyrrolidone | 3 |
| Magnesium stearate | 1 |

referred to as **PC-VIII.**

In one embodiment, the tablets of the present invention have the following formulation:

| | % by wgt. |
|---|---|
| Compound 1 Form I | 31 |
| Solid dispersion comprising substantially amorphous Compound 2 | 32 |
| Microcrystalline cellulose | 26 |
| Croscarmellose sodium | 6 |
| Sodium lauryl sulfate | 1 |
| Polyvinylpyrrolidone | 3 |
| Magnesium stearate | 1 |

referred to as **PC-IX.**

Tablets having the following formulation are disclosed herein:

| | % by wgt. |
|---|---|
| Compound 1 Form I | 41 |
| Solid dispersion comprising substantially amorphous Compound 2 | 22 |
| Microcrystalline cellulose | 26 |
| Croscarmellose sodium | 6 |
| Sodium lauryl sulfate | 1 |
| Polyvinylpyrrolidone | 3 |
| Magnesium stearate | 1 |

referred to as **PC-X.**

In one embodiment, the tablets of the present invention have the following formulation:

| | mg |
|---|---|
| Compound 1 Form I | 200 |
| Solid dispersion comprising substantially amorphous Compound 2 | 156 |
| Microcrystalline cellulose | 150 |
| Croscarmellose sodium | 34 |
| Sodium lauryl sulfate | 4 |
| Polyvinylpyrrolidone | 15 |
| Magnesium stearate | 6 |

referred to as **PC-XI.**

Disclosed herein are tablets having the following formulation:

| | mg |
|---|---|
| Compound 1 Form I | 150 |
| Solid dispersion comprising substantially amorphous Compound 2 | 156 |
| Microcrystalline cellulose | 129 |
| Croscarmellose sodium | 30 |
| Sodium lauryl sulfate | 4 |
| Polyvinylpyrrolidone | 13 |
| Magnesium stearate | 5 |

referred to as **PC-XII.**

Disclosed herein are tablets having the following formulation:

| | mg |
|---|---|
| Compound 1 Form I | 200 |
| Solid dispersion comprising substantially amorphous Compound 2 | 104 |
| Microcrystalline cellulose | 128 |
| Croscarmellose sodium | 29 |
| Sodium lauryl sulfate | 4 |
| Polyvinylpyrrolidone | 13 |
| Magnesium stearate | 5 |

referred to as **PC-XIII.**

In one embodiment, the tablets of the present invention have the following formulation:

| | % by wgt. |
|---|---|
| Compound 1 Form I | 34 |
| Solid dispersion comprising substantially amorphous Compound 2 | 27 |
| Microcrystalline cellulose | 25 |
| Croscarmellose sodium | 6 |

(continued)

| | % by wgt. |
|---|---|
| Sodium lauryl sulfate | 1 |
| Polyvinylpyrrolidone | 3 |
| Magnesium stearate | 1 |
| Colorant | 3 |

referred to as **PC-XIV.**

In one embodiment, the tablets of the present invention have the following formulation:

| | % by wgt. |
|---|---|
| Compound 1 Form I | 30 |
| Solid dispersion comprising substantially amorphous Compound 2 | 31 |
| Microcrystalline cellulose | 25 |
| Croscarmellose sodium | 6 |
| Sodium lauryl sulfate | 1 |
| Polyvinylpyrrolidone | 3 |
| Magnesium stearate | 1 |
| Colorant | 3 |

referred to as **PC-XV.**

Disclosed herein are tablets having the following formulation:

| | % by wgt. |
|---|---|
| Compound 1 Form I | 40 |
| Solid dispersion comprising substantially amorphous Compound 2 | 21 |
| Microcrystalline cellulose | 25 |
| Croscarmellose sodium | 6 |
| Sodium lauryl sulfate | 1 |
| Polyvinylpyrrolidone | 3 |
| Magnesium stearate | 1 |
| Colorant | 3 |

referred to as **PC-XVI.**

In one embodiment, the tablets of the present invention have the following formulation:

| | mg |
|---|---|
| Compound 1 Form I | 200 |
| Solid dispersion comprising substantially amorphous Compound 2 | 156 |
| Microcrystalline cellulose | 150 |
| Croscarmellose sodium | 34 |
| Sodium lauryl sulfate | 4 |
| Polyvinylpyrrolidone | 15 |

(continued)

| | mg |
|---|---|
| Magnesium stearate | 6 |
| Colorant | 17 |

referred to as **PC-XVII.**

In one embodiment, the tablets of the present invention have the following formulation:

| | mg |
|---|---|
| Compound 1 Form I | 200 |
| Substantially amorphous Compound 2 | 125 |
| Microcrystalline cellulose | 150 |
| Croscarmellose sodium | 34 |
| Sodium lauryl sulfate | 4 |
| Polyvinylpyrrolidone | 15 |
| Magnesium stearate | 6 |
| Colorant | 17 |

referred to as **PC-XVIII**

Disclosed herein are tablets having the following formulation:

| | mg |
|---|---|
| Compound 1 Form I | 150 |
| Solid dispersion comprising substantially amorphous Compound 2 | 156 |
| Microcrystalline cellulose | 129 |
| Croscarmellose sodium | 29 |
| Sodium lauryl sulfate | 4 |
| Polyvinylpyrrolidone | 13 |
| Magnesium stearate | 5 |
| Colorant | 15 |

referred to as **PC-XIX.**

Disclosed herein are tablets having the following formulation:

| | mg |
|---|---|
| Compound 1 Form I | 200 |
| Solid dispersion comprising substantially amorphous Compound 2 | 104 |
| Microcrystalline cellulose | 128 |
| Croscarmellose sodium | 29 |
| Sodium lauryl sulfate | 4 |
| Polyvinylpyrrolidone | 13 |
| Magnesium stearate | 5 |
| Colorant | 14 |

referred to as **PC-XX.**

Disclosed herein are tablets having the following formulation:

| | mg |
|---|---|
| Compound 1 Form I | 200 |
| Solid dispersion comprising substantially amorphous Compound 2 | 83 |
| Microcrystalline cellulose | 128 |
| Croscarmellose sodium | 29 |
| Sodium lauryl sulfate | 4 |
| Polyvinylpyrrolidone | 13 |
| Magnesium stearate | 5 |
| Colorant | 14 |

referred to as **PC-XXI.**

Disclosed herein are tablets having the following formulation:

| Component | % by wgt. |
|---|---|
| Compound 1 Form I | 20-40 |
| Solid dispersion comprising substantially amorphous Compound 2 | 30-40 |
| Microcrystalline cellulose | 20-30 |
| Croscarmellose sodium | 1-10 |
| Polyvinylpyrrolidone | 1-5 |
| Sodium lauryl sulfate | 0.1-1 |
| Magnesium stearate | 0.5-1.5 |

referred to as **PC-XXII.**

In one embodiment, the tablets of the present invention have the following formulation:

| Compound 1/Compound 2 100 mg/125 mg | | | |
|---|---|---|---|
| Component | % in Granule | % in Tablet | mg/Tablet |
| Compound 1 Form I | 30 | 25 | 100 |
| Solid dispersion comprising substantially amorphous Compound 2 | 47 | 38 | 156 |
| Microcrystalline cellulose | 17 | 13 | 55 |
| Croscarmellose sodium | 2 | 2 | 7 |
| Polyvinylpyrrolidone | 3 | 3 | 11 |
| Sodium lauryl sulfate | 1 | 1 | 3 |
| Total Granules | 100 | 82 | 332 |
| Croscarmellose sodium | | 4 | 18 |
| Microcrystalline cellulose | | 13 | 53 |
| Magnesium stearate | | 1 | 4 |
| Total Tablet | | 100 | 407 |

referred to as **PC-XXIII.**

Disclosed herein are tablets having the following formulation:

| Compound 1/Compound 2 150 mg/125 mg | | | |
|---|---|---|---|
| Component | % in Granule | % in Tablet | mg/Tablet |
| Compound 1 Form I | 38 | 31 | 150 |
| Solid dispersion comprising substantially amorphous Compound 2 | 40 | 32 | 156 |
| Microcrystalline cellulose | 16 | 13 | 65 |
| Croscarmellose sodium | 2 | 2 | 8 |
| Polyvinylpyrrolidone | 3 | 3 | 13 |
| Sodium lauryl sulfate | 1 | 1 | 4 |
| Total Granules | 100 | 82 | 396 |
| Croscarmellose sodium | | 4 | 22 |
| Microcrystalline cellulose | | 13 | 64 |
| Magnesium stearate | | 1 | 5 |
| Total Tablet | | 100 | 487 |

referred to as **PC-XXIV.**

Disclosed herein are tablets having the following formulation:

| Compound 1/Compound 2 75 mg/125 mg | | | |
|---|---|---|---|
| Component | % in Granule | % in Tablet | mg/Tablet |
| Compound 1 Form I | 25 | 20 | 75 |
| Solid dispersion comprising substantially amorphous Compound 2 | 52 | 43 | 156 |
| Microcrystalline cellulose | 17 | 13 | 49 |
| Croscarmellose sodium | 2 | 2 | 6 |
| Polyvinylpyrrolidone | 3 | 3 | 10 |
| Sodium lauryl sulfate | 1 | 1 | 3 |
| Total Granules | 100 | 82 | 299 |
| Croscarmellose sodium | | 4 | 17 |
| Microcrystalline cellulose | | 13 | 48 |
| Magnesium stearate | | 1 | 4 |
| Core Tablet | | 100 | 368 |
| Pink Opadry | | 3 | 11 |
| Film Coated Tablet | | | 379 |

referred to as **PC-XXV.**

A pharmaceutical composition, granular pharmaceutical composition, or tablet of the present disclosure for use in a method of treating, lessening the severity of, or symptomatically treating cystic fibrosis in a patient, wherein the method comprises administering to the patient an effective amount of the pharmaceutical composition, granular pharmaceutical composition, or tablet are disclosed herein.

Disclosed herein is a tablet according to claim 6, 8, 9, or 10 for use in a method of treating, lessening the severity of, or symptomatically treating cystic fibrosis in a patient, wherein the method comprises administering to the patient an effective amount of the pharmaceutical composition according to claim 6, 8, 9, or 10.

In one aspect of the disclosure, the patient has a ΔF508 CFTR mutation. In another aspect of the disclosure,

the patient is homozygous in ΔF508. In another aspect of the disclosure, the patient is heterozygous in ΔF508. In another aspect of the disclosure, two tablets are administered to the patient per day.

In one aspect, the present invention features a method of preparing a tablet according to any one of claims 1-9, wherein the method comprises

a) wet granulating the following components to produce a granule:

a. Compound 1 Form I;

b. a solid dispersion comprising substantially amorphous Compound 2;

c. a filler;

d. a disintegrant;

e. a surfactant; and

f. a binder;

and
b) compressing the following components to produce a tablet:

i) a plurality of granules from step a)

ii) a disintegrant;

iii) a filler; and

iv) a lubricant;

wherein Compound 1 Form I is characterized by one or more peaks at 15.4, 16.3, and 14.5 degrees in an X-ray powder diffraction pattern, and
wherein substantially amorphous Compound 2 has less than 15% crystallinity.

In one aspect, the present invention features a kit comprising tablets of the present invention, and a separate therapeutic agent.

In one embodiment, the tablets of the present invention, and the separate therapeutic agent are in separate containers. In another embodiment, the separate containers are bottles. In another embodiment, the separate containers are vials. In another embodiment, the separate containers are blister packs.

Disclosed herein is a continuous or semi-continuous process for making the pharmaceutical compositions described herein by a twin screw wet granulation process comprising the steps of screening and weighing Compound 1, Compound 2, and excipients; mixing Compound 1, Compound 2, and excipients in a blender and feeding the blend into a continuous granulator while adding a granulation fluid comprising surfactant and a binder at a suitable rate for a suitable amount of time and chopping the mixture into granules; drying the granules; blending the granules with extra-granular excipients for a suitable amount of time; compressing the blend into tablets; coating the tablets; and, optionally, printing a monogram on one or both tablet faces.

## BRIEF DESCRIPTION OF DRAWINGS

**Figure 1** is an X-ray diffraction pattern calculated from a single crystal structure of Compound 1 Form I.

**Figure 2** is an actual X-ray powder diffraction pattern of Compound 1 Form I.

**Figure 3** is a graph depicting Compound 1 pH gradient dissolution profiles for a tablet made by a high shear granulation (HSG) process and a twin screw wet granulation (TSWG) process (LOD stands for loss on drying, a measure to define the amount of water in a powder/granule).

**Figure 4** is a graph depicting the stability of the substantially amorphous form of Compound 2 in tablet formulation PC-XVII at 50 °C after pre-equilibrating at 60% relative humidity by showing only a small amount of crystallinity over time.

**Figure 5** is a graph depicting the stability of the substantially amorphous form of Compound 2 in tablet formulation PC-XVII at 60 °C after pre-equilibrating at 60% relative humidity by showing only a small amount of crystallinity over time.

**Figure 6** is a graph depicting the stability of the substantially amorphous form of Compound 2 in tablet formulation PC-XX at 60 °C after pre-equilibrating at 60% relative humidity by showing only a small amount of crystallinity over time.

**Figure 7** is a graph depicting the stability of the substantially amorphous form of Compound 2 in tablet formulation PC-XX at 50 °C after pre-equilibrating at 60% relative humidity by showing only a small amount of crystallinity over time.

**Figure 8** is an $^1$HNMR spectrum of Compound 1.

**Figure 9** is an $^1$HNMR spectrum of Compound 1 HCl salt.

**Figure 10** is a differential scanning calorimetry (DSC) trace of Compound 1 Form I.

**Figure 11** is a conformational picture of Compound 1 Form I based on single crystal X-ray analysis.

## DETAILED DESCRIPTION

### DEFINITIONS

As used herein, "CFTR" stands for cystic fibrosis transmembrane conductance regulator.

As used herein, a "ΔF508 mutation" or "F508-del mutation" is a specific mutation within the CFTR protein. The mutation is a deletion of the three nucleotides that comprise the codon for amino acid phenylalanine at position 508, resulting in CFTR protein that lacks this phenylalanine residue.

As used herein, a patient who is "homozygous" for a particular mutation, e.g. ΔF508, has the same mutation on each allele.

As used herein, a patient who is "heterozygous" for a particular mutation, e.g. ΔF508, has this mutation on one allele, and a different mutation on the other allele.

As used herein, the term "CFTR corrector" refers to a compound that increases the amount of functional CFTR protein to the cell surface, resulting in enhanced ion transport.

As used herein, the term "CFTR potentiator" refers to a compound that increases the channel activity of CFTR protein located at the cell surface, resulting in enhanced ion transport.

As used herein, the term "active pharmaceutical ingredient" or "API" refers to a biologically active compound.

The terms "solid form", "solid forms" and related terms, when used herein refer to Compound 1 or Compound 2, in a particular solid form e.g. crystals, and amorphous states.

As used herein, the term "substantially amorphous" refers to a solid material having little or no long range order in the position of its molecules. For example, substantially amorphous materials have less than about 15% crystallinity (e.g., less than about 10% crystallinity or less than about 5% crystallinity). It is also noted that the term 'substantially amorphous' includes the descriptor, 'amorphous', which refers to materials having no (0%) crystallinity.

As used herein, the term "substantially crystalline" (as in the phrase substantially crystalline Compound 1 Form I refers to a solid material having predominantly long range order in the position of its molecules. For example, substantially crystalline materials have more than about 85% crystallinity (e.g., more than about 90% crystallinity or more than about 95% crystallinity). It is also noted that the term 'substantially crystalline' includes the descriptor, 'crystalline', which refers to materials having 100% crystallinity.

The term "crystalline" and related terms used herein, when used to describe a substance, component, product, or form, means that the substance, component or product is substantially crystalline as determined by X-ray diffraction. (See, e.g., Remington: The Science and Practice of Pharmacy, 21st Ed., Lippincott Williams & Wilkins, Baltimore, Md. (2003); The United States Pharmacopeia, 23rd ed., 1843-1844 (1995)).

As used herein, an "excipient" includes functional and non-functional ingredients in a pharmaceutical composition.

As used herein, a "disintegrant" is an excipient that hydrates a pharmaceutical composition and aids in tablet dispersion. As used herein, a "diluent" or "filler" is an excipient that adds bulkiness to a pharmaceutical composition.

As used herein, a "surfactant" is an excipient that imparts pharmaceutical compositions with enhanced solubility and/or wetability.

As used herein, a "binder" is an excipient that imparts a pharmaceutical composition with enhanced cohesion or tensile strength (e.g., hardness).

As used herein, a "glidant" is an excipient that imparts a pharmaceutical compositions with enhanced flow properties.

As used herein, a "colorant" is an excipient that imparts a pharmaceutical composition, e.g. a tablet, with a desired color. Examples of colorants include commercially available pigments such as FD&C Blue # 1 Aluminum Lake, FD&C Blue #2, other FD&C Blue colors, titanium dioxide, iron oxide, and/or combinations thereof. In one embodiment, the tablet provided by the invention is pink.

As used herein, a "lubricant" is an excipient that is added to pharmaceutical compositions that are pressed into tablets. The lubricant aids in compaction of granules into tablets and ejection of a tablet of a pharmaceutical composition from a die press.

As used herein, "cubic centimeter" and "cc" are used interchangeably to represent a unit of volume. Note that 1 cc = 1 mL.

As used herein, "kiloPond" and "kP" are used interchangeably and refer to the measure of force where a kP = approximately 9.8 Newtons.

As used herein, "friability" refers to the property of a tablet to remain intact and hold its form despite an external force of pressure. Friability can be quantified using the mathematical expression presented in equation 1:

$$\% friabiliy = 100 \times \frac{(W_0 - W_f)}{W_0} \qquad (1)$$

wherein $W_0$ is the original weight of the tablet and $W_f$ is the final weight of the tablet after it is put through the friabilator. Friability is measured using a standard USP testing apparatus that tumbles experimental tablets for 100 or 400 revolutions. Some tablets of the invention have a friability of less than 5.0%. In another embodiment, the friability is less than 2.0%. In another embodiment, the target friability is less than 1.0% after 400 revolutions.

As used herein, "mean particle diameter" is the average particle diameter as measured using techniques such as laser light scattering, image analysis, or sieve analysis. In one embodiment, the granules used to prepare the tablets provided by the invention have a mean particle diameter of less than 1.0 mm.

As used herein, "bulk density" is the mass of particles of material divided by the total volume the particles occupy. The total volume includes particle volume, inter-particle void volume and internal pore volume. Bulk density is not an intrinsic property of a material; it can change depending on how the material is processed. In one embodiment, the granules used to prepare the tablets provided by the invention have a bulk density of about 0.5-0.7 g/cc.

An "effective amount" or "therapeutically effective amount" of a compound of the invention may vary according to factors such as the disease state, age, and weight of the subject, and the ability of the compound of the invention to elicit a desired response in the subject. Dosage regimens may be adjusted to provide the optimum therapeutic response. An effective amount is also one in which any toxic or detrimental effects (e.g., side effects) of the compound of the invention are outweighed by the therapeutically beneficial effects.

As used herein, and unless otherwise specified, the terms "therapeutically effective amount" and "effective amount" of a compound mean an amount sufficient to provide a therapeutic benefit in the treatment or management of a disease or disorder, or to delay or minimize one or more symptoms associated with the disease or disorder. A "therapeutically effective amount" and "effective amount" of a compound mean an amount of therapeutic agent, alone or in combination with one or more other agent(s), which provides a therapeutic benefit in the treatment or management of the disease or disorder. The terms "therapeutically effective amount" and "effective amount" can encompass an amount that improves overall therapy, reduces or avoids symptoms or causes of disease or disorder, or enhances the therapeutic efficacy of another therapeutic agent.

"Substantially pure" as used in the phrase "substantially pure Compound 1 Form I" means greater than about 90% purity. In another embodiment, substantially pure refers to greater than about 95% purity. In another embodiment, substantially pure refers to greater than about 98% purity. In another embodiment, substantially pure refers to greater than about 99% purity.

With respect to Compound 1 Form I, or a solid dispersion comprising substantially amorphous Compound 2, the terms "about" and "approximately", when used in connection with doses, amounts, or weight percent of ingredients of a composition or a dosage form, mean a dose, amount, or weight percent that is recognized by one of ordinary skill

in the art to provide a pharmacological effect equivalent to that obtained from the specified dose, amount, or weight percent. Specifically the term "about" or "approximately" means an acceptable error for a particular value as determined by one of ordinary skill in the art, which depends in part on how the value is measured or determined. In certain embodiments, the term "about" or "approximately" means within 1, 2, 3, or 4 standard deviations. In certain embodiments, the term "about" or "approximately" means within 30%, 25%, 20%, 15%, 10%, 9%, 8%, 7%, 6%, 5%, 4%, 3%, 2%, 1%, 0.5%, 0.1%, or 0.05% of a given value or range.

**PHARMACEUTICAL COMPOSITIONS**

The invention provides tablets comprising Compound 1 Form I and a solid dispersion comprising substantially amorphous Compound 2. The amount of Compound 1 Form I that is present in the tablet is 100 mg or 200 mg. In these and other embodiments, Compound 1 Form I is present as substantially pure Compound 1 Form I. The amount of substantially amorphous Compound 2 that is present in the tablet is 125 mg. In these and other embodiments, substantially amorphous Compound 2 is present as substantially pure and amorphous Compound 2. "Substantially pure" means greater than ninety percent pure; preferably greater than 95 percent pure; more preferably greater than 99.5 percent pure.
In one embodiment, the invention provides a tablet comprising:

a. Compound 1 Form I;

b. a solid dispersion of substantially amorphous Compound 2;

c. a filler;

d. a disintegrant;

e. a surfactant; and

f. a binder.

In this aspect, the tablet comprises 200 mg of Compound 1 Form I.
The tablet comprises 125 mg of substantially amorphous Compound 2.
In some embodiments, the tablets comprise Compound 1 Form I, wherein Compound 1 Form I is present in an amount of at least 30 wt%, or at least 40 wt% by weight of the tablet.
The tablets comprise substantially amorphous Compound 2, wherein the substantially amorphous Compound 2 is present in an amount of at least 15 wt% (e.g., at least 20 wt%, or at least 30 wt%) by weight of the tablet.
In some embodiments, the tablet comprises Compound 1 Form I, a solid dispersion comprising substantially amorphous Compound 2, a filler, a disintegrant, a surfactant, and a binder.
The concentration of Compound 1 Form I and substantially amorphous Compound 2 in the tablet depends on several factors such as the amount of tablet needed to provide a desired amount of Compound 1 Form I and substantially amorphous Compound 2 and the desired dissolution profile of the tablet.
In another embodiment, the tablet comprises Compound 1 Form I, in which Compound 1 Form I in its solid form has a mean particle diameter, measured by light scattering (e.g., using a Malvern Mastersizer available from Malvern Instruments in England) of 0.1 microns to 10 microns. In another embodiment, the particle size of Compound 1 Form I is 1 micron to 5 microns. In another embodiment, Compound 1 Form I has a particle size D50 of 2.0 microns.
As indicated, in addition to Compound 1 Form I and a solid dispersion of substantially amorphous Compound 2, in some embodiments of the invention, the tablets also comprise one or more excipients such as fillers, disintegrants, surfactants, diluents, binders, glidants, lubricants, colorants, or fragrances and any combination thereof.
Fillers suitable for the invention are compatible with the ingredients of the tablet, i.e., they do not substantially reduce the solubility, the hardness, the chemical stability, the physical stability, or the biological activity of the tablet. Exemplary fillers include: celluloses, modified celluloses, (e.g. sodium carboxymethyl cellulose, ethyl cellulose hydroxymethyl cellulose, hydroxypropylcellulose), cellulose acetate, microcrystalline cellulose, calcium phosphates, dibasic calcium phosphate, starches (e.g. corn starch, potato starch), sugars (e.g., sorbitol, lactose, sucrose), or any combination thereof.
Thus, in one embodiment, the tablet comprises at least one filler in an amount of at least 5 wt% (e.g., at least about 20 wt%, at least about 30 wt%, or at least about 40 wt%) by weight of the composition. For example, the tablet comprises from about 10 wt% to about 60 wt% (e.g., from about 20 wt% to about 55 wt%, from about 25 wt% to about 50 wt%, or from about 27 wt% to about 45 wt%) of filler, by weight of the composition. In another example, the tablet comprises at least about 20 wt% (e.g., at least 30 wt% or at least 40 wt%) of microcrystalline cellulose, for example

MCC Avicel PH102, by weight of the composition. In yet another example, the tablet comprises from about 10 wt% to about 60 wt% (e.g., from about 20 wt% to about 55 wt% or from about 25 wt% to about 45 wt%) of microcellulose, by weight of the composition.

Disintegrants suitable for the invention enhance the dispersal of the tablet and are compatible with the ingredients of the tablet, i.e., they do not substantially reduce the chemical stability, the physical stability, the hardness, or the biological activity of the tablet. Exemplary disintegrants include croscarmellose sodium, sodium starch glycolate, or a combination thereof.

Thus, in one embodiment, the tablet comprises disintegrant in an amount of about 10 wt% or less (e.g., about 7 wt% or less, about 6 wt% or less, or about 5 wt% or less) by weight of the composition. For example, the tablet comprises from about 1 wt% to about 10 wt% (e.g., from about 1.5 wt% to about 7.5 wt% or from about 2.5 wt% to about 6 wt%) of disintegrant, by weight of the composition. In another example, the tablet comprises about 10 wt% or less (e.g., 7 wt% or less, 6 wt% or less, or 5 wt% or less) of croscarmellose sodium, by weight of the composition. In yet another example, the tablet comprises from about 1 wt% to about 10 wt% (e.g., from about 1.5 wt% to about 7.5 wt% or from about 2.5 wt% to about 6 wt%) of croscarmellose sodium, by weight of the composition. In some examples, the tablet comprises from about 0.1% to about 10 wt% (e.g., from about 0.5 wt% to about 7.5 wt% or from about 1.5 wt% to about 6 wt%) of disintegrant, by weight of the composition. In still other examples, the tablet comprises from about 0.5% to about 10 wt% (e.g., from about 1.5 wt% to about 7.5 wt% or from about 2.5 wt% to about 6 wt%) of disintegrant, by weight of the composition.

Surfactants suitable for the invention enhance the wettability of the tablet and are compatible with the ingredients of the tablet, i.e., they do not substantially reduce the chemical stability, the physical stability, the hardness, or the biological activity of the tablet. Exemplary surfactants include sodium lauryl sulfate (SLS), sodium stearyl fumarate (SSF), polyoxyethylene 20 sorbitan mono-oleate (e.g., Tween™), or any combination thereof.

Thus, in one embodiment, the tablet comprises a surfactant in an amount of about 10 wt% or less (e.g., about 5 wt% or less, about 2 wt% or less, about 1 wt% or less, about 0.8 wt% or less, or about 0.6 wt% or less) by weight of the composition. For example, the tablet includes from about 10 wt% to about 0.1 wt% (e.g., from about 5 wt% to about 0.2 wt% or from about 2 wt% to about 0.3 wt%) of surfactant, by weight of the composition. In another example, the tablet comprises 10 wt% or less (e.g., about 5 wt% or less, about 2 wt% or less, about 1 wt% or less, about 0.8 wt% or less, or about 0.6 wt% or less) of sodium lauryl sulfate, by weight of the composition. In yet another example, the tablet comprises from about 10 wt% to about 0.1 wt% (e.g., from about 5 wt% to about 0.2 wt% or from about 2 wt% to about 0.3 wt%) of sodium lauryl sulfate, by weight of the composition.

Binders suitable for the invention enhance the tablet strength of the tablet and are compatible with the ingredients of the tablet, i.e., they do not substantially reduce the chemical stability, the physical stability, or the biological activity of the tablet. Exemplary binders include polyvinylpyrrolidone, dibasic calcium phosphate, sucrose, corn (maize) starch, modified cellulose (e.g., hydroxymethyl cellulose), or any combination thereof.

Thus, in one embodiment, the tablet comprises a binder in an amount of at least about 0.1 wt% (e.g., at least about 1 wt%, at least about 3 wt%, at least about 4 wt%, or at least about 5 wt%) by weight of the composition. For example, the tablet comprises from about 0.1 wt% to about 10 wt% (e.g., from about 1 wt% to about 10 wt% or from about 2 wt% to about 7 wt%) of binder, by weight of the composition. In another example, the tablet comprises at least about 0.1 wt% (e.g., at least about 1 wt%, at least about 2 wt%, at least about 3 wt%, or at least about 4 wt%) of polyvinylpyrrolidone, by weight of the composition. In yet another example, the tablet comprises a glidant in an amount ranging from about 0.1 wt% to about 10 wt% (e.g., from about 1 wt% to about 8 wt% or from about 2 wt% to about 5 wt%) of polyvinylpyrrolidone, by weight of the composition.

Diluents suitable for the invention may add necessary bulk to a formulation to prepare tablets of the desired size and are generally compatible with the ingredients of the tablet, i.e., they do not substantially reduce the solubility, the hardness, the chemical stability, the physical stability, or the biological activity of the tablet. Exemplary diluents include: sugars, for example, confectioner's sugar, compressible sugar, dextrates, dextrin, dextrose, lactose, mannitol, sorbitol, cellulose, and modified celluloses, for example, powdered cellulose, talc, calcium phosphate, starch, or any combination thereof.

Thus, in one embodiment, the tablet comprises a diluent in an amount of 40 wt% or less (e.g., 35 wt% or less, 30 wt% or less, or 25 wt% or less, or 20 wt% or less, or 15 wt% or less, or 10 wt% or less) by weight of the composition. For example, the tablet comprises from about 40 wt% to about 1 wt% (e.g., from about 35 wt% to about 5 wt% or from about 30 wt% to about 7 wt%, from about 25 wt% to about 10 wt%, from about 20 wt% to about 15 wt%) of diluent, by weight of the composition. In another example, the tablet comprises 40 wt% or less (e.g., 35 wt% or less, 25 wt% or less, or 15 wt% or less) of mannitol, by weight of the composition. In yet another example, the tablet comprises from about 35 wt% to about 1 wt% (e.g., from about 30 wt% to about 5 wt% or from about 25 wt% to about 10 wt%) of mannitol, by weight of the composition.

Glidants suitable for the invention enhance the flow properties of the tablet and are compatible with the ingredients of the tablet, i.e., they do not substantially reduce the solubility, the hardness, the chemical stability, the physical

stability, or the biological activity of the tablet. Exemplary glidants include colloidal silicon dioxide, talc, or a combination thereof.

Thus, in one embodiment, the tablet comprises a glidant in an amount of 2 wt% or less (e.g., 1.75 wt%, 1.25 wt% or less, or 1.00 wt% or less) by weight of the composition. For example, the tablet comprises from about 2 wt% to about 0.05 wt% (e.g., from about 1.5 wt% to about 0.07 wt% or from about 1.0 wt% to about 0.09 wt%) of glidant, by weight of the composition. In another example, the tablet comprises 2 wt% or less (e.g., 1.75 wt%, 1.25 wt% or less, or 1.00 wt% or less) of colloidal silicon dioxide, by weight of the composition. In yet another example, the tablet comprises from about 2 wt% to about 0.05 wt% (e.g., from about 1.5 wt% to about 0.07 wt% or from about 1.0 wt% to about 0.09 wt%) of colloidal silicon dioxide, by weight of the composition.

In some embodiments, the tablet can comprise a lubricant that can prevent adhesion of a granulate-bead admixture to a surface (e.g., a surface of a mixing bowl, a compression die and/or punch). A lubricant can also reduce interparticle friction within the granulate and improve the compression and ejection of compressed tablets from a die press. The lubricant is also compatible with the ingredients of the tablet, i.e., they do not substantially reduce the solubility, the hardness, or the biological activity of the tablet. Exemplary lubricants include magnesium stearate, calcium stearate, zinc stearate, sodium stearate, stearic acid, aluminum stearate, leucine, glyceryl behenate, hydrogenated vegetable oil or any combination thereof. In one embodiment, the tablet comprises a lubricant in an amount of 5 wt% or less (e.g., 4.75 wt%, 4.0 wt% or less, or 3.00 wt% or less, or 2.0 wt% or less) by weight of the composition. For example, the tablet comprises from about 5 wt% to about 0.10 wt% (e.g., from about 4.5 wt% to about 0.5 wt% or from about 3 wt% to about 1 wt%) of lubricant, by weight of the composition. In another example, the tablet comprises 5 wt% or less (e.g., 4.0 wt% or less, 3.0 wt% or less, or 2.0 wt% or less, or 1.0 wt% or less) of magnesium stearate, by weight of the composition. In yet another example, the tablet comprises from about 5 wt% to about 0.10 wt% (e.g., from about 4.5 wt% to about 0.15 wt% or from about 3.0 wt% to about 0.50 wt%) of magnesium stearate, by weight of the composition.

Tablets of the invention can optionally comprise one or more colorants, flavors, and/or fragrances to enhance the visual appeal, taste, and/or scent of the tablet. Suitable colorants, flavors, or fragrances are compatible with the ingredients of the tablet, i.e., they do not substantially reduce the solubility, the chemical stability, the physical stability, the hardness, or the biological activity of the tablet. In one embodiment, the tablet comprises a colorant, a flavor, and/or a fragrance. In one embodiment, the tablets provided by the invention are purple.

In some embodiments, the tablets can be coated with a colorant and optionally labeled with a logo, other image and/or text using a suitable ink. In still other embodiments, the tablets can be coated with a colorant, waxed, and optionally labeled with a logo, other image and/or text using a suitable ink. Suitable colorants and inks are compatible with the ingredients of the tablet, i.e., they do not substantially reduce the solubility, the chemical stability, the physical stability, the hardness, or the biological activity of the tablet. The suitable colorants and inks can be any color and are water based or solvent based. In one embodiment, the tablets are coated with a colorant and then labeled with a logo, other image, and/or text using a suitable ink. For example, tablets of the invention can be coated with about 3 wt% (e.g., less than about 6 wt% or less than about 4 wt%) of film coating comprising a colorant. The colored tablets can be labeled with a logo and text indicating the strength of the active ingredient in the tablet using a suitable ink. In another example, tablets of the invention can be coated with about 3 wt% (e.g., less than about 6 wt% or less than about 4 wt%) of a film coating comprising a colorant.

In another embodiment, tablets of the invention are coated with a colorant, waxed, and then labeled with a logo, other image, and/or text using a suitable ink. For example, tablets of the invention can be coated with about 3 wt% (e.g., less than about 6 wt% or less than about 4 wt%) of film coating comprising a colorant. The colored tablets can be waxed with Carnauba wax powder weighed out in the amount of about 0.01% w/w of the starting tablet core weight. The waxed tablets can be labeled with a logo and text indicating the strength of the active ingredient in the tablet using a suitable ink. In another example, tablets of the invention can be coated with about 3 wt% (e.g., less than about 6 wt% or less than about 4 wt%) of a film coating comprising a colorant. The colored tablets can be waxed with Carnauba wax powder weighed out in the amount of about 0.01% w/w of the starting tablet core weight. The waxed tablets can be labeled with a logo and text indicating the strength of the active ingredient in the tablet using a pharmaceutical grade ink such as a black ink (e.g., Opacode® S-1-17823, a solvent based ink, commercially available from Colorcon, Inc. of West Point, PA.).

One exemplary pharmaceutical composition disclosed herein comprises from about 15 wt% to about 70 wt% (e.g., from about 15 wt% to about 60 wt%, from about 15 wt% to about 50 wt%, or from about 20 wt% to about 70 wt%, or from about 30 wt% to about 70 wt%) of Compound 1 Form I, by weight of the composition; and from about 15 wt% to about 40 wt% (e.g., about 20-35 wt%) of substantially amorphous Compound 2 by weight of the composition, and more typically, from 25 wt% to about 30 wt% of substantially amorphous Compound 2 by weight of the composition. The aforementioned compositions can also include one or more pharmaceutically acceptable excipients, for example, from about 20 wt% to about 50 wt% of a filler; from about 1 wt% to about 5 wt% of a disintegrant; from about 2 wt% to about 0.3 wt% of a surfactant; and from about 0.1 wt% to about 5 wt% of a binder.

Another exemplary pharmaceutical composition disclosed herein comprises from about 15 wt% to about 70

wt% (e.g., from about 15 wt% to about 60 wt%, from about 15 wt% to about 50 wt%, or from about 15 wt% to about 40 wt% or from about 20 wt% to about 70 wt%, or from about 30 wt% to about 70 wt%, or from about 40 wt% to about 70 wt%, or from about 50 wt% to about 70 wt%) of Compound 1 Form I by weight of the composition, from about 15 wt% to about 40 wt% (e.g., about 20-35 wt%) of substantially amorphous Compound 2 by weight of the composition, and more typically, from 25 wt% to about 30 wt% of substantially amorphous Compound 2 by weight of the composition, and one or more excipients, for example, from about 20 wt% to about 50 wt% of a filler; from about 1 wt% to about 5 wt% of a disintegrant; from about 2 wt% to about 0.3 wt% of a surfactant; from about 0.1 wt% to about 5 wt% of a binder; and from about 2 wt% to about 0.1 wt% of a lubricant.

Another exemplary pharmaceutical composition disclosed herein comprises from about 15 wt% to about 70 wt% (e.g., from about 15 wt% to about 60 wt%, from about 15 wt% to about 50 wt%, or from about 15 wt% to about 40 wt% or from about 20 wt% to about 70 wt%, or from about 30 wt% to about 70 wt%, or from about 40 wt% to about 70 wt%, or from about 50 wt% to about 70 wt%) of Compound 1 Form I by weight of the composition, from about 15 wt% to about 40 wt% (e.g., about 20-35 wt%) of substantially amorphous Compound 2 by weight of the composition, and more typically, from 25 wt% to about 30 wt% of substantially amorphous Compound 2 by weight of the composition, and one or more excipients, for example, from about 20 wt% to about 50 wt% of a filler; from about 1 wt% to about 5 wt% of a disintegrant; from about 2 wt% to about 0.3 wt% of a surfactant; from about 0.1 wt% to about 5 wt% of a binder; from about 2 wt% to about 0.1 wt% of a lubricant; from about 2 wt% to about 4 wt% colorant; and about 0.005 wt% to about 0.015 wt% wax.

Also disclosed herein is a granular pharmaceutical composition comprising:

a. about 43 wt% of Compound 1 Form I by weight of the composition;

b. about 34 wt% of a solid dispersion comprising substantially amorphous Compound 2 by weight of the composition;

c. about 17 wt% of microcrystalline cellulose by weight of the composition;

d. about 2 wt% of croscarmellose sodium by weight of the composition;

e. about 1 wt% of sodium lauryl sulfate by weight of the composition; and

f. about 3 wt% of polyvinylpyrrolidone by weight of the composition.

In one embodiment, the invention is a tablet comprising:

a. about 35 wt% of Compound 1 Form I by weight of the composition;

b. about 28 wt% of a solid dispersion comprising substantially amorphous Compound 2 by weight of the composition;

c. about 26 wt% of microcrystalline cellulose by weight of the composition;

d. about 6 wt% of croscarmellose sodium by weight of the composition;

e. about 3 wt% of polyvinylpyrrolidone by weight of the composition;

f. about 1 wt% of sodium lauryl sulfate by weight of the composition; and

g. about 1 wt% of magnesium stearate by weight of the composition.

In one embodiment, the invention is a tablet comprising:

a. about 34 wt% of Compound 1 Form I by weight of the composition;

b. about 27 wt% of a solid dispersion comprising substantially amorphous Compound 2 by weight of the composition;

c. about 26 wt% of microcrystalline cellulose by weight of the composition;

d. about 6 wt% of croscarmellose sodium by weight of the composition;

e. about 2 wt% of polyvinylpyrrolidone by weight of the composition

f. about 1 wt% of sodium lauryl sulfate by weight of the composition;

g. about 1 wt% of magnesium stearate by weight of the composition;

h. about 3 wt% of a colorant by weight of the composition; and

i. about 0.010 wt% of a wax by weight of the composition.

Another tablet disclosed herein comprises:

a. about 150 to 250 mg of Compound 1 Form I;
b. about 100 to 150 mg of substantially amorphous Compound 2;
c. about 125 to 175 mg of microcrystalline cellulose;
d. about 20 to 40 mg of croscarmellose sodium;
e. about 10 to 20 mg of polyvinylpyrrolidone;
f. about 2 to 6 mg of sodium lauryl sulfate; and
g. about 3 to 7 mg of magnesium stearate.

Another tablet of the invention comprises:

a. 200 mg of Compound 1 Form I;
b. 125 mg of substantially amorphous Compound 2;
c. about 150 mg of microcrystalline cellulose;
d. about 34 mg of croscarmellose sodium;
e. about 15 mg of polyvinylpyrrolidone;
f. about 4 mg of sodium lauryl sulfate; and
g. about 6 mg of magnesium stearate.

Another tablet of the invention comprises:

a. 200 mg of Compound 1 Form I;
b. 125 mg of substantially amorphous Compound 2;
c. about 150 mg of microcrystalline cellulose;
d. about 34 mg of croscarmellose sodium;
e. about 15 mg of polyvinylpyrrolidone;
f. about 4 mg of sodium lauryl sulfate;
g. about 6 mg of magnesium stearate;
h. about 17 mg of a colorant; and
i. about 0.06 mg of a wax.

Also disclosed herein is a granular pharmaceutical composition comprising:

a. about 38 wt% of Compound 1 Form I by weight of the composition;

b. about 40 wt% of a solid dispersion comprising substantially amorphous Compound 2 by weight of the composition;

c. about 16 wt% of microcrystalline cellulose by weight of the composition;

d. about 2 wt% of croscarmellose sodium by weight of the composition;

e. about 1 wt% of sodium lauryl sulfate by weight of the composition; and

f. about 3 wt% of polyvinylpyrrolidone by weight of the composition.

In one embodiment, the invention is a tablet comprising:

a. about 31 wt% of Compound 1 Form I by weight of the composition;

b. about 32 wt% of a solid dispersion comprising substantially amorphous Compound 2 by weight of the composition;

c. about 26 wt% of microcrystalline cellulose by weight of the composition;

d. about 6 wt% of croscarmellose sodium by weight of the composition;

e. about 3 wt% of polyvinylpyrrolidone by weight of the composition

f. about 1 wt% of sodium lauryl sulfate by weight of the composition;

g. about 1 wt% of magnesium stearate by weight of the composition; and

h. about 3 wt% of a colorant by weight of the composition.

Another tablet disclosed herein comprises:

a. about 100 to 200 mg of Compound 1 Form I;

b. about 100 to 150 mg of substantially amorphous Compound 2;

c. about 100 to 150 mg of microcrystalline cellulose;

d. about 20 to 40 mg of croscarmellose sodium;

e. about 10 to 20 mg of polyvinylpyrrolidone;

f. about 2 to 6 mg of sodium lauryl sulfate; and

g. about 3 to 7 mg of magnesium stearate.

Another tablet disclosed herein comprises:

a. about 150 mg of Compound 1 Form I;

b. about 125 mg of substantially amorphous Compound 2;

c. about 129 mg of microcrystalline cellulose;

d. about 29 mg of croscarmellose sodium;

e. about 13 mg of polyvinylpyrrolidone;

f. about 4 mg of sodium lauryl sulfate;

g. about 5 mg of magnesium stearate; and

h. about 15 mg of a colorant.

The pharmaceutical compositions disclosed herein can be processed into a tablet form, capsule form, pouch form, lozenge form, or other solid form that is suited for oral administration. The pharmaceutical compositions of the invention are in tablet form.

Another aspect of the invention provides a tablet that includes Compound 1 Form I, a solid dispersion comprising substantially amorphous Compound 2, and excipients (e.g., a filler, a disintegrant, a surfactant, a binder, a colorant, a lubricant, or any combination thereof), each of which is described above and in the Examples below, wherein the tablet has a dissolution of at least about 50% (e.g., at least about 60%, at least about 70%, at least about 80%, at least about 90%, or at least about 99%) in about 30 minutes.

In one example, the tablet includes Compound 1 Form I in an amount of 100 mg, or 200 mg, substantially amorphous Compound 2 in an amount of 125 mg, and one or more excipients (e.g., a filler, a disintegrant, a surfactant, a binder, a colorant, a lubricant, or any combination thereof) each of which is described above and in the Examples below, wherein the tablet has a dissolution of from about 50% to about 100% (e.g., from about 55% to about 95% or from about 60% to about 90%) in about 30 minutes.

Dissolution can be measured with a standard USP Type II apparatus that employs a dissolution media of 0.1% CTAB dissolved in 900 mL of DI water, buffered at pH 6.8 with 50 mM potassium phosphate monoasic, stirring at about 50-75 rpm at a temperature of about 37 °C. A single experimental tablet is tested in each test vessel of the apparatus. Dissolution can also be measured with a standard USP Type II apparatus that employs a dissolution media of 0.7% sodium lauryl sulfate dissolved in 900 mL of 50 mM sodium phosphate buffer (pH 6.8), stirring at about 65 rpm at a temperature of about 37 °C. A single experimental tablet is tested in each test vessel of the apparatus. Dissolution can also be measured with a standard USP Type II apparatus that employs a dissolution media of 0.5% sodium lauryl sulfate dissolved in 900 mL of 50 mM sodium phosphate buffer (pH 6.8), stirring at about 65 rpm at a temperature of about 37 °C. A single experimental tablet is tested in each test vessel of the apparatus.

**METHODS FOR MAKING COMPOUND 1 FORM I AND A SOLID DISPERSION COMPRISING SUBSTANTIALLY AMORPHOUS COMPOUND 2**

Compound 1

Compound 1 is used as the starting point for Compound 1 Form I and can be prepared by coupling an acid chloride moiety with an amine moiety according to Schemes 1-4.

**Scheme 1. Synthesis of the acid chloride moiety.**

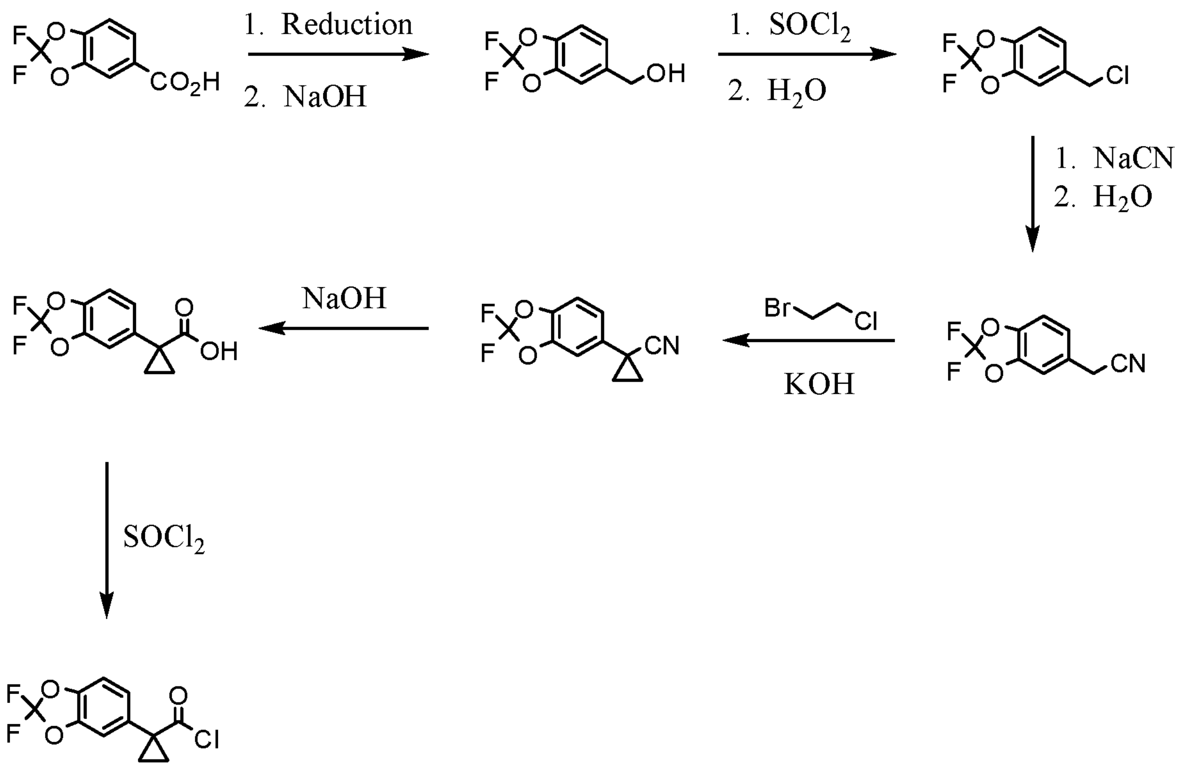


Scheme 1 depicts the preparation of 1-(2,2-difluorobenzo[d][1,3]dioxol-5-yl)cyclopropanecarbonyl chloride, which is used in Scheme 3 to make the amide linkage of Compound 1.

The starting material, 2,2-difluorobenzo[d][1,3]dioxole-5-carboxylic acid, is commercially available from Saltigo (an affiliate of the Lanxess Corporation). Reduction of the carboxylc acid moiety in 2,2-difluorobenzo[d][1,3]dioxole-5-carboxylic acid to the primary alcohol, followed by conversion to the corresponding chloride using thionyl chloride (SOCl2), provides 5-(chloromethyl)-2,2-difluorobenzo[d][1,3]dioxole, which is subsequently converted to 2-(2,2-difluorobenzo[d][1,3]dioxol-5-yl)acetonitrile using sodium cyanide. Treatment of 2-(2,2-difluorobenzo[d][1,3]dioxol-5-yl)acetonitrile

with base and 1-bromo-2-chloroethane provides 1-(2,2-difluorobenzo[d][1,3]dioxol-5-yl)cyclopropanecarbonitrile. The nitrile moiety in 1-(2,2-difluorobenzo[d][1,3]dioxol-5-yl)cyclopropanecarbonitrile is converted to a carboxylic acid using base to give 1-(2,2-difluorobenzo[d][1,3]dioxol-5-yl)cyclopropanecarboxylic acid, which is converted to the desired acid chloride using thionyl chloride.

**Scheme 2. Alternative synthesis of the acid chloride moiety.**

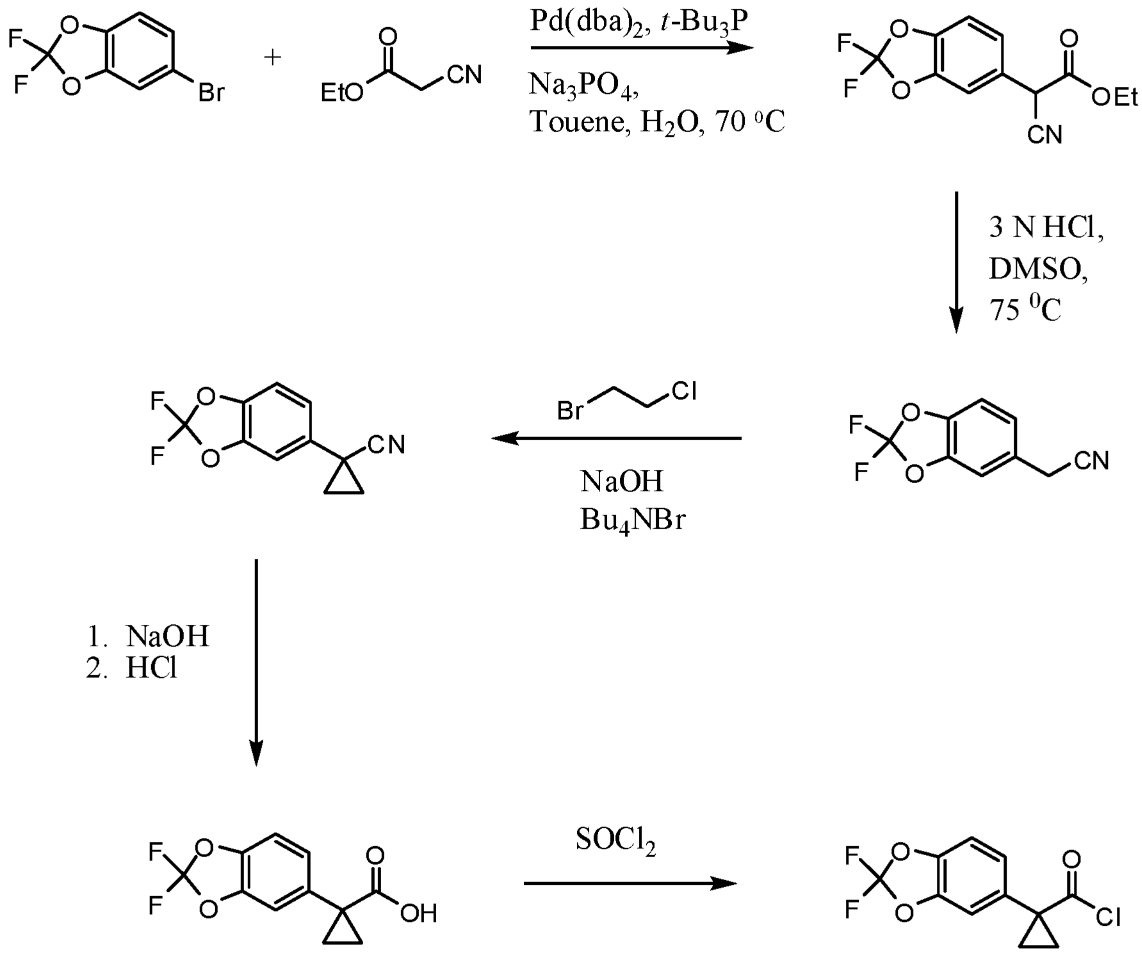


Scheme 2 depicts an alternative synthesis of the requisite acid chloride. 5-bromomethyl-2,2-difluoro-1,3-benzodioxole is coupled with ethyl cyanoacetate in the presence of a palladium catalyst to form the corresponding alpha cyano ethyl ester. Saponification of the ester moiety to the carboxylic acid gives the cyanoethyl compound. Alkylation of the cyanoethyl compound with 1-bromo-2-chloro ethane in the presence of base gives the cyanocyclopropyl compound. Treatment of the cyanocyclopropyl compound with base gives the carboxylate salt, which is converted to the carboxylic acid by treatment with acid. Conversion of the carboxylic acid to the acid chloride is then accomplished using a chlorinating agent such as thionyl chloride.

**Scheme 3. Synthesis of the amine moiety.**

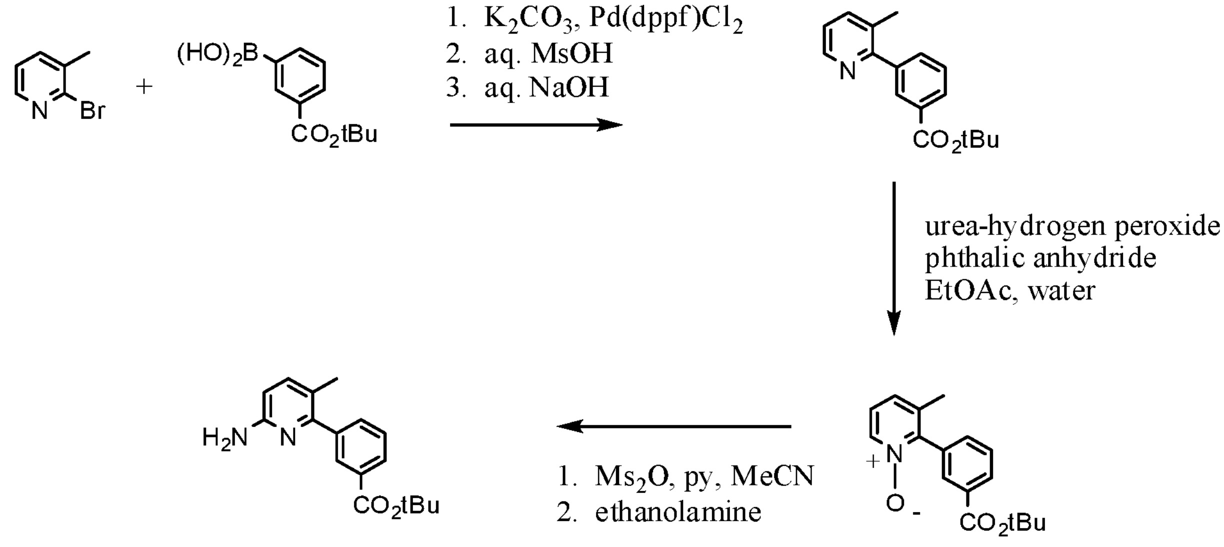


Scheme 3 depicts the preparation of the requisite tert-butyl 3-(6-amino-3-methylpyridin-2-yl)benzoate, which is coupled with 1-(2,2-difluorobenzo[d][1,3]dioxol-5-yl)cyclopropanecarbonyl chloride in Scheme 3 to give Compound 1. Palladium-catalyzed coupling of2-bromo-3-methylpyridine with 3-(tert-butoxycarbonyl)phenylboronic acid gives tert-butyl 3-(3-methylpyridin-2-yl)benzoate, which is subsequently converted to the desired compound.

**Scheme 4. Formation of an acid salt of 3-(6-(1-(2,2-difluorobenzo[d][1,3]dioxol-5-yl) cyclopropanecarboxamido)-3-methylpyridin-2-yl)benzoic acid.**

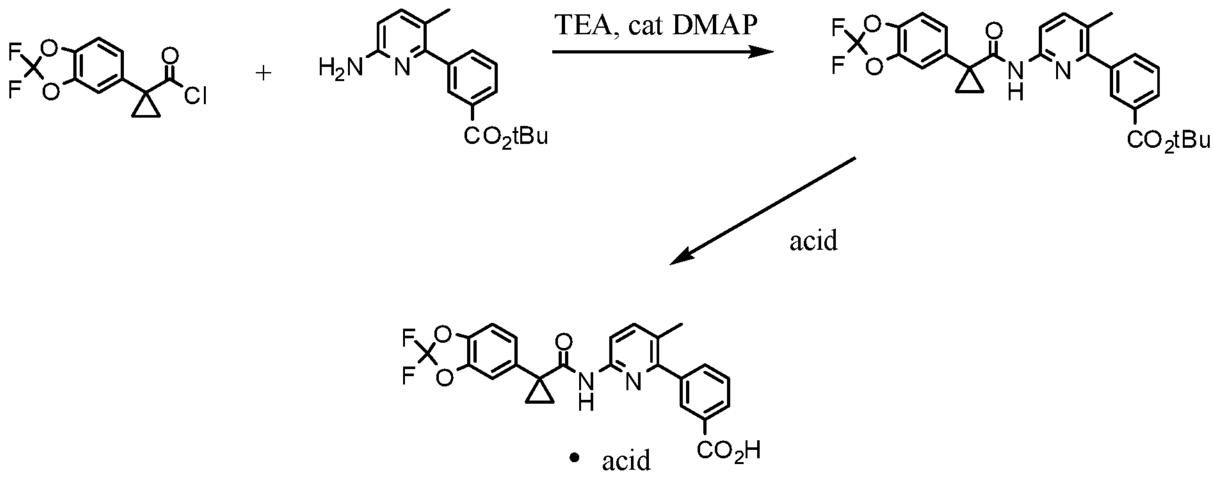


Scheme 4 depicts the coupling of 1-(2,2-difluorobenzo[d][1,3]dioxol-5-yl)cyclopropanecarbonyl chloride with tert-butyl 3-(6-amino-3-methylpyridin-2-yl)benzoate using triethyl amine and 4-dimethylaminopyridine to initially provide the tert-butyl ester of Compound 1.

Compound 1 Form I

Compound 1 Form I is prepared by dispersing or dissolving a salt form, such as the HCl salt, of Compound 1 in an appropriate solvent for an effective amount of time. Treatment of the tert-butyl ester with an acid such as HCl, gives the HCL salt of Compound 1, which is typically a crystalline solid. Compound 1 Form I may also be prepared directly from the t-butyl ester precursor by treatment with an appropriate acid, such as formic acid.

The HCl salt of 3-(6-(1-(2,2-difluorobenzo[d][1,3]dioxol-5-yl) cyclopropanecarboxamido)-3-methylpyridin-2-yl)benzoic acid can be used to make Form I by dispersing or dissolving the HCl salt of 3-(6-(1-(2,2-difluorobenzo[d][1,3]dioxol-5-yl) cyclopropanecarboxamido)-3-methylpyridin-2-yl)benzoic acid in an appropriate solvent for an effective amount

of time. Other salts of 3-(6-(1-(2,2-difluorobenzo[d][1,3]dioxol-5-yl) cyclopropanecarboxamido)-3-methylpyridin-2-yl)benzoic acid may be used, such as, for example, salts derived from other mineral or organic acids. The other salts result from acid-mediated hydrolysis of the t-butyl ester moiety. Salts derived from other acids may include, for example, nitric, sulfuric, phosphoric, boric, acetic, benzoic and malonic. These salt forms of 3-(6-(1-(2,2-difluorobenzo[d][1,3]dioxol-5-yl) cyclopropanecarboxamido)-3-methylpyridin-2-yl)benzoic acid may or may not be soluble, depending upon the solvent used, but lack of solubility does not hinder formation of Compound 1 Form I. For example, in one embodiment, the appropriate solvent may be water or an alcohol/water mixture such as 50% methanol/water mixture, even though the HCl salt form of 3-(6-(1-(2,2-difluorobenzo[d][1,3]dioxol-5-yl) cyclopropanecarboxamido)-3-methylpyridin-2-yl)benzoic acid is only sparingly soluble in water. In one embodiment, the appropriate solvent is water.

The effective amount of time for formation of Compound 1 Form I from the salt of 3-(6-(1-(2,2-difluorobenzo[d][1,3]dioxol-5-yl) cyclopropanecarboxamido)-3-methylpyridin-2-yl)benzoic acid can be any time between 2 to 24 hours or greater. It is recognized that the amount of time needed is inversely proportional to the temperature. That is, the higher the temperature the less time needed to affect dissociation of acid to form Compound 1 Form I. When the solvent is water, stirring the dispersion for approximately 24 hours at room temperature provides Compound 1 Form I in an approximately 98% yield. If a solution of the salt of 3-(6-(1-(2,2-difluorobenzo[d][1,3]dioxol-5-yl) cyclopropanecarboxamido)-3-methylpyridin-2-yl)benzoic acid is desired for process purposes, an elevated temperature may be used. After stirring the solution for an effective amount of time at the elevated temperature, recrystallization upon cooling provides substantially pure Compound 1 Form I. In one embodiment, substantially pure refers to greater than about 90% purity. In another embodiment, substantially pure refers to greater than about 95% purity. In another embodiment, substantially pure refers to greater than about 98% purity. In another embodiment, substantially pure refers to greater than about 99% purity. The temperature selected depends in part on the solvent used and is well within the determination capabilities of one of ordinary skill in the art. In one embodiment, the temperature is between room temperature and about 80 °C. In another embodiment, the temperature is between room temperature and about 40 °C. In another embodiment, the temperature is between about 40 °C and about 60 °C. In another embodiment, the temperature is between about 60 °C and about 80 °C.

Compound 1 Form I may also be formed directly from 3-(6-(1-(2,2-difluorobenzo[d][1,3]dioxol-5-yl) cyclopropanecarboxamido)-3-methylpyridin-2-yl)-t-butylbenzoate (cf. Scheme 3), which is a precursor to the salt of Compound 1. Thus, 3-(6-(1-(2,2-difluorobenzo[d][1,3]dioxol-5-yl) cyclopropanecarboxamido)-3-methylpyridin-2-yl)-t-butylbenzoate is allowed to undergo reaction with an appropriate acid, such as, for example, formic acid under appropriate reaction conditions to give Compound 1 Form I.

Compound 1 Form I may be further purified by recrystallization from an organic solvent. Examples of organic solvents include toluene, cumene, anisol, 1-butanol, isopropyl acetate, butyl acetate, isobutyl acetate, methyl *t*-butyl ether, methyl isobutyl ketone and 1-propanol-water mixtures. The temperature may be as described above. For example, Compound 1 Form I is dissolved in 1-butanol at 75 °C until it is completely dissolved. Cooling down the solution to 10 °C at a rate of 0.2 °C/min yields crystals of Compound 1 Form I which may be isolated by filtration.

Compound 1 Form I is characterized by one or more peaks at 15.4, 16.3, and 14.5 degrees. In another embodiment, Compound 1 Form I is further characterized by a peak at 14.6 to 15.0 degrees. In another embodiment, Compound 1 Form I is further characterized by a peak at 14.8 degrees. In another embodiment, Compound 1 Form I is further characterized by a peak at 17.6 to 18.0 degrees. In another embodiment, Compound 1 Form I is further characterized by a peak at 17.8 degrees. In another embodiment, Compound 1 Form I is further characterized by a peak at 16.4 to 16.8 degrees. In another embodiment, Compound 1 Form I is further characterized by a peak at 16.4 to 16.8 degrees. In another embodiment, Compound 1 Form I is further characterized by a peak at 16.6 degrees. In another embodiment, Compound 1 Form I is further characterized by a peak at 7.6 to 8.0 degrees. In another embodiment, Compound 1 Form I is further characterized by a peak at 7.8 degrees. In another embodiment, Compound 1 Form I is further characterized by a peak at 25.8 to 26.2 degrees. In another embodiment, Compound 1 Form I is further characterized by a peak at 26.0 degrees. In another embodiment, Compound 1 Form I is further characterized by a peak at 21.4 to 21.8 degrees. In another embodiment, Compound 1 Form I is further characterized by a peak at 21.6 degrees. In another embodiment, Compound 1 Form I is further characterized by a peak at 23.1 to 23.5 degrees. In another embodiment, Compound 1 Form I is further characterized by a peak at 23.3 degrees. In some embodiments, Compound 1 Form I is characterized by a diffraction pattern substantially similar to that of Figure 1. In some embodiments, Compound 1 Form I is characterized by a diffraction pattern substantially similar to that of Figure 2.

In some embodiments, the particle size distribution of D90 is about 82 μm or less for Compound 1 Form I. In some embodiments, the particle size distribution of D50 is about 30 μm or less for Compound 1 Form I.

## Compound 2

Compound 2 is the starting point for the solid dispersion comprising substantially amorphous Compound 2 and can be prepared by coupling a 4-oxo-dihydroquinoline carboxylic acid moiety with an amine moiety according to Schemes

5-7.

**Scheme 5: Synthesis of the 4-oxo- dihydroquinoline carboxylic acid moiety.**

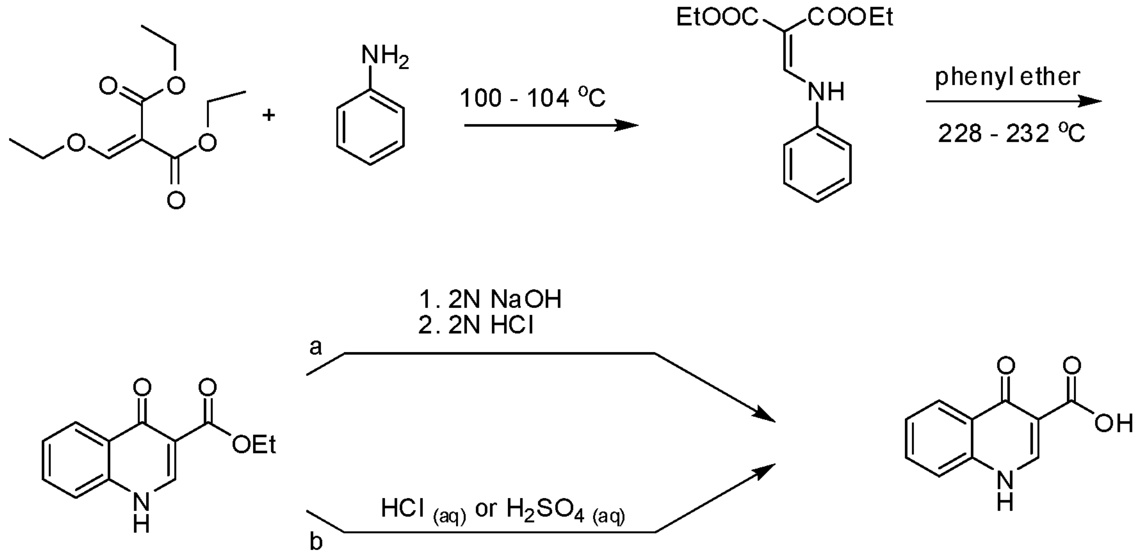


**Scheme 6: Synthesis of the amine moiety.**

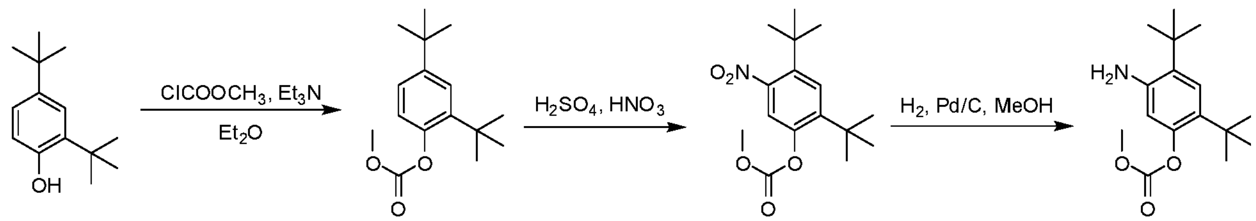


Scheme 7: Coupling of the 4-oxo-dihydroquinoline carboxylic acid moiety with the amine moiety.

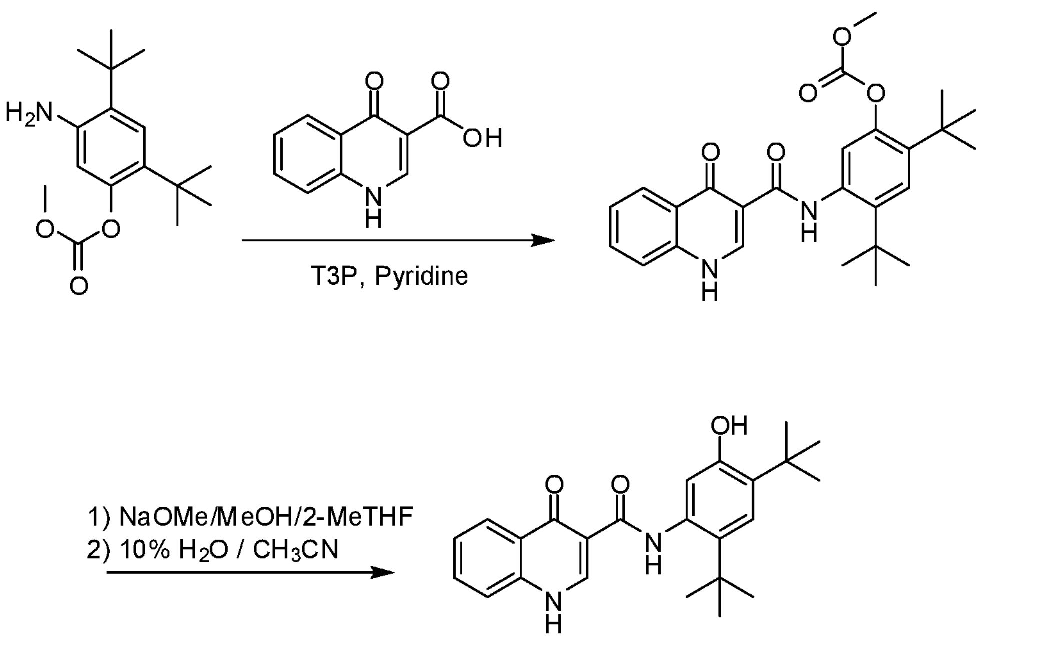


**Compound 2**

Solid Dispersion Comprising Substantially Amorphous Compound 2

Starting from Compound 2 the amorphous form of Compound 2 may be prepared by spray dried methods. Spray drying is a process that converts a liquid feed to a dried particulate form. Optionally, a secondary drying process such as fluidized bed drying or vacuum drying, may be used to reduce residual solvents to pharmaceutically acceptable levels. Typically, spray drying involves contacting a highly dispersed liquid suspension or solution, and a sufficient volume of hot air to produce evaporation and drying of the liquid droplets. The preparation to be spray dried can be any solution, coarse suspension, slurry, colloidal dispersion, or paste that may be atomized using the selected spray drying apparatus. In a standard procedure, the preparation is sprayed into a current of warm filtered air that evaporates the solvent and conveys the dried product to a collector (e.g. a cyclone). The spent air is then exhausted with the solvent, or alternatively the spent air is sent to a condenser to capture and potentially recycle the solvent. Commercially available types of apparatus may be used to conduct the spray drying. For example, commercial spray dryers are manufactured by Buchi Ltd. And Niro (e.g., the PSD line of spray driers manufactured by Niro) (see, US 2004/0105820; US 2003/0144257).

Spray drying typically employs solid loads of material from about 3% to about 30% by weight, (i.e., drug and excipients), for example about 4% to about 20% by weight, preferably at least about 10%. In general, the upper limit of solid loads is governed by the viscosity of (e.g., the ability to pump) the resulting solution and the solubility of the components in the solution. Generally, the viscosity of the solution can determine the size of the particle in the resulting powder product.

Techniques and methods for spray drying may be found in Perry's Chemical Engineering Handbook, 6th Ed., R. H. Perry, D. W. Green & J. O. Maloney, eds.), McGraw-Hill book co. (1984); and Marshall "Atomization and Spray-Drying" 50, Chem. Eng. Prog. Monogr. Series 2 (1954). In general, the spray drying is conducted with an inlet temperature of from about 60 °C to about 200 °C, for example, from about 95 °C to about 185 °C, from about 110 °C to about 182 °C, from about 96 °C to about 180 °C, e.g., about 145 °C. The spray drying is generally conducted with an outlet temperature of from about 30 °C to about 90 °C, for example from about 40 °C to about 80 °C, about 45 °C to about 80 °C e.g., about 75 °C. The atomization flow rate is generally from about 4 kg/h to about 12 kg/h, for example, from about 4.3 kg/h to about 10.5 kg/h, e.g., about 6 kg/h or about 10.5 kg/h. The feed flow rate is generally from about 3 kg/h to about 10 kg/h, for example, from about 3.5 kg/h to about 9.0 kg/h, e.g., about 8 kg/h or about 7.1 kg/h. The atomization ratio is generally from about 0.3 to 1.7, e.g., from about 0.5 to 1.5, e.g., about 0.8 or about 1.5.

Removal of the solvent may require a subsequent drying step, such as tray drying, fluid bed drying (e.g., from about room temperature to about 100 °C), vacuum drying, microwave drying, rotary drum drying or biconical vacuum drying (e.g., from about room temperature to about 200 °C).

In one embodiment, the spray dried dispersion is fluid bed dried.

In one process, the solvent includes a volatile solvent, for example a solvent having a boiling point of less than about 100 °C. In some embodiments, the solvent includes a mixture of solvents, for example a mixture of volatile solvents or a mixture of volatile and non-volatile solvents. Where mixtures of solvents are used, the mixture can include one or more non-volatile solvents, for example, where the non-volatile solvent is present in the mixture at less than about 15%, e.g., less than about 12%, less than about 10%, less than about 8%, less than about 5%, less than about 3%, or less than about 2%.

Preferred solvents are those solvents where Compound 2 has a solubility of at least about 10 mg/ml, (e.g., at least about 15 mg/ml, 20 mg/ml, 25 mg/ml, 30 mg/ml, 35 mg/ml, 40 mg/ml, 45 mg/ml, 50 mg/ml, or greater). More preferred solvents include those where Compound 2 has a solubility of at least about 20 mg/ml.

Exemplary solvents that could be tested include acetone, cyclohexane, dichloromethane, N,N-dimethylacetamide (DMA), N,N-dimethylformamide (DMF), 1,3-dimethyl-2-imidazolidinone (DMI), dimethyl sulfoxide (DMSO), dioxane, ethyl acetate, ethyl ether, glacial acetic acid (HAc), methyl ethyl ketone (MEK), N-methyl-2-pyrrolidinone (NMP), methyl tert-butyl ether (MTBE), tetrahydrofuran (THF), pentane, acetonitrile, methanol, ethanol, isopropyl alcohol, isopropyl acetate, and toluene. Exemplary co-solvents include acetone/DMSO, acetone/DMF, acetone/water, MEK/water, THF/water, dioxane/water. In a two solvent system, the solvents can be present in of from about 0.1% to about 99.9%. In some preferred embodiments, water is a co-solvent with acetone where water is present from about 0.1% to about 15%, for example about 9% to about 11%, e.g., about 10%. In some preferred embodiments, water is a co-solvent with MEK where water is present from about 0.1% to about 15%, for example about 9% to about 11%, e.g., about 10%. In some embodiments the solvent solution include three solvents. For example, acetone and water can be mixed with a third solvent such as DMA, DMF, DMI, DMSO, or HAc. In instances where substantially amorphous Compound 2 is a component of a solid dispersion, preferred solvents dissolve both Compound 2 and the polymer. Suitable solvents include those described above, for example, MEK, acetone, water, methanol, and mixtures thereof.

The particle size and the temperature drying range may be modified to prepare an optimal spray dry dispersion. As would be appreciated by skilled practitioners, a small particle size would lead to improved solvent removal. Applicants have found however, that smaller particles can lead to fluffy particles that, under some circumstances do not provide optimal spray dry dispersions for downstream processing such as tableting. At higher temperatures, crystallization or

chemical degradation of substantially amorphous Compound 2 may occur. At lower temperatures, a sufficient amount of the solvent may not be removed. The methods herein provide an optimal particle size and an optimal drying temperature.

In general, particle size is such that D10 (μm) is less than about 5, e.g., less than about 4.5, less than about 4.0, or less than about 3.5, D50 (μm) is generally less than about 17, e.g., less than about 16, less than about 15, less than about 14, less than about 13, and D90 (μm) is generally less than about 175, e.g., less than about 170, less than about 170, less than about 150, less than about 125, less than about 100, less than about 90, less than about 80, less than about 70, less than about 60, or less than about less than about 50. In general bulk density of the spray dried particles is from about 0.08 g/cc to about 0.20 g/cc, e.g., from about 0.10 to about 0.15 g/cc, e.g., about 0.11 g/cc or about 0.14 g/cc. Tap density of the spray dried particles generally ranges from about 0.08 g/cc to about 0.20 g/cc, e.g., from about 0.10 to about 0.15 g/cc, e.g., about 0.11 g/cc or about 0.14 g/cc, for 10 taps; 0.10 g/cc to about 0.25 g/cc, e.g., from about 0.11 to about 0.21 g/cc, e.g., about 0.15 g/cc, about 0.19 g/cc, or about 0.21 g/cc for 500 taps; 0.15 g/cc to about 0.27 g/cc, e.g., from about 0.18 to about 0.24 g/cc, e.g., about 0.18 g/cc, about 0.19 g/cc, about 0.20 g/cc, or about 0.24 g/cc for 1250 taps; and 0.15 g/cc to about 0.27 g/cc, e.g., from about 0.18 to about 0.24 g/cc, e.g., about 0.18 g/cc, about 0.21 g/cc, about 0.23 g/cc, or about 0.24 g/cc for 2500 taps.

## Polymers

Spray dried dispersions including amorphous Compound 2 and a polymer (or solid state carrier) also are included herein. For example, Compound 2 is present as an amorphous compound as a component of a solid amorphous dispersion. The solid amorphous dispersion, generally includes substantially amorphous Compound 2 and a polymer. Exemplary polymers include cellulosic polymers such as HPMC or HPMCAS and pyrrolidone containing polymers such as PVP/VA. In some embodiments, the solid amorphous dispersion includes one or more additional excipients, such as a surfactant.

In one embodiment, a polymer is able to dissolve in aqueous media. The solubility of the polymers may be pH-independent or pH-dependent. The latter include one or more enteric polymers. The term "enteric polymer" refers to a polymer that is preferentially soluble in the less acidic environment of the intestine relative to the more acid environment of the stomach, for example, a polymer that is insoluble in acidic aqueous media but soluble when the pH is above 5-6. An appropriate polymer should be chemically and biologically inert. In order to improve the physical stability of the spray dry dispersions, the glass transition temperature ($T_g$) of the polymer should be as high as possible. For example, preferred polymers have a glass transition temperature at least equal to or greater than the glass transition temperature of the drug (i.e., Compound 2). Other preferred polymers have a glass transition temperature that is within about 10 to about 15 °C of the drug (i.e., Compound 2). Examples of suitable glass transition temperatures of the polymers include at least about 90 °C, at least about 95 °C, at least about 100 °C, at least about 105 °C, at least about 110 °C, at least about 115 °C, at least about 120 °C, at least about 125 °C, at least about 130 °C, at least about 135 °C, at least about 140 °C, at least about 145 °C, at least about 150 °C, at least about 155 °C, at least about 160 °C, at least about 165 °C, at least about 170 °C, or at least about 175 °C (as measured under dry conditions). Without wishing to be bound by theory, it is believed that the underlying mechanism is that a polymer with a higher $T_g$ generally has lower molecular mobility at room temperature, which can be a crucial factor in stabilizing the physical stability of the amorphous spray dry dispersion.

Additionally, the hygroscopicity of the polymers should be as low, e.g., less than about 10%. For the purpose of comparison in this application, the hygroscopicity of a polymer or composition is characterized at about 60% relative humidity. In some preferred embodiments, the polymer has less than about 10% water absorption, for example less than about 9%, less than about 8%, less than about 7%, less than about 6%, less than about 5%, less than about 4%, less than about 3%, or less than about 2% water absorption. The hygroscopicity can also affect the physical stability of the spray dry dispersions. Generally, moisture adsorbed in the polymers can greatly reduce the $T_g$ of the polymers as well as the resulting spray dry dispersions, which will further reduce the physical stability of the spray dry dispersions as described above.

In one embodiment, the polymer is one or more water-soluble polymer(s) or partially water-soluble polymer(s). Water-soluble or partially water-soluble polymers include cellulose derivatives (e.g., hydroxypropylmethylcellulose (HPMC), hydroxypropylcellulose (HPC)) or ethylcellulose; polyvinylpyrrolidones (PVP); polyethylene glycols (PEG); polyvinyl alcohols (PVA); acrylates, such as polymethacrylate (e.g., Eudragit® E); cyclodextrins (e.g., β-cyclodextin) and copolymers and derivatives thereof, including for example PVP-VA (polyvinylpyrollidone-vinyl acetate).

In some embodiments, the polymer is hydroxypropylmethylcellulose (HPMC), such as HPMCAS, HPMC E50, HPMCE15, or HPMC60SH50).

As discussed herein, the polymer can be a pH-dependent enteric polymer. Such pH-dependent enteric polymers include cellulose derivatives (e.g., cellulose acetate phthalate (CAP)), hydroxypropyl methyl cellulose phthalates (HPMCP), hydroxypropyl methyl cellulose acetate succinate (HPMCAS), carboxymethylcellulose (CMC) or a salt thereof (e.g., a sodium salt such as (CMC-Na)); cellulose acetate trimellitate (CAT), hydroxypropylcellulose acetate phthalate (HPCAP), hydroxypropylmethyl-cellulose acetate phthalate (HPMCAP), and methylcellulose acetate phthalate (MCAP),

or polymethacrylates (e.g., Eudragit® S). In some embodiments, the polymer is hydroxypropyl methyl cellulose acetate succinate (HPMCAS). In some embodiments, the polymer is hydroxypropyl methyl cellulose acetate succinate HG grade (HPMCAS-HG).

In yet another embodiment, the polymer is a polyvinylpyrrolidone co-polymer, for example, a vinylpyrrolidone/vinyl acetate co-polymer (PVP/VA).

In embodiments where Compound 2 forms a spray dry dispersion with a polymer, for example with an HPMC, HPMCAS, or PVP/VA polymer, the amount of polymer relative to the total weight of the spray dry dispersion ranges from about 0.1% to 99% by weight. Unless otherwise specified, percentages of drug, polymer and other excitpients as described within a dispersion are given in weight percentages. The amount of polymer is typically at least about 20%, and preferably at least about 30%, for example, at least about 35%, at least about 40%, at least about 45%, or about 50% (e.g., 49.5%). The amount is typically about 99% or less, and preferably about 80% or less, for example about 75% or less, about 70% or less, about 65% or less, about 60% or less, or about 55% or less. In one embodiment, the polymer is in an amount of up to about 50% of the total weight of the dispersion (and even more specifically, between about 40% and 50%, such as about 49%, about 49.5%, or about 50%). HPMC and HPMCAS are available in a variety of grades from ShinEtsu, for example, HPMCAS is available in a number of varieties, including AS-LF, AS-MF, AS-HF, AS-LG, AS-MG, AS-HG. Each of these grades vary with the percent substitution of acetate and succinate.

In some embodiments, substantially amorphous Compound 2 and polymer are present in roughly equal amounts, for example each of the polymer and the drug make up about half of the percentage weight of the dispersion. For example, the polymer is present in about 49.5% and the drug is present in about 50%.

In some embodiments, substantially amorphous Compound 2 and the polymer combined represent 1% to 20% w/w total solid content of the non-spray dry dispersion prior to spray drying. In some embodiments, substantially amorphous Compound 2 and the polymer combined represent 5% to 15% w/w total solid content of the non-spray dry dispersion prior to spray drying. In some embodiments, substantially amorphous Compound 2 and the polymer combined represent about 11% w/w total solid content of the non-spray dry dispersion prior to spray drying.

In some embodiments, the dispersion further includes other minor ingredients, such as a surfactant (e.g., SLS). In some embodiments, the surfactant is present in less than about 10% of the dispersion, for example less than about 9%, less than about 8%, less than about 7%, less than about 6%, less than about 5%, less than about 4%, less than about 3%, less than about 2%, about 1%, or about 0.5%.

In embodiments including a polymer, the polymer should be present in an amount effective for stabilizing the spray dry dispersion. Stabilizing includes inhibiting or preventing, the crystallization of substantially amorphous Compound 2. Such stabilizing would inhibit the conversion Compound 2 from amorphous to crystalline form. For example, the polymer would prevent at least a portion (e.g., about 5%, about 10%, about 15%, about 20%, about 25%, about 30%, about 35%, about 40%, about 45%, about 50%, about 55%, about 60%, about 65%, about 70%, about 75%, or greater) of Compound 2 from converting from an amorphous to a crystalline form. Stabilization can be measured, for example, by measuring the glass transition temperature of the spray dry dispersion, measuring the rate of relaxation of the amorphous material, or by measuring the solubility or bioavailability of Compound 2.

Suitable polymers for use in combination with Compound 2, for example to form a spray dry dispersion such as an amorphous spray dry dispersion, should have one or more of the following properties:

The glass transition temperature of the polymer should have a temperature of no less than about 10-15 °C lower than the glass transition temperature of substantially amorphous Compound 2. Preferably, the glass transition temperature of the polymer is greater than the glass transition temperature of substantially amorphous Compound 2, and in general at least 50°C higher than the desired storage temperature of the drug product. For example, at least about 100 °C, at least about 105 °C, at least about 105 °C, at least about 110 °C, at least about 120 °C, at least about 130 °C, at least about 140 °C, at least about 150 °C, at least about 160 °C, at least about 160 °C, or greater.

The polymer should be relatively non-hygroscopic. For example, the polymer should, when stored under standard conditions, absorb less than about 10% water, for example, less than about 9%, less than about 8%, less than about 7%, less than about 6%, or less than about 5%, less than about 4%, or less than about 3% water. Preferably the polymer will, when stored under standard conditions, be substantially free of absorbed water.

The polymer should have similar or better solubility in solvents suitable for spray drying processes relative to that of Compound 2. In preferred embodiments, the polymer will dissolve in one or more of the same solvents or solvent systems as Compound 2. It is preferred that the polymer is soluble in at least one non-hydroxy containing solvent such as methylene chloride, acetone, or a combination thereof.

The polymer, when combined with substantially amorphous Compound 2, for example in a spray dry dispersion or in a liquid suspension, should increase the solubility of Compound 2 in aqueous and physiologically relative media either relative to the solubility of Compound 2 in the absence of polymer or relative to the solubility of Compound 2 when combined with a reference polymer. For example, the polymer could increase the solubility of amorphous Compound 2 by reducing the amount of amorphous Compound 2 that converts to crystalline Compound 2, either from a solid amorphous dispersion or from a liquid suspension.

The polymer should decrease the relaxation rate of the amorphous substance.

The polymer should increase the physical and/or chemical stability of substantially amorphous Compound 2.

The polymer should improve the manufacturability of substantially amorphous Compound 2.

The polymer should improve one or more of the handling, administration or storage properties of substantially amorphous Compound 2.

The polymer should not interact unfavorably with other pharmaceutical components, for example excipients.

The suitability of a candidate polymer (or other component) can be tested using the spray drying methods (or other methods) described herein to form an amorphous composition. The candidate composition can be compared in terms of stability, resistance to the formation of crystals, or other properties, and compared to a reference preparation, e.g., a preparation of neat amorphous Compound 2 or crystalline Compound 2. For example, a candidate composition could be tested to determine whether it inhibits the time to onset of solvent mediated crystallization, or the percent conversion at a given time under controlled conditions, by at least 50 %, 75 %, 100%, or 110% as well as the reference preparation, or a candidate composition could be tested to determine if it has improved bioavailability or solubility relative to crystalline Compound 2.

Surfactants

The spray dry dispersion may include a surfactant. A surfactant or surfactant mixture would generally decrease the interfacial tension between the spray dry dispersion and an aqueous medium. An appropriate surfactant or surfactant mixture may also enhance aqueous solubility and bioavailability of Compound 2 from a spray dry dispersion. The surfactants for use in connection with the present invention include sorbitan fatty acid esters (e.g., Spans®), polyoxyethylene sorbitan fatty acid esters (e.g., Tweens®), sodium lauryl sulfate (SLS), sodium dodecylbenzene sulfonate (SDBS) dioctyl sodium sulfosuccinate (Docusate), dioxycholic acid sodium salt (DOSS), Sorbitan Monostearate, Sorbitan Tristearate, hexadecyltrimethyl ammonium bromide (HTAB), Sodium N-lauroylsarcosine, Sodium Oleate, Sodium Myristate, Sodium Stearate, Sodium Palmitate, Gelucire 44/14, ethylenediamine tetraacetic acid (EDTA), Vitamin E d-alpha tocopheryl polyethylene glycol 1000 succinate (TPGS), Lecithin, MW 677-692, Glutanic acid monosodium monohydrate, Labrasol, PEG 8 caprylic/capric glycerides, Transcutol, diethylene glycol monoethyl ether, Solutol HS-15, polyethylene glycol/hydroxystearate, Taurocholic Acid, Pluronic F68, Pluronic F108, and Pluronic F127 (or any other polyoxyethylene-polyoxypropylene co-polymers (Pluronics®) or saturated polyglycolized glycerides (Gelucirs®)). Specific example of such surfactants that may be used in connection with this invention include Span 65, Span 25, Tween 20, Capryol 90, Pluronic F108, sodium lauryl sulfate (SLS), Vitamin E TPGS, pluronics and copolymers. SLS is generally preferred.

The amount of the surfactant (e.g., SLS) relative to the total weight of the spray dry dispersion may be between 0.1-15%. Preferably, it is from about 0.5% to about 10%, more preferably from about 0.5 to about 5%, e.g., about 0.5 to 4%, about 0.5 to 3%, about 0.5 to 2%, about 0.5 to 1%, or about 0.5%.

In certain embodiments, the amount of the surfactant relative to the total weight of the spray dry dispersion is at least about 0.1%, preferably about 0.5%. In these embodiments, the surfactant would be present in an amount of no more than about 15%, and preferably no more than about 12%, about 11%, about 10%, about 9%, about 8%, about 7%, about 6%, about 5%, about 4%, about 3%, about 2% or about 1%. An embodiment wherein the surfactant is in an amount of about 0.5% by weight is preferred.

Candidate surfactants (or other components) can be tested for suitability for use in the invention in a manner similar to that described for testing polymers.

**METHODS FOR MAKING THE PHARMACEUTICAL COMPOSITIONS**

As used herein, "tablet" includes compressed pharmaceutical dosage unit forms of all shapes and sizes, whether coated or uncoated.

The term "tablet" as used herein refers to a physically discrete unit of agent appropriate for the patient to be treated. In general, a compacted mixture has a density greater than that of the mixture prior to compaction. A dosage tablet of the invention can have almost any shape including concave and/or convex faces, rounded or angled corners, and a rounded to rectilinear shape. In some embodiments, the compressed tablets of the invention comprise a rounded tablet having flat faces. In particular embodiments, the formulations provided herein may be prepared using conventional methods known to those skilled in the field of pharmaceutical formulation, as described, e.g., in pertinent textbooks. See, e.g., Remington: The Science and Practice of Pharmacy, 21st Ed., Lippincott Williams & Wilkins, Baltimore, Md. (2003); Ansel et al., Pharmaceutical Dosage Forms And Drug Delivery Systems, 7th Edition, Lippincott Williams & Wilkins, (1999); The Handbook of Pharmaceutical Excipients, 4th edition, Rowe et al., Eds., American Pharmaceuticals Association (2003); Gibson, Pharmaceutical Preformulation And Formulation, CRC Press (2001).

Granulation and Compression

In some embodiments, the ingredients are weighed according to the formula set herein. Next, all of the intragranular ingredients are sifted and mixed well. The ingredients can be lubricated with a suitable lubricant, for example, magnesium stearate. The next step can comprise compaction/slugging of the powder admixture and sized ingredients. Next, the compacted or slugged blends are milled into granules and sifted to obtain the desired size. Next, the granules can be further lubricated with, for example, magnesium stearate. Next the granular composition disclosed herein can be compressed on suitable punches into various tablets in accordance with the invention. Optionally the tablets can be coated with a film, colorant or other coating.

In another embodiment, a wet granulation process is performed to yield the tablet of the invention from an admixture of powdered and liquid ingredients. For example, a tablet comprising an admixture of a composition comprising Compound 1 Form I, a solid dispersion comprising substantially amorphous Compound 2, and one or more excipients selected from: a filler, a binder, a surfactant, or a disintegrant, are weighed as per the formula set herein. Next, all of the intragranular ingredients are sifted and mixed in a high shear or low shear granulator using water or water with a surfactant or water with a binder or water with a surfactant and a binder to granulate the powder blend. A fluid other than water can also be used with or without surfactant and/or binder to granulate the powder blend. Next, the wet granules can optionally be milled using a suitable mill. Next, water may optionally be removed from the admixture by drying the ingredients in any suitable manner. Next, the dried granules can optionally be milled to the required size. Next, extra granular excipients can be added by blending (for example a filler, a diluent, and a disintegrant). Next, the sized granules can be further lubricated with magnesium stearate and a disintegrant, for example, croscarmellose sodium. Next the granular composition disclosed herein can be sifted for sufficient time to obtain the correct size and then compressed on suitable punches into various tablets in accordance with the invention. Optionally, the tablets can be coated with a film, colorant or other coating. Surprisingly, wet granulation can be carried out without substantial loss of the solid state forms of Compound 1 Form I or substantially amorphous Compound 2.

In a particularly favored embodiment, the tablets of the present invention are prepared by a continuous twin screw wet granulation (TSWG) process. Continuous manufacturing delivers high quality and highly consistent product with on-line monitoring and control. Continuous manufacturing also facilitates quality by design development with a "data rich" design space and an easier to understand impact of upstream variables on the downstream process and final product quality. In addition, the tablets of the present invention can be finalized early on commercial scale equipment which avoids scale-up risks and formulation changes late in development. Finally, continuous manufacturing has commercial manufacturing advantages such as improved process control, reduced product handling, and real time release efficiencies. The overall result is a more robust, controllable, and scalable process that has fewer process checks resulting in increased product quality and therefore greater patient safety. These advantages address Janet Woodcock's (director of the Center for Drug Evaluation and Research (CDER)) concerns that chemistry, manufacturing, and controls (CMC) won't be able to keep up with rapid clinical development of highly effective therapies ("What we are seeing is that often the rate limiting step is going to be manufacturing," July 24, 2013 Friends of Cancer hosted congressional briefing "Answering a Compelling Need: Expediting Life-Saving Treatments to Patients" to discuss the Food and Drug Administration's Breakthrough Therapy Designation).

For example, high shear granulation (HSG), a common granulation technique is well known for the risk of over-granulation and poor process control. Scale-up of this process is very challenging and involves significant risk. Changing from a HSG process to a continuous TSWG process, allows scale-up using the same equipment to produce different batch sizes, by running for a longer time. This eliminates the scale-up risk commonly encountered with other granulation processes. Additionally, it was found that the TSWG process is more robust, being less sensitive to over-granulation. As can be seen in Figure 3 for a Compound 1 tablet, the HSG process showed significant dissolution slow-down with increasing water content, while the TSWG process did not show a change for a similar range of water addition. Surprisingly, no performance changes were found with the tablet formulations comprising Compound 1 between 45-55 percent by weight and the tablet formulations comprising Compound 1 between 60-70 percent by weight using the twin screw wet granulation process. This was not the case with the HSG process. Additionally, this continuous and increased product quality process addresses a common complaint by the FDA regarding the lack of drug availability for patients in need thereof.

In one embodiment the continuous process starts with feeding individual excipients, Compound 1, and Compound 2 into a continuous in-line blender through loss-in-weight feeding. From this blender, the material is continuously conveyed and processed through twin screw wet granulation, drying, milling, extra-granular excipient addition, blending, compression and film coating.

For example, in one embodiment, a tablet comprising Compound 1 and Compound 2 may be prepared continuously according to the below flow chart.

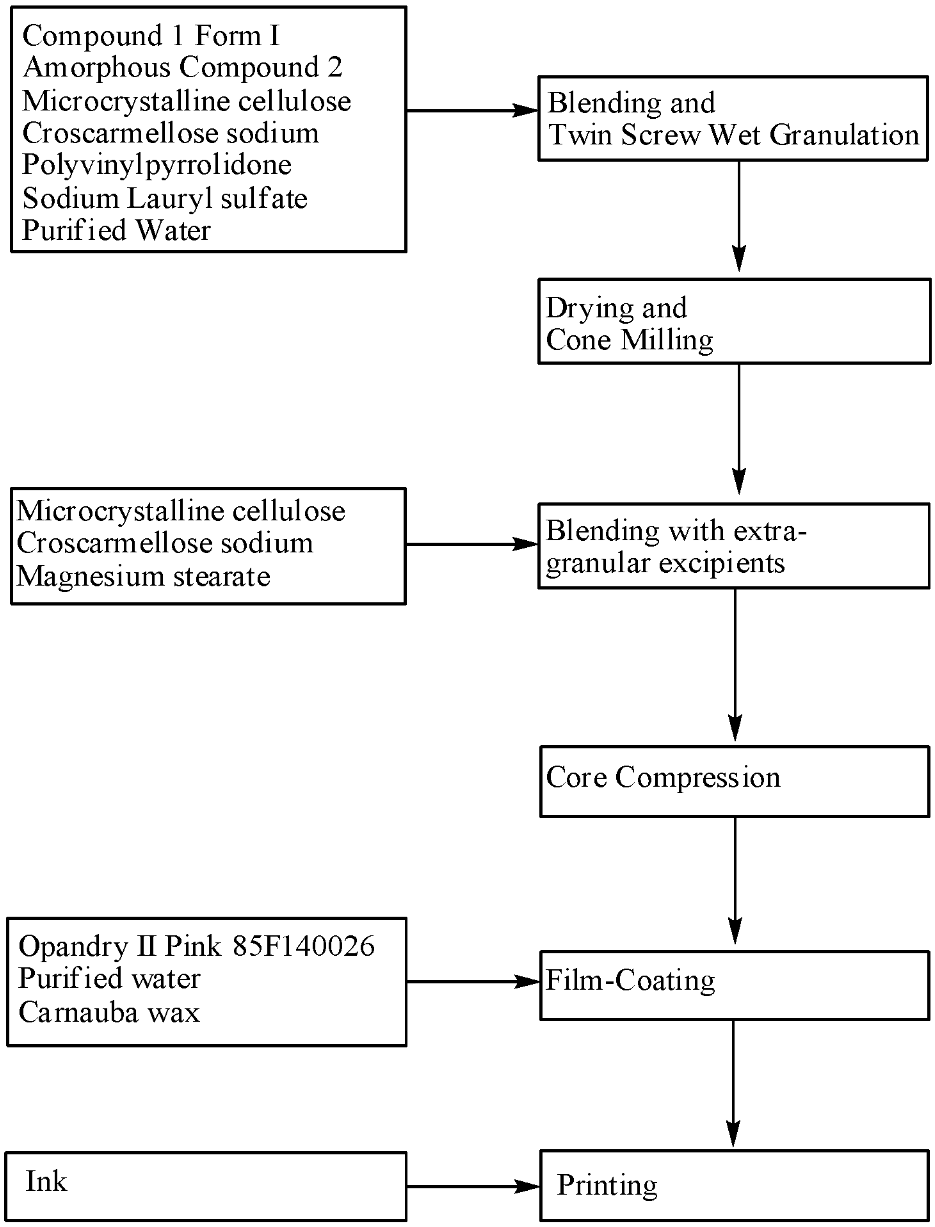


Each of the ingredients of this exemplary admixture is described above and in the Examples below. Furthermore, the admixture can comprise optional additives, such as, one or more colorants, one or more flavors, and/or one or more fragrances as described above and in the Examples below. In some embodiments, the relative concentrations (e.g., wt%) of each of these ingredients (and any optional additives) in the admixture are also presented above and in the Examples below. The ingredients constituting the admixture can be provided sequentially or in any combination of additions; and, the ingredients or combination of ingredients can be provided in any order. In one embodiment, the lubricant is the last component added to the admixture.

In another embodiment, the admixture comprises a composition of Compound 1 Form I, a solid dispersion of substantially amorphous Compound 2, and any one or more of the excipients; a binder, a surfactant, a diluent, a lubricant, a disintegrant, and a filler, wherein each of these ingredients is provided in a powder form (e.g., provided as particles having a mean or average diameter, measured by light scattering, of 250 μm or less (e.g., 150 μm or less, 100 μm or less, 50 μm or less, 45 μm or less, 40 μm or less, or 35 μm or less)).

In another embodiment, compressing the admixture into a tablet is accomplished by filling a form (e.g., a mold) with the admixture and applying pressure to admixture. This can be accomplished using a die press or other similar apparatus. In some embodiments, the admixture of Compound 1 Form I, a solid dispersion comprising substantially amorphous Compound 2, and excipients can be first processed into granular form. The granules can then be sized and compressed into tablets or formulated for encapsulation according to known methods in the pharmaceutical art. It is also

noted that the application of pressure to the admixture in the form can be repeated using the same pressure during each compression or using different pressures during the compressions. In another example, the admixture of powdered ingredients or granules can be compressed using a die press that applies sufficient pressure to form a tablet having a dissolution of about 50% or more at about 30 minutes (e.g., about 55% or more at about 30 minutes or about 60% or more at about 30 minutes). For instance, the admixture is compressed using a die press to produce a tablet hardness of at least about 5 kP (at least about 5.5 kP, at least about 6 kP, at least about 7 kP, at least about 10 kP, or at least 15 kP). In some instances, the admixture is compressed to produce a tablet hardness of between about 5 and 20 kP.

In some embodiments, tablets of the invention can be coated with about 3.0 wt% of a film coating comprising a colorant by weight of the tablet. In certain instances, the colorant suspension or solution used to coat the tablets comprises about 20%w/w of solids by weight of the colorant suspension or solution. In still further instances, the coated tablets can be labeled with a logo, other image or text.

In another embodiment, the method for producing a tablet comprises providing an admixture of a solid forms, e.g. an admixture of powdered and/or liquid ingredients, the admixture comprising Compound 1 Form I, a solid dispersion comprising substantially amorphous Compound 2, and one or more excipients selected from: a binder, a diluent, a surfactant, a lubricant, a disintegrant, and a filler; mixing the admixture until the admixture is substantially homogenous, and compressing or compacting the admixture into a granular form. Then the granular composition comprising Compound 1 Form I and a solid dispersion comprising substantially amorphous Compound 2 can be compressed into tablets or formulated into capsules as described above or in the Examples below. Alternatively, methods for producing a tablet comprises providing an admixture of Compound 1 Form I, a solid dispersion comprising substantially amorphous Compound 2, and one or more excipients, e.g. a binder, a diluent, a surfactant, a lubricant, a disintegrant, and a filler; mixing the admixture until the admixture is substantially homogenous, and compressing/compacting the admixture into a granular form using a high shear wet granule compaction process as set forth in the Examples below. A tablet as described herein, can be made using the granules prepared incorporating Compound 1 Form I and a solid dispersion comprising substantially amorphous Compound 2 in addition to the selected excipients described herein.

In some embodiments, the admixture is mixed by stirring, blending, or shaking, using hand mixing, a mixer, a blender, or any combination thereof. When ingredients or combinations of ingredients are added sequentially, mixing can occur between successive additions, continuously throughout the ingredient addition, after the addition of all of the ingredients or combinations of ingredients, or any combination thereof. The admixture is mixed until it has a substantially homogenous composition.

In another embodiment, the present invention comprises jet milling a pharmaceutical composition comprising Compound 1 Form I and a solid dispersion comprising substantially amorphous Compound 2 in a suitable, conventional milling apparatus using air pressure suitable to produce particles having a significant particle size fraction between 0.1 microns and 50 microns. In another embodiment, the particle size is between 0.1 microns and 20 microns. In another embodiment, the particles size is between 0.1 microns and 10 microns. In another embodiment, the particle size is between 1.0 microns and 5 microns. In still another embodiment, the pharmaceutical composition has a particle size D50 of 2.0 microns.

The formulations of the present invention provide a fixed dosage of two APIs for the effective treatment of cystic fibrosis, a combination that has received one of only two Breakthrough Therapy Designation from the FDA, and does so with surprising stability as measured by the small loss of the amorphous solid form of Compound 2. Figure 4 depicts the small amount of crystallinity of Compound 2 over time in PC-XVII at 50 °C after pre-equilibration at 60% relative humidity. Even after close to 1000 hours under these conditions, less than 5% by weight of Compound 2 has crystallized. Figure 5 shows for PC-XVII that even at the higher temperature of 60 °C after pre-equilibrating at 60% relative humidity, at close to 1000 hours under these conditions, less than 10% by weight of Compound 2 has crystallized. Figures 6 and 7 show similar results for PC-XIX. The present formulations, therefore, provide the convenience of a fixed dosage of two breakthrough API's in a surprisingly stable tablet. Such tablets increase patient compliance which directly relates to the effective treatment of diseases.

Dosage forms prepared as above can be subjected to in vitro dissolution evaluations according to Test 711 "Dissolution" in United States Pharmacopoeia 29, United States Pharmacopeial Convention, Inc., Rockville, Md., 2005 ("USP"), to determine the rate at which the active substance is released from the dosage forms. The content of active substance and the impurity levels are conveniently measured by techniques such as high performance liquid chromatography (HPLC).

In some embodiments, the invention includes use of packaging materials such as containers and closures of high-density polyethylene (HDPE), low-density polyethylene (LDPE) and or polypropylene and/or glass, glassine foil, aluminum pouches, and blisters or strips composed of aluminum or high-density polyvinyl chloride (PVC), optionally including a desiccant, polyethylene (PE), polyvinylidene dichloride (PVDC), and PVC/PE/PVDC. These package materials can be used to store the various tablets in a sterile fashion after appropriate sterilization of the package and its contents using chemical or physical sterilization techniques commonly employed in the pharmaceutical arts.

## METHODS FOR ADMINISTERING THE PHARMACEUTICAL COMPOSITIONS

The tablets of the invention can be administered to a patient once daily or about every twenty four hours. Alternatively, the tablets of the invention can be administered to a patient twice daily. Alternatively, the tablet of the invention can be administered about every twelve hours. These tablets contain 100 mg or 200 mg of Compound 1 Form I; and 125 mg of substantially amorphous Compound 2. In this aspect, in addition to Compound 1 Form I and substantially amorphous Compound 2, the tablets comprise a filler; a disintegrant; a surfactant; a binder; and a lubricant. For instance, a dose of 400 mg of Compound 1 Form I, may comprise two tablets of the invention each containing 200 mg of Compound 1 Form I. A dose of 250 mg of substantially amorphous Compound 2, may comprise two tablets of the invention each containing 125 mg of substantially amorphous Compound 2.

It will also be appreciated that the tablets of the invention can be employed in combination therapies; that is, Compound 1 Form I and a solid dispersion of substantially amorphous Compound 2 and pharmaceutically acceptable compositions thereof can be administered concurrently with, prior to, or subsequent to, one or more other desired therapeutics or medical procedures.

In one embodiment, the additional therapeutic agent is selected from a mucolytic agent, bronchodialator, an antibiotic, an anti-infective agent, an anti-inflammatory agent, a compound that induces CFTR activity other than Compound 1 Form I and substantially amorphous Compound 2, or a nutritional agent.

In one embodiment, the additional agent is (*R*)-1-(2,2-difluorobenzo[d][1,3]dioxol-5-yl)-N-(1-(2,3-dihydroxypropyl)-6-fluoro-2-(1-hydroxy-2-methylpropan-2-yl)-1H-indol-5-yl)cyclopropanecarboxamide. In another embodiment, the additional agent is 4-(3-(1-(2,2-difluorobenzo[d][1,3]dioxol-5-yl)cyclopropanecarboxamido) isoquinolin-1-yl)benzoic acid. In another embodiment, the additional agent is selected from Table 1:

**Table 1.**

| 1 | 2 | 3 |
|---|---|---|
| 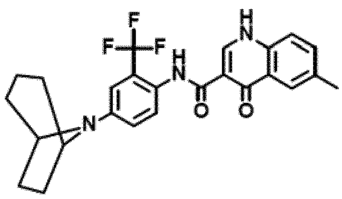 | 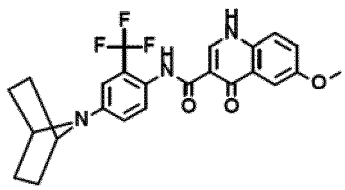 | 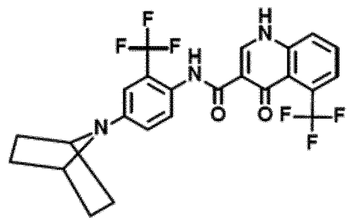 |
| 4 | 5 | 6 |
| 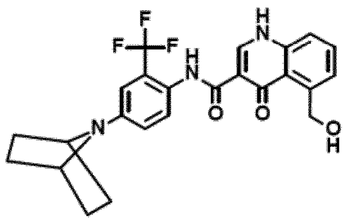 | 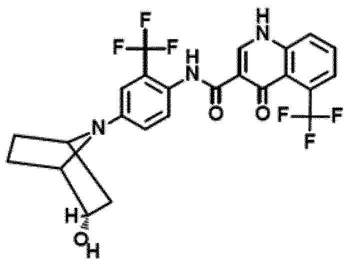 | 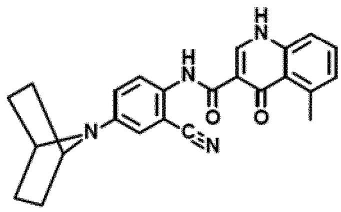 |
| 7 | 8 | 9 |

(continued)

| | | |
|---|---|---|
| 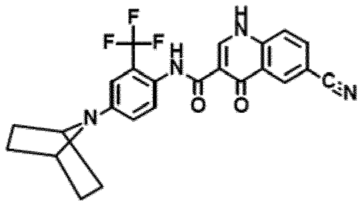 | 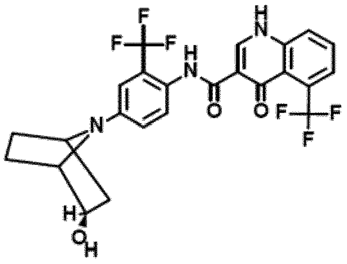 | 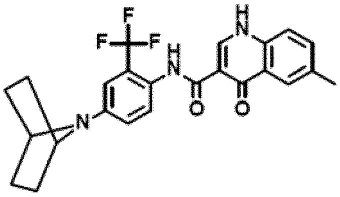 |
| 10 | 11 | 12 |
| 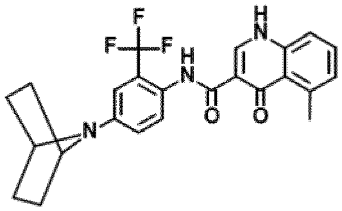 | 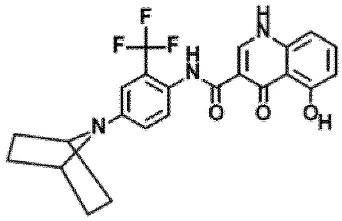 | 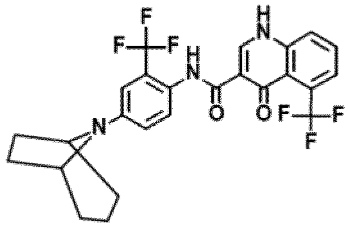 |
| 13 | 14 | |
| 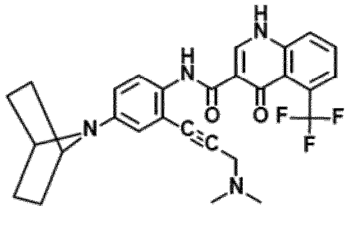 | 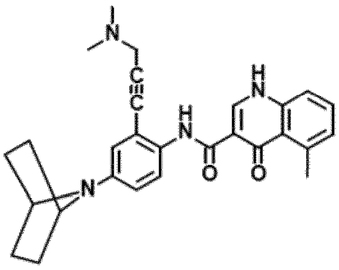 | |

In another embodiment, the additional agent is any combination of the above agents. For example, the combination may comprise a tablet of the present invention comprising Compound 1 Form I and a solid dispersion of substantially amorphous Compound 2, and the additional therapeutic agent is (*R*)-1-(2,2-difluorobenzo[d][1,3]dioxol-5-yl)-N-(1-(2,3-dihydroxypropyl)-6-fluoro-2-(1-hydroxy-2-methylpropan-2-yl)-1H-indol-5-yl)cyclopropanecarboxamide. In another example, the combination may comprise a tablet of the present invention comprising Compound 1 Form I and a solid dispersion of substantially amorphous Compound 2, and the additional therapeutic agent is 4-(3-(1-(2,2-difluorobenzo[d][1,3]dioxol-5-yl)cyclopropanecarboxamido) isoquinolin-1-yl)benzoic acid. In another example, the combination may comprise a tablet of the present invention comprising Compound 1 Form I and a solid dispersion of substantially amorphous Compound 2, and the additional therapeutic agent is any one of the compounds from Table 1, i.e. compounds 1 through 14 of Table 1, or any combination thereof.

In another embodiment, the additional agent is selected from Table 1:

| **TABLE 1** |
|---|
| Compounds disclosed in US Patent No. 7,407,976 (Col 13, ln 35- col 66, ln 67; Compounds 1-100 in Table 1 at col 67, ln 1-col 127, ln 42) |
| Compounds disclosed in US Patent No. 7,645,789 (Col 16, ln 52-col 50, ln 22; Compounds 1-322 in Table 1 at col 50, ln 24-col 167, ln 42) |
| Compounds disclosed in US Patent No. 7,659,268 (Col 16, ln 20-col 70, ln 52; Compounds 1-528 in Table 1 at col 70, ln 53-col 331, ln 34) |

(continued)

| TABLE 1 |
| --- |
| Compounds disclosed in US Patent No. 7,671,221 (Col 16, In 12-col 54, In 48; Compounds 1-1216 in Table 1 at col 54, In 49-col 699, In 27) |
| Compounds disclosed in US Patent No. 7,691,902 (Col 16, In 11-col 54, In 29; Compounds 1-959 in Table 1 at col 54, In 29-col 683, In 44) |
| Compounds disclosed in US Patent No. 7,741,321 (Col 16, In 25-col 72, In 17; Compounds 1-422 in Table 1 at col 72, In 20-col 279, In 15) |
| Compounds disclosed in US Patent No. 7,754,739 (Col 16, In 1-col 22, In 47; Compounds 1-2 in Table 1 at col 18, In 26-65) |
| Compounds disclosed in US Patent No. 7,776,905 (Col 16, In 23-col 38, In 40; Compounds 1-306 in Table 1 at col 38, In 45-col 96, In 40) |
| Compounds disclosed in US Patent No. 7,973,169 (Col 9, In 16-col 40, In 40; Compounds 1-289 in Table 1 at col. 40, In 41-col 289, In 39) |
| Compounds disclosed in US Patent No. 7,977,322 (Col 6, In 26-col 37, In 47; Compounds 1-498 in Table 1 at col 37, In 50-col 141, In 40) |
| Compounds disclosed in US Patent No. 7,999,113 (Col 6, In 13-col 10, In 67; Compounds 1-13 in Table 1 at col 11, In 5-col 13, In 65) |
| Compounds disclosed in US Patent No. 8,227,615 (Col 6, In 10-col 29, In 66; Compounds 1-78 in Table 1 at col 30, In 1-col 46, In 48) |
| Compounds disclosed in US Patent No. 8,299,099 (Col 6, In 10-col 34, In 18; Compounds 1-47 in Table 1 at col 34, In 20-col 42, In 35) |
| Compounds disclosed in US Published Application No. 2006-0052358 (Paragraphs [0034]-[0056]; [0077]-[0240]; Compounds 1-320 in Table 1 at paragraph [0241]) |
| Compounds disclosed in US Published Application No. 2009-0143381 (Paragraphs [0102]-[0263]; Compounds 1-28 in Table 1 at paragraph [0264]) |
| Compounds disclosed in US Published Application No. 2009-0170905 (Paragraphs [0012]-[0013]; [0030]-[0051]) |
| Compounds disclosed in US Published Application No. 2009-0253736 (Paragraphs [0031]-[0162]; Compounds 1-15 in Table 1 at paragraph [0163]) |
| Compounds disclosed in US Published Application No. 2011-0263654 (Paragraphs [0012]-[0013]; [0066]-[0141]) |
| Compounds disclosed in US Published Application No. 2011-0251253 (Paragraphs [0012]-[0013]; [0054]-[0079]) |
| Compounds disclosed in PCT application WO2008141119 (Paragraphs [0100]-[0339]; Compounds 1-117 in Table 1 at paragraph [0340]) |
| Compounds disclosed in US Application No. 11/047,361 |
| Compounds disclosed in US Published Application No. 2013-0116238 (Paragraphs [0028]-[0044]; [0117]-[0128]), or combinations thereof. |

In another embodiment, the additional agent is selected from Table 2:

| TABLE 2 |
| --- |
| Compounds disclosed in US Published Application No. 2005-0113423 (Paragraph [00146]; Compounds IA-1-IA-136 and Compounds I-1-I-21 in Tables 1 and 2 at paragraphs [0391]-[0392]) |
| Compounds disclosed in US Published Application No. 2005-0059687 (Paragraphs [00100]-[00101]; Compounds 1-405 in Table 1 at paragraph [0169]) |
| Compounds 1-108 disclosed in US Patent No. 7,598,412 (Col 22, In 14-col 79, In 20;Table 1) |
| Compounds 1-485 disclosed in US Patent No. 7,495,103 (Col 51, In 1-col 63, In 43; Table 1) |

(continued)

| TABLE 2 |
|---|
| Compounds 1-718 disclosed in US Patent No. 8,354,427 (Col 51, In 3-col 71, In 46; Table 1) |
| Compounds 1-233 disclosed in US Published Application No. 2007-0105833 (Paragraph [00145]; Table 1) |
| Compounds 1-26 disclosed in US Patent No. 8,242,149 (Col 46, In 47-col 57, In 37; Table 1) |
| Compounds 1-18 disclosed in US Patent No. 8,314,256 (Col 21, In 1-col 26, In 19) |
| Compounds 1-14 disclosed in US Patent No. 8,399,479 (Col 36, In 20-col 38, In 40; Table 1) |
| Compounds 1-18 disclosed in US Patent No. 8,188,283 (Col 38, In 43-col 43, In 36; Table 1) |
| Compounds 1-16 disclosed in US Published Application No. 2010-0249180 (Paragraph [0173]; Table 1) |
| Compounds 1-19 disclosed in US Published Application No. 2011-0008259 (Paragraph [0172]; Table 1) |
| Compounds 1-129 disclosed in US Patent No. 8,367,660 (Col 57, In 31-col 81, In 24; Table 1) |

In one embodiment, the additional therapeutic agent is an antibiotic. Exemplary antibiotics useful herein include tobramycin, including tobramycin inhaled powder (TIP), azithromycin, cayston, aztreonam, including the aerosolized form of aztreonam, amikacin, including liposomal formulations thereof, ciprofloxacin, including formulations thereof suitable for administration by inhalation, levoflaxacin, including aerosolized formulations thereof, and combinations of two antibiotics, e.g., fosfomycin and tobramycin.

In another embodiment, the additional agent is a mucolyte. Exemplary mucolytes useful herein includes Pulmozyme®.

In another embodiment, the additional agent is a bronchodialator. Exemplary bronchodialtors include albuterol, metaprotenerol sulfate, pirbuterol acetate, salmeterol, or tetrabuline sulfate.

In another embodiment, the additional agent is effective in restoring lung airway surface liquid. Such agents improve the movement of salt in and out of cells, allowing mucus in the lung airway to be more hydrated and, therefore, cleared more easily. Exemplary such agents include hypertonic saline, denufosol tetrasodium ([[(3S,5R)-5-(4-amino-2-oxopyrimidin-1-yl)-3-hydroxyoxolan-2-yl]methoxy-hydroxyphosphoryl] [[[(2R,3S,4R,5R)-5-(2,4-dioxopyrimidin-1-yl)-3,4-dihydroxyoxolan-2-yl]methoxy-hydroxyphosphoryl]oxy-hydroxyphosphoryl] hydrogen phosphate), or bronchitol (inhaled formulation of mannitol).

In another embodiment, the additional agent is an anti-inflammatory agent, i.e., an agent that can reduce the inflammation in the lungs. Exemplary such agents useful herein include ibuprofen, docosahexanoic acid (DHA), sildenafil, inhaled glutathione, pioglitazone, hydroxychloroquine, or simavastatin.

In another embodiment, the additional agent is a compound that augments or induces CFTR activity other than Compound 1 Form I or a solid dispersion comprising substantially amorphous Compound 2, i.e., an agent that has the effect of inducing or augmenting CFTR activity. Exemplary such agents include ataluren ("PTC124®"; 3-[5-(2-fluorophenyl)-1,2,4-oxadiazol-3-yl]benzoic acid), sinapultide, lancovutide, depelestat (a human recombinant neutrophil elastase inhibitor), and cobiprostone (7-{(2R, 4aR, 5R, 7aR)-2-[(3S)-1,1-difluoro-3-methylpentyl]-2-hydroxy-6-oxooctahydrocyclopenta[b]pyran-5-yl}heptanoic acid).

In another embodiment, the additional agent is a nutritional agent. Exemplary nutritional agents include pancrelipase (pancreating enzyme replacement), including Pancrease®, Pancreacarb®, Ultrase®, or Creon®, Liprotomase® (formerly Trizytek®), Aquadeks®, or glutathione inhalation. In one embodiment, the additional nutritional agent is pancrelipase.

In another embodiment, the additional agent is a compound selected from gentamicin, curcumin, cyclophosphamide, 4-phenylbutyrate, miglustat, felodipine, nimodipine, Philoxin B, geniestein, Apigenin, cAMP/cGMP augmenters or inducers such as rolipram, sildenafil, milrinone, tadalafil, amrinone, isoproterenol, albuterol, and almeterol, deoxyspergualin, HSP 90 inhibitors, HSP 70 inhibitors, proteosome inhibitors such as epoxomicin, lactacystin, etc.

In another embodiment, the additional agent is a compound selected from 3-amino-6-(4-fluoro-phenyl)-5-trifluoromethyl-pyridine-2-carboxylic acid (3,3,3-trifluoro-2-hydroxy -2-methyl-propyl)-amide; 5-amino-6'-methyl-3-trifluoromethyl-[2,3]bipyridinyl-6-carboxylic acid (3,3,3-trifluoro-2-hydroxy -2-methyl-propyl)-amide; 3-amino-6-cyclopropyl-N-(3,3,3-trifluoro-2-hydroxy-2-methylpropyl)-5-(trifluoromethyl)picolinamide; 3-amino-6-methoxy-N-(3,3,3-trifluoro-2-hydroxy-2-(trifluoromethyl)propyl)-5-(trifluoro methyl)picolinamide; 3-amino-6-(4-fluoro-phenyl)-5-trifluoromethyl-pyridine-2-carboxylic acid ((S)-3,3,3-trifluoro-2-hydroxy -2-methyl-propyl)-amide; 3-amino-6-methoxy-5-trifluoromethyl-pyridine-2-carboxylic acid((S-3,3,3-trifluoro-2-hydroxy-2-methyl-propyl)-amide; 3-amino-6-methoxy-5-trifluoromethyl-pyridine-2-carboxylic acid ((R)-3,3,3-trifluoro-2-hydroxy-2-methyl-propyl)-amide; 3-amino-6-(2,4-dichloro-phenyl)-5-trifluoromethyl-pyridine-2-carboxylic acid ((S)-3,3,3-trifluoro-2-hydroxy-2-methyl-propyl)-amide; 3-amino-6-(2,4-dichloro-phenyl)-5-trifluoromethyl-pyridine-2-carboxylic acid ((R)-3,3,3-trifluoro -2-hydroxy-2-methylpropyl)-amide; 3-amino-6-(4-fluoro-phenyl)-5-trifluoromethyl-pyridine-2-carboxylic acid (2-hydroxy-2-methyl-propyl)-amide; 3-amino-5,6-bis-trifluoromethyl-pyridine-2-carboxylic acid ((S)-3,3,3-trifluoro-2-hydroxy-2-methyl-propyl)-amide; 3-amino-5,6-bis-trifluoromethyl-pyridine-2-carboxylic acid ((R)-3,3,3-trifluoro-2-hydroxy-2-methyl-propyl)-amide; (S)-3-amino-6-ethoxy-N-(3,3,3-trifluoro-2-hydroxy-2-methylpropyl)-5-(trifluoro methyl)picolinamide; 3-amino-6-methoxy-5-trifluoromethyl-pyridine-2-carboxylic acid ((S)-3,3,3-trifluoro-2-hydroxy-2-methyl-propyl)-amide; 3-amino-6-methoxy-5-trifluoromethyl-pyridine-2-carboxylic acid ((R)-3,3,3-trifluoro-2-hydroxy-2-methyl-propyl)-amide; 3-amino-6-(4-fluoro-phenyl)-5-trifluoromethyl-pyridine-2-carboxylic acid (3,3,3-trifluoro-2-hydroxy-2-methyl-propyl)-amide; 3-amino-5,6-bis-trifluoromethyl-pyridine-2-carboxylic acid ((S)-3,3,3-trifluoro-2-hydroxy-2-methyl-propyl)-amide; 3-amino-5,6-bis-trifluoromethyl-pyridine-2-carboxylic acid ((R)-3,3,3-trifluoro-2-hydroxy-2-methyl-propyl)-amide, or pharmaceutically acceptable salts thereof. In another embodiment, the additional agent is a compound disclosed in United States Patent No. 8,247,436 and International PCT Publication WO 2011113894.

In another embodiment, the additional agent may be an epithelial sodium channel (ENac) modulator disclosed in PCT publications WO2012035158, WO2009074575, WO2011028740, WO2009150137, WO2011079087, or WO2008135557.

In other embodiments, the additional agent is a compound disclosed in WO 2004028480, WO 2004110352, WO 2005094374, WO 2005120497, or WO 2006101740. In another embodiment, the additional agent is a benzo[c]quinolizinium derivative that exhibits CFTR inducing or augmenting activity or a benzopyran derivative that exhibits CFTR inducing or augmenting activity. In another embodiment, the additional agent is a compound disclosed in U.S. Pat. No. 7,202,262, U.S. Pat. No. 6,992,096, US20060148864, US20060148863, US20060035943, US20050164973, WO2006110483, WO2006044456, WO2006044682, WO2006044505, WO2006044503, WO2006044502, or WO2004091502. In another embodiment, the additional agent is a compound disclosed in WO2004080972, WO2004111014, WO2005035514, WO2005049018, WO2006099256, WO2006127588, or WO2007044560.

In one aspect of the disclosure, 400 mg of Compound 1 Form I and 250 mg of substantially amorphous Compound 2 may be administered to a subject in need thereof. In this disclosure, the dosage amounts may be achieved by administration of tablets of the invention. For example, administration of 400 mg of Compound 1 Form I and 250 mg of substantially amorphous Compound 2 may be achieved by administering two tablets each containing 200 mg of Compound 1 Form I, and 125 mg of substantially amorphous Compound 2. The duration of administration may continue until amelioration of the disease is achieved or until a subject's physician advises, e.g. duration of administration may be less than a week, 1 week, 2 weeks, 3 weeks, four weeks (28 days), or a month or longer. In one aspect of the disclosure, two tablets each comprising 200 mg of Compound 1 Form I, and 125 mg of substantially amorphous Compound 2 may be administered to the patient per day. In a further aspect of the disclosure, the two tablets may be administered at the same time or at different times during the day. In a further aspect of the disclosure, one tablet is administered every 12 hours.

In one aspect of the disclosure, 400 mg of Compound 1 Form I and 500 mg of substantially amorphous Compound 2 may be administered to a subject in need thereof. In one aspect of the disclosure the dosage amount may be achieved by administering two tablets, each containing 100 mg of Compound 1 Form I and 125 mg of substantially amorphous Compound 2, every 12 hours. In another aspect of the disclosure, the dosage amounts may also be achieved by administering Compound 1 Form I and substantially amorphous Compound 2 in separate tablets. For example, the dosage amounts may be achieved by administering two tablets containing 200 mg of Compound 1 Form I, and four tablets containing 125 mg of substantially amorphous Compound 2. The duration of administration may continue until amelioration of the disease is achieved or until a subject's physician advises, e.g. duration of administration may be less than a week, 1 week, 2 weeks, 3 weeks, four weeks (28 days), or a month or longer. In one aspect of the disclosure, two tablets comprising 200 mg of Compound 1 Form I, and four tablets comprising 125 mg of substantially amorphous Compound 2 may be administered to the patient per day in a further aspect of the disclosure, the two tablets may be administered at the same time or at different times during the day.

The duration of administration may continue until amelioration of the disease is achieved or until a subject's physician advises, e.g. duration of administration may be less than a week, 1 week, 2 weeks, 3 weeks, four weeks (28 days), or a month or longer. In a further embodiment, one tablet is administered every 12 hours.

In one aspect of the disclosure, 600 mg of Compound 1 Form I and 500 mg of substantially amorphous Compound 2 may be administered to a subject in need thereof. In this disclosure, the dosage amounts may be achieved by administration of tablets of the invention. The duration of administration may continue until amelioration of the disease is achieved or until a subject's physician advises, e.g. duration of administration may be less than a week, 1 week, 2 weeks, 3 weeks, four weeks (28 days), or a month or longer. In a further aspect of the disclosure, two tablets are administered every 12 hours.

In one aspect of the disclosure, 800 mg of Compound 1 Form I and 500 mg of substantially amorphous Compound 2 may be administered to a subject in need thereof. In this disclosure, the dosage amounts may be achieved by administration of tablets of the invention. For example, administration of 800 mg of Compound 1 Form I and 500 mg of substantially amorphous Compound 2 may be achieved by administering four tablets each containing 200 mg of Compound 1 Form I, and 125 mg of substantially amorphous Compound 2. The duration of administration may continue until amelioration of the disease is achieved or until a subject's physician advises, e.g. duration of administration may be less than a week, 1 week, 2 weeks, 3 weeks, four weeks (28 days), or a month or longer. In one aspect of the disclosure, four tablets each comprising 200 mg of Compound 1 Form I, and 125 mg of substantially amorphous Compound 2 may be administered to the patient per day. In a further embodiment, the fourtablets may be administered at the same time or at different times during the day. In a further aspect of the disclosure, two tablets are administered per dosing occasion, and there are two dosing occasions per day. In a further aspect of the disclosure, 800 mg of Compound 1 and 500 mg of Compound 2 are administered to the patient by administering two tablets each comprising 200 mg of Compound 1 and 125 mg of Compound 2 twice a day (BID). In a further aspect of the disclosure, 800 mg of Compound 1 and 500 mg of Compound 2 are administered to the patient by administering two tablets each comprising 200 mg of Compound 1 and 125 mg of Compound 2 every 12 hours (q12h).

In one aspect of the disclosure, 600 mg of Compound 1 Form I and 250 mg of substantially amorphous Compound 2 may be administered to a subject in need thereof. In this disclosure, the dosage amounts may be achieved by administration of tablets of the invention. The duration of administration may continue until amelioration of the disease is achieved or until a subject's physician advises, e.g. duration of administration may be less than a week, 1 week, 2 weeks, 3 weeks, four weeks (28 days), or a month or longer. In a further aspect of the disclosure, three tablets are administered at the same time.

In one aspect of the disclosure, 600 mg of Compound 1 Form I and 500 mg of substantially amorphous Compound 2 may be administered to a subject in need thereof. In this disclosure, the dosage amounts may be achieved by administration of tablets of the invention. The duration of administration may continue until amelioration of the disease is achieved or until a subject's physician advises, e.g. duration of administration may be less than a week, 1 week, 2 weeks, 3 weeks, four weeks (28 days), or a month or longer.

These combinations are useful for treating the diseases described herein including cystic fibrosis. These combinations are also useful in the kits described herein. In another aspect, the present invention features a kit comprising a tablet of the present invention comprising Compound 1 Form I and a solid dispersion comprising substantially amorphous Compound 2, and a separate additional therapeutic agent. In another embodiment, the tablet of the present invention, separate additional therapeutic agent are in separate containers. In another embodiment, the separate containers are bottles. In another embodiment, the separate containers are vials. In another embodiment, the separate containers are blister packs.

The amount of additional therapeutic agent present in the tablets of this invention will be no more than the amount that would normally be administered in a tablet comprising that therapeutic agent as the only active agent. Preferably the amount of additional therapeutic agent in the presently disclosed compositions will range from about 50% to 100% of the amount normally present in a composition comprising that agent as the only therapeutically active agent.

## THERAPEUTIC USES OF THE COMPOSITION

Also disclosed is a tablet for use in a method of treating, lessening the severity of, or symptomatically treating a disease in a patient, the method comprising administering an effective amount of the tablet of the invention to the patient, preferably a mammal, wherein the disease is cystic fibrosis. Disclosed herein is a tablet for use in a method of treating, lessening the severity of, or symptomatically treating asthma, smoke induced COPD, chronic bronchitis, rhinosinusitis, constipation, pancreatitis, pancreatic insufficiency, male infertility caused by congenital bilateral absence of the vas deferens (CBAVD), mild pulmonary disease, idiopathic pancreatitis, allergic bronchopulmonary aspergillosis (ABPA), liver disease, hereditary emphysema, hereditary hemochromatosis, coagulation-fibrinolysis deficiencies, such as protein C deficiency, Type 1 hereditary angioedema, lipid processing deficiencies, such as familial hypercholesterolemia, Type 1 chylomicronemia, abetalipoproteinemia, lysosomal storage diseases, such as I-cell disease/pseudo-Hurler, mucopolysaccharidoses, Sandhof/Tay-Sachs, Crigler-Najjar type II, polyendocrinopathy/hyperinsulemia, Diabetes mellitus, Laron dwarfism, myleoperoxidase deficiency, primary hypoparathyroidism, melanoma, glycanosis CDG type 1, congenital hyperthyroidism, osteogenesis imperfecta, hereditary hypofibrinogenemia, ACT deficiency, Diabetes insipidus (DI), neurophyseal DI, neprogenic DI, Charcot-Marie Tooth syndrome, Perlizaeus-Merzbacher disease, neurodegenerative diseases such as Alzheimer's disease, Parkinson's disease, amyotrophic lateral sclerosis, progressive supranuclear plasy, Pick's disease, several polyglutamine neurological disorders such as Huntington's, spinocerebullar ataxia type I, spinal and bulbar muscular atrophy, dentatorubal pallidoluysian, and myotonic dystrophy, as well as spongiform encephalopathies, such as hereditary Creutzfeldt-Jakob disease (due to prion protein processing defect), Fabry disease, Straussler-Scheinker syndrome, COPD, dry-eye disease, or Sjogren's disease, osteoporosis, osteopenia, bone healing and bone growth (including bone repair, bone regeneration, reducing bone resorption and increasing bone deposition), Gorham's Syndrome, chloride channelopathies such as myotonia congenita (Thomson and Becker forms), Bartter's

syndrome type III, Dent's disease, hyperekplexia, epilepsy, lysosomal storage disease, Angelman syndrome, and Primary Ciliary Dyskinesia (PCD), a term for inherited disorders of the structure and/or function of cilia, including PCD with situs inversus (also known as Kartagener syndrome), PCD without situs inversus and ciliary aplasia.

Disclosed herein is a method of treating, lessening the severity of, or symptomatically treating a disease in a patient comprising administering an effective amount of the tablet of the invention to the patient, preferably a mammal, wherein the disease is selected from generalized epilepsy with ferbrile seizures plus (GEFS+), general epilepsy with ferbile and aferbrile seizures, myotonia, paramyotonia congenital, potassium-aggravated myotonia, hyperkalemic periodic paralysis, LQTS, LQTS/Brugada syndrome, autosomal-dominant LQTS with deafness, autosomal-recessive LQTS, LQTS with dysmorphic features, congenital and acquired LQTS, Timothy syndrome, persistent hyperinsulinemic hypolglycemia of infancy, dilated cardiomyopathy, autosomal-dominant LQTS, Dent disease, Osteopetrosis, Bartter syndrome type III, central core disease, malignant hyperthermia, and catecholaminergic polymorphic tachycardia.

Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation N1303K, ΔI507, or R560T.

Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation G551D. In another aspect of the disclosure, the patient is homozygous in G551D. In another aspect of the disclosure, the patient is heterozygous in G551D wherein the other CFTR genetic mutation is any one of ΔF508, G542X, N1303K, W1282X, R117H, R553X, 1717-1G->A, 621+1G->T, 2789+5G->A, 3849+10kbC->T, R1162X, G85E, 3120+1G->A, ΔI507, 1898+1G->A, 3659delC, R347P, R560T, R334W, A455E, 2184delA, or 711+1G->T.

Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation ΔF508. In another aspect of the disclosure, the patient is homozygous in ΔF508. In another aspect of the disclosure, the patient is heterozygous in ΔF508 wherein the other CFTR genetic mutation is any one of G551D, G542X, N1303K, W1282X, R117H, R553X, 1717-1G->A, 621+1G->T, 2789+5G->A, 3849+10kbC->T, R1162X, G85E, 3120+1G->A, ΔI507, 1898+1G->A, 3659delC, R347P, R560T, R334W, A455E, 2184delA, or 711+1G->T.

Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from G178R, G551S, G970R, G1244E, S1255P, G1349D, S549N, S549R, S1251N, E193K, F1052V, G1069R, R117C, D110H, R347H, R352Q, E56K, P67L, L206W, A455E, D579G, S1235R, S945L, R1070W, F1074L, D110E, D1270N, D1152H, 1717-1G->A, 621+1G->T, 3120+1G->A, 1898+1G->A, 711+1G->T, 2622+1G->A, 405+1G->A, 406-1G->A, 4005+1G->A, 1812-1G->A, 1525-1G->A, 712-1G->T, 1248+1G->A, 1341+1G->A, 3121-1G->A, 4374+1G->T, 3850-1G->A, 2789+5G->A, 3849+10kbC->T, 3272-26A->G, 711+5G->A, 3120G->A, 1811+1.6kbA->G, 711+3A->G, 1898+3A->G, 1717-8G->A, 1342-2A->C, 405+3A->C, 1716G/A, 1811+1G->C, 1898+5G->T, 3850-3T->G, IVS14b+5G->A, 1898+1G->T, 4005+2T->C and 621+3A->G.

Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from G178R, G551S, G970R, G1244E, S1255P, G1349D, S549N, S549R, S1251N, E193K, F1052V and G1069R. Also disclosed is a tablet for use in a method of treating CFTR comprising administering Compound 1 to a patient possessing a human CFTR mutation selected from G178R, G551S, G970R, G1244E, S1255P, G1349D, S549N, S549R and S1251N. Also disclosed is a tablet for use in a method of treating, lessening the severity of, or symptomatically treating cystic fibrosis in a patient comprising administering an effective amount of the tablet of the invention to the patient, preferably a mammal, wherein the patient possesses the CFTR genetic mutation is selected from E193K, F1052V and G1069R. In some aspects of the disclosure, the method produces a greater than 10-fold increase in chloride transport relative to baseline chloride transport.

Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from R117C, D110H, R347H, R352Q, E56K, P67L, L206W, A455E, D579G, S1235R, S945L, R1070W, F1074L, D110E, D1270N and D1152H. In one aspect of the disclosure, the method produces an increase in chloride transport which is greater or equal to 10% above the baseline chloride transport.

Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from 1717-1G->A, 621+1G->T, 3120+1G->A, 1898+1G->A, 711+1G->T, 2622+1G->A, 405+1G->A, 406-1G->A, 4005+1G->A, 1812-1G->A, 1525-1G->A, 712-1G->T, 1248+1G->A, 1341+1G->A, 3121-1G->A, 4374+1G->T, 3850-1G->A, 2789+5G->A, 3849+10kbC->T, 3272-26A->G, 711+5G->A, 3120G->A, 1811+1.6kbA->G, 711+3A->G, 1898+3A->G, 1717-8G->A, 1342-2A->C, 405+3A->C, 1716G/A, 1811+1G->C, 1898+5G->T, 3850-3T->G, IVS14b+5G->A, 1898+1G->T, 4005+2T->C and 621+3A->G. Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from 1717-1G->A, 1811+1.6kbA->G, 2789+5G->A, 3272-26A->G and 3849+10kbC->T. Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from 2789+5G->A and 3272-26A->G.

Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from G178R, G551S, G970R, G1244E, S1255P, G1349D, S549N, S549R, S1251N, E193K, F1052V, G1069R, R117C, D110H, R347H, R352Q, E56K, P67L, L206W, A455E, D579G, S1235R, S945L, R1070W, F1074L, D110E, D1270N, D1152H, 1717-1G->A, 621+1G->T, 3120+1G->A, 1898+1G->A, 711+1G->T, 2622+1G->A, 405+1G->A, 406-1G->A, 4005+1G->A, 1812-1G->A, 1525-1G->A, 712-1G->T, 1248+1G->A, 1341+1G->A, 3121-1G->A, 4374+1G->T, 3850-1G->A, 2789+5G->A, 3849+10kbC->T, 3272-26A->G, 711+5G->A, 3120G->A, 1811+1.6kbA->G, 711+3A->G, 1898+3A->G, 1717-8G->A, 1342-2A->C, 405+3A->C, 1716G/A, 1811+1G->C, 1898+5G->T, 3850-3T->G, IVS14b+5G->A, 1898+1G->T, 4005+2T->C and 621+3A->G, and a human CFTR mutation selected from ΔF508, R117H, and G551D.

Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from G178R, G551S, G970R, G1244E, S1255P, G1349D, S549N, S549R, S1251N, E193K, F1052V and G1069R, and a human CFTR mutation selected from ΔF508, R117H, and G551D. Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from G178R, G551S, G970R, G1244E, S1255P, G1349D, S549N, S549R and S1251N, and a human CFTR mutation selected from ΔF508, R117H, and G551D. Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from E193K, F1052V and G1069R, and a human CFTR mutation selected from ΔF508, R117H, and G551D. In some aspects of the disclosure, the method produces a greater than 10-fold increase in chloride transport relative to baseline chloride transport.

Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from R117C, D110H, R347H, R352Q, E56K, P67L, L206W, A455E, D579G, S1235R, S945L, R1070W, F1074L, D110E, D1270N and D1152H, and a human CFTR mutation selected from ΔF508, R117H, and G551D. In one aspect of the disclosure, the method produces an increase in chloride transport which is greater or equal to 10% above the baseline chloride transport.

Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from 1717-1G->A, 621+1G->T, 3120+1G->A, 1898+1G->A, 711+1G->T, 2622+1G->A, 405+1G->A, 406-1G->A, 4005+1G->A, 1812-1G->A, 1525-1G->A, 712-1G->T, 1248+1G->A, 1341+1G->A, 3121-1G->A, 4374+1G->T, 3850-1G->A, 2789+5G->A, 3849+10kbC->T, 3272-26A->G, 711+5G->A, 3120G->A, 1811+1.6kbA->G, 711+3A->G, 1898+3A->G, 1717-8G->A, 1342-2A->C, 405+3A->C, 1716G/A, 1811+1G->C, 1898+5G->T, 3850-3T->G, IVS14b+5G->A, 1898+1G->T, 4005+2T->C and 621+3A->G, and a human CFTR mutation selected from ΔF508, R117H, and G551D. Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from 1717-1G->A, 1811+1.6kbA->G, 2789+5G->A, 3272-26A->G and 3849+10kbC->T, and a human CFTR mutation selected from ΔF508, R117H, and G551D. Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from 2789+5G->A and 3272-26A->G, and a human CFTR mutation selected from ΔF508, R117H.

Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from G178R, G551S, G970R, G1244E, S1255P, G1349D, S549N, S549R, S1251N, E193K, F1052V, G1069R, R117C, D110H, R347H, R352Q, E56K, P67L, L206W, A455E, D579G, S1235R, S945L, R1070W, F1074L, D110E, D1270N, D1152H, 1717-1G->A, 621+1G->T, 3120+1G->A, 1898+1G->A, 711+1G->T, 2622+1G->A, 405+1G->A, 406-1G->A, 4005+1G->A, 1812-1G->A, 1525-1G->A, 712-1G->T, 1248+1G->A, 1341+1G->A, 3121-1G->A, 4374+1G->T, 3850-1G->A, 2789+5G->A, 3849+10kbC->T, 3272-26A->G, 711+5G->A, 3120G->A, 1811+1.6kbA->G, 711+3A->G, 1898+3A->G, 1717-8G->A, 1342-2A->C, 405+3A->C, 1716G/A, 1811+1G->C, 1898+5G->T, 3850-3T->G, IVS14b+5G->A, 1898+1G->T, 4005+2T->C and 621+3A->G, and a human CFTR mutation selected from ΔF508, R117H, and G551D.

Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from G178R, G551S, G970R, G1244E, S1255P, G1349D, S549N, S549R, S1251N, E193K, F1052V and G1069R. Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from G178R, G551S, G970R, G1244E, S1255P, G1349D, S549N, S549R and S1251N. Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from E193K, F1052V and G1069R. In some aspects of the disclosure, the method produces a greater than 10-fold increase in chloride transport relative to baseline chloride transport.

Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from R117C, D110H, R347H, R352Q, E56K, P67L, L206W, A455E, D579G, S1235R, S945L, R1070W, F1074L, D110E, D1270N and D1152H. In one aspect of the disclosure, the method produces an increase in chloride transport which is greater or equal to 10% above the baseline chloride transport.

Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from 1717-1G->A, 621+1G->T, 3120+1G->A, 1898+1G->A, 711+1G->T, 2622+1G->A, 405+1G->A, 406-1G->A, 4005+1G->A, 1812-1G->A, 1525-1G->A, 712-1G->T, 1248+1G->A, 1341+1G->A, 3121-1G->A, 4374+1G->T, 3850-1G->A, 2789+5G->A, 3849+10kbC->T, 3272-26A->G, 711+5G->A, 3120G->A, 1811+1.6kbA->G, 711+3A->G, 1898+3A->G, 1717-8G->A, 1342-2A->C, 405+3A->C, 1716G/A, 1811+1G->C, 1898+5G->T, 3850-3T->G, IVS14b+5G->A, 1898+1G->T, 4005+2T->C and 621+3A->G. Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from 1717-1G->A, 1811+1.6kbA->G, 2789+5G->A, 3272-26A->G and 3849+10kbC->T. Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from 2789+5G->A and 3272-26A->G.

Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from G178R, G551S, G970R, G1244E, S1255P, G1349D, S549N, S549R, S1251N, E193K, F1052V, G1069R, R117C, D110H, R347H, R352Q, E56K, P67L, L206W, A455E, D579G, S1235R, S945L, R1070W, F1074L, D110E, D1270N, D1152H, 1717-1G->A, 621+1G->T, 3120+1G->A, 1898+1G->A, 711+1G->T, 2622+1G->A, 405+1G->A, 406-1G->A, 4005+1G->A, 1812-1G->A, 1525-1G->A, 712-1G->T, 1248+1G->A, 1341+1G->A, 3121-1G->A, 4374+1G->T, 3850-1G->A, 2789+5G->A, 3849+10kbC->T, 3272-26A->G, 711+5G->A, 3120G->A, 1811+1.6kbA->G, 711+3A->G, 1898+3A->G, 1717-8G->A, 1342-2A->C, 405+3A->C, 1716G/A, 1811+1G->C, 1898+5G->T, 3850-3T->G, IVS14b+5G->A, 1898+1G->T, 4005+2T->C and 621+3A->G, and a human CFTR mutation selected from ΔF508, R117H, and G551D, and one or more human CFTR mutations selected from ΔF508, R117H, and G551D.

Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from G178R,

G551S, G970R, G1244E, S1255P, G1349D, S549N, S549R, S1251N, E193K, F1052V and G1069R, and one or more human CFTR mutations selected from ΔF508, R117H, and G551D. Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from G178R, G551S, G970R, G1244E, S1255P, G1349D, S549N, S549R and S1251N, and one or more human CFTR mutations selected from ΔF508, R117H, and G551D. Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from E193K, F1052V and G1069R, and one or more human CFTR mutations selected from ΔF508, R117H, and G551D. In some aspects of the disclosure, the method produces a greater than 10-fold increase in chloride transport relative to baseline chloride transport.

Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from R117C, D110H, R347H, R352Q, E56K, P67L, L206W, A455E, D579G, S1235R, S945L, R1070W, F1074L, D110E, D1270N and D1152H, and one or more human CFTR mutations selected from ΔF508, R117H, and G551D. In one aspect of the disclosure, the method produces an increase in chloride transport which is greater or equal to 10% above the baseline chloride transport.

Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from 1717-1G->A, 621+1G->T, 3120+1G->A, 1898+1G->A, 711+1G->T, 2622+1G->A, 405+1G->A, 406-1G->A, 4005+1G->A, 1812-1G->A, 1525-1G->A, 712-1G->T, 1248+1G->A, 1341+1G->A, 3121-1G->A, 4374+1G->T, 3850-1G->A, 2789+5G->A, 3849+10kbC->T, 3272-26A->G, 711+5G->A, 3120G->A, 1811+1.6kbA->G, 711+3A->G, 1898+3A->G, 1717-8G->A, 1342-2A->C, 405+3A->C, 1716G/A, 1811+1G->C, 1898+5G->T, 3850-3T->G, IVS14b+5G->A, 1898+1G->T, 4005+2T->C and 621+3A->G, and one or more human CFTR mutations selected from ΔF508, R117H, and G551D. Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from 1717-1G->A, 1811+1.6kbA->G, 2789+5G->A, 3272-26A->G and 3849+10kbC->T, and one or more human CFTR mutations selected from ΔF508, R117H, and G551D. Also disclosed is a tablet for use in a method of treating, lessening the severity of, or symptomatically treating cystic fibrosis in a patient, wherein the method comprises administering an effective amount of the tablet of the invention to the patient, preferably a mammal, wherein the patient possesses the CFTR genetic mutation is selected from 2789+5G->A and 3272-26A->G, and one or more human CFTR mutations selected from ΔF508, R117H, and G551D.

In certain embodiments, the tablet of the present invention comprising Compound 1 Form I and a solid dispersion of substantially amorphous Compound 2 are useful for treating, lessening the severity of, or symptomatically treating cystic fibrosis in patients who exhibit residual CFTR activity in the apical membrane of respiratory and non-respiratory epithelia. The presence of residual CFTR activity at the epithelial surface can be readily detected using methods known in the art, e.g., standard electrophysiological, biochemical, or histochemical techniques. Such methods identify CFTR activity using *in vivo* or *ex vivo* electrophysiological techniques, measurement of sweat or salivary $Cl^-$ concentrations, or *ex vivo* biochemical or histochemical techniques to monitor cell surface density. Using such methods, residual CFTR activity can be readily detected in patients heterozygous or homozygous for a variety of different mutations, including patients homozygous or heterozygous for the most common mutation, ΔF508, as well as other mutations such as the G551D mutation, or the R117H mutation. In certain embodiments, the tablets comprising Compound 1 Form I and a solid dispersion comprising substantially amorphous Compound 2 are useful for treating, lessening the severity of, or symptomatically treating cystic fibrosis in patients who exhibit little to no residual CFTR activity. In certain embodiments, the tablets comprising Compound 1 Form I and a solid dispersion comprising substantially amorphous Compound 2 are useful for treating, lessening the severity of, or symptomatically treating cystic fibrosis in patients who exhibit little to no residual CFTR activity in the apical membrane of respiratory epithelia.

In another embodiment, the tablets of the present invention are useful for treating or lessening the severity of cystic fibrosis in patients who have residual CFTR activity induced or augmented using pharmacological methods. In another embodiment, the tablets of the present invention are useful for treating or lessening the severity of cystic fibrosis in patients who have residual CFTR activity induced or augmented using or gene therapy. Such methods increase the amount of CFTR present at the cell surface, thereby inducing a hitherto absent CFTR activity in a patient or augmenting the existing level of residual CFTR activity in a patient.

In one embodiment, tablets of the present invention comprising Compound 1 Form I and a solid dispersion comprising substantially amorphous Compound 2, as described herein, are useful for treating or lessening the severity

of cystic fibrosis in patients within certain genotypes exhibiting residual CFTR activity, e.g., Class I mutations (not synthesized), class II mutation (misfolding), class III mutations (impaired regulation or gating), class IV mutations (altered conductance), or class V mutations (reduced synthesis).

In one embodiment, tablets of the present invention comprising Compound 1 Form I and a solid dispersion comprising substantially amorphous Compound 2, as described herein, are useful for treating, lessening the severity of, or symptomatically treating cystic fibrosis in patients within certain clinical phenotypes, e.g., a moderate to mild clinical phenotype that typically correlates with the amount of residual CFTR activity in the apical membrane of epithelia. Such phenotypes include patients exhibiting pancreatic sufficiency.

In one embodiment, tablets of the present invention comprising Compound 1 Form I and a solid dispersion comprising substantially amorphous Compound 2, as described herein, are useful for treating, lessening the severity of, or symptomatically treating patients diagnosed with pancreatic sufficiency, idiopathic pancreatitis and congenital bilateral absence of the vas deferens, or mild lung disease wherein the patient exhibits residual CFTR activity.

In one embodiment, tablets of the present invention comprising Compound 1 Form I and a solid dispersion comprising substantially amorphous Compound 2, as described herein, are useful for treating, lessening the severity of, or symptomatically treating patients diagnosed with pancreatic sufficiency, idiopathic pancreatitis and congenital bilateral absence of the vas deferens, or mild lung disease wherein the patient has wild type CFTR.

In addition to cystic fibrosis, modulation of CFTR activity may be beneficial for other diseases not directly caused by mutations in CFTR, such as secretory diseases and other protein folding diseases mediated by CFTR. These include chronic obstructive pulmonary disease (COPD), dry eye disease, and Sjögren's Syndrome. COPD is characterized by airflow limitation that is progressive and not fully reversible. The airflow limitation is due to mucus hypersecretion, emphysema, and bronchiolitis. Activators of mutant or wild-type CFTR offer a potential treatment of mucus hypersecretion and impaired mucociliary clearance that is common in COPD. Specifically, increasing anion secretion across CFTR may facilitate fluid transport into the airway surface liquid to hydrate the mucus and optimized periciliary fluid viscosity. This would lead to enhanced mucociliary clearance and a reduction in the symptoms associated with COPD. Dry eye disease is characterized by a decrease in tear aqueous production and abnormal tear film lipid, protein and mucin profiles. There are many causes of dry eye, some of which include age, Lasik eye surgery, arthritis, medications, chemical/thermal burns, allergies, and diseases, such as cystic fibrosis and Sjögrens's syndrome. Increasing anion secretion via CFTR would enhance fluid transport from the corneal endothelial cells and secretory glands surrounding the eye to increase corneal hydration. This would help to alleviate the symptoms associated with dry eye disease. Sjögrens's syndrome is an autoimmune disease in which the immune system attacks moisture-producing glands throughout the body, including the eye, mouth, skin, respiratory tissue, liver, vagina, and gut. Symptoms, include, dry eye, mouth, and vagina, as well as lung disease. The disease is also associated with rheumatoid arthritis, systemic lupus, systemic sclerosis, and polymypositis/dermatomyositis. Defective protein trafficking is believed to cause the disease, for which treatment options are limited. Augmenters or inducers of CFTR activity may hydrate the various organs afflicted by the disease and help to elevate the associated symptoms.

Also disclosed is a method of augmenting or inducing anion channel activity *in vitro*, comprising contacting the channel with any one of pharmaceutical compositions **PC-I** to **PC-XXV.** The anion channel is a chloride channel or a bicarbonate channel, or the anion channel is a chloride channel.

The exact amount required will vary from subject to subject, depending on the species, age, and general condition of the subject, the severity of the infection, the particular agent, and its mode of administration. The tablets of the invention are preferably formulated in dosage unit form for ease of administration and uniformity of dosage. The expression "dosage unit form" as used herein refers to a physically discrete unit of agent appropriate for the patient to be treated. It will be understood, however, that the total daily usage of the tablets of the invention will be decided by the attending physician within the scope of sound medical judgment. The specific effective dose level for any particular patient or organism will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed, and like factors well known in the medical arts. The term "patient", as used herein, means an animal, preferably a mammal, and most preferably a human.

Anywhere in the present application where a name of a compound may not correctly describe the structure of the compound, the structure supersedes the name and governs.

## EXAMPLES

### XRPD (X-ray Powder Diffraction)

The X-Ray diffraction (XRD) data of Compound 1 Form I were collected on a Bruker D8 DISCOVER powder

diffractometer with HI-STAR 2-dimensional detector and a flat graphite monochromator. Cu sealed tube with Kα radiation was used at 40 kV, 35mA. The samples were placed on zero-background silicon wafers at 25°C. For each sample, two data frames were collected at 120 seconds each at 2 different $\theta_2$ angles: 8° and 26°. The data were integrated with GADDS software and merged with DIFFRACT$^{plus}$EVA software. Uncertainties for the reported peak positions are ± 0.2 degrees.

Differential Scanning Calorimetry (DSC)

The Differential scanning calorimetry (DSC) data of Compound 1 Form I were collected using a DSC Q100 V9.6 Build 290 (TA Instruments, New Castle, DE). Temperature was calibrated with indium and heat capacity was calibrated with sapphire. Samples of 3-6 mg were weighed into aluminum pans that were crimped using lids with 1 pin hole. The samples were scanned from 25°C to 350°C at a heating rate of 1.0°C/min and with a nitrogen gas purge of 50 ml/min. Data were collected by Thermal Advantage Q SeriesTM version 2.2.0.248 software and analyzed by Universal Analysis software version 4.1D (TA Instruments, New Castle, DE). The reported numbers represent single analyses.

Compound 1 Form I Single Crystal Structure Determination

Diffraction data were acquired on Bruker Apex II diffractometer equipped with sealed tube Cu K-alpha source and an Apex II CCD detector. The structure was solved and refined using SHELX program (Sheldrick, G.M., Acta Cryst., (2008) A64, 112-122). Based on systematic absences and intensities statistics the structure was solved and refined in $P2_1/n$ space group.

Vitride® (sodium bis(2-methoxyethoxy)aluminum hydride [or $NaAlH_2(OCH_2CH_2OCH_3)_2$], 65 wgt% solution in toluene) was purchased from Aldrich Chemicals.

2,2-Difluoro-1,3-benzodioxole-5-carboxylic acid was purchased from Saltigo (an affiliate of the Lanxess Corporation).

**Preparation of Compound 1**

**Preparation of (2,2-difluoro-1,3-benzodioxol-5-yl)-methanol.**

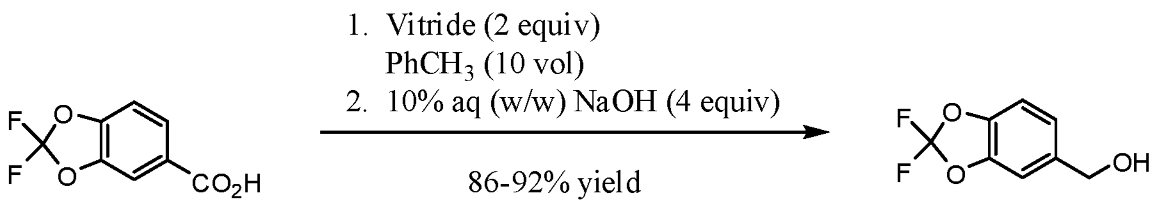


Commercially available 2,2-difluoro-1,3-benzodioxole-5-carboxylic acid (1.0 eq) was slurried in toluene (10 vol). Vitride® (2 eq) was added via addition funnel at a rate to maintain the temperature at 15-25 °C. At the end of the addition, the temperature was increased to 40 °C for 2 hours (h), then 10% (w/w) aqueous (aq) NaOH (4.0 eq) was carefully added via addition funnel, maintaining the temperature at 40-50 °C. After stirring for an additional 30 minutes (min), the layers were allowed to separate at 40 °C. The organic phase was cooled to 20 °C, then washed with water (2 x 1.5 vol), dried ($Na_2SO_4$), filtered, and concentrated to afford crude (2,2-difluoro-1,3-benzodioxol-5-yl)-methanol that was used directly in the next step.

**Preparation of 5-chloromethyl-2,2-difluoro-1,3-benzodioxole.**

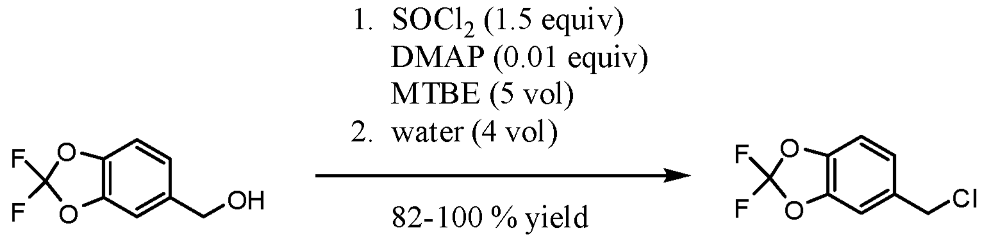


(2,2-difluoro-1,3-benzodioxol-5-yl)-methanol (1.0 eq) was dissolved in MTBE (5 vol). A catalytic amount of 4-(N,N-dimethyl)aminopyridine (DMAP) (1 mol %) was added and $SOCl_2$ (1.2 eq) was added via addition funnel. The $SOCl_2$ was added at a rate to maintain the temperature in the reactor at 15-25 °C. The temperature was increased to 30 °C for 1 h, and then was cooled to 20 °C. Water (4 vol) was added via addition funnel while maintaining the temperature at less than 30 °C. After stirring for an additional 30 min, the layers were allowed to separate. The organic layer was stirred and 10% (w/v) aq NaOH (4.4 vol) was added. After stirring for 15 to 20 min, the layers were allowed to separate. The organic phase was then dried ($Na_2SO_4$), filtered, and concentrated to afford crude 5-chloromethyl-2,2-difluoro-1,3-benzodioxole that was used directly in the next step.

**Preparation of (2,2-difluoro-1,3-benzodioxol-5-yl)-acetonitrile.**

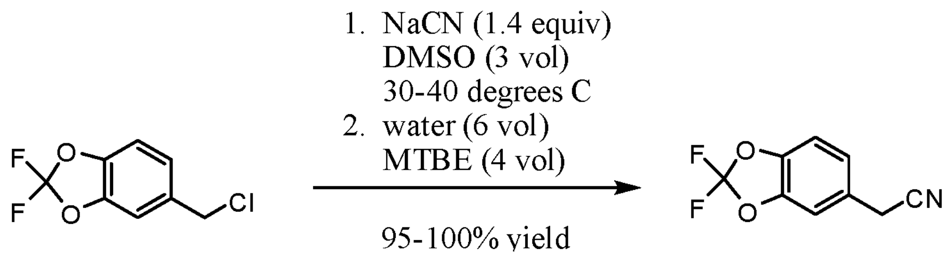


A solution of 5-chloromethyl-2,2-difluoro-1,3-benzodioxole (1 eq) in DMSO (1.25 vol) was added to a slurry of NaCN (1.4 eq) in DMSO (3 vol), while maintaining the temperature between 30-40 °C. The mixture was stirred for 1 h, and then water (6 vol) was added, followed by methyl *tert*-butyl ether (MTBE) (4 vol). After stirring for 30 min, the layers were separated. The aqueous layer was extracted with MTBE (1.8 vol). The combined organic layers were washed with water (1.8 vol), dried ($Na_2SO_4$), filtered, and concentrated to afford crude (2,2-difluoro-1,3-benzodioxol-5-yl)-acetonitrile (95%) that was used directly in the next step.

**Synthesis of (2,2-difluoro-1,3-benzodioxol-5-yl)-1-ethylacetate-acetonitrile**

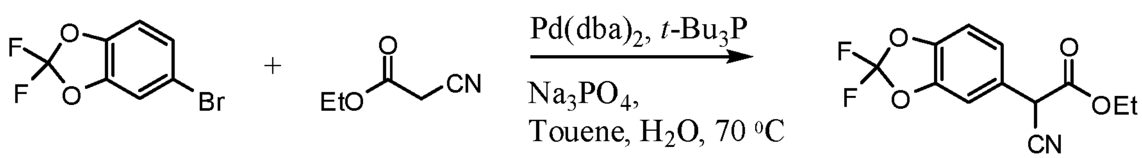


A reactor was purged with nitrogen and charged with 900 mL of toluene. The solvent was degassed via nitrogen sparge for no less than 16 h. To the reactor was then charged $Na_3PO_4$ (155.7 g, 949.5 mmol), followed by bis(dibenzylideneacetone) palladium (0) (7.28 g, 12.66 mmol). A 10% w/w solution of tert-butylphosphine in hexanes (51.23 g, 25.32 mmol) was charged over 10 min at 23 °C from a nitrogen purged addition funnel. The mixture was allowed to stir for 50 min, at which time 5-bromo-2,2-difluoro-1,3-benzodioxole (75 g, 316.5 mmol) was added over 1 min. After stirring for an additional 50 min, the mixture was charged with ethyl cyanoacetate (71.6 g, 633.0 mmol) over 5 min followed by water (4.5 mL) in one portion. The mixture was heated to 70 °C over 40 min and analyzed by HPLC every 1 - 2 h for the percent conversion of the reactant to the product. After complete conversion was observed (typically 100% conversion after 5 - 8 h), the mixture was cooled to 20 - 25 °C and filtered through a celite pad. The celite pad was rinsed with

toluene (2 X 450 mL) and the combined organics were concentrated to 300 mL under vacuum at 60 - 65 °C. The concentrate was charged with 225mL DMSO and concentrated under vacuum at 70 - 80 °C until active distillation of the solvent ceased. The solution was cooled to 20 - 25 °C and diluted to 900 mL with DMSO in preparation for Step 2. $^1H$ NMR (500 MHz, $CDCl_3$) δ 7.16 - 7.10 (m, 2H), 7.03 (d, *J* = 8.2 Hz, 1H), 4.63 (s, 1H), 4.19 (m, 2H), 1.23 (t, *J* = 7.1 Hz, 3H).

**Synthesis of (2,2-difluoro-1,3-benzodioxol-5-yl)-acetonitrile.**

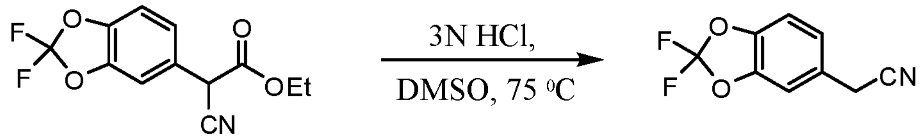


The DMSO solution of (2,2-difluoro-1,3-benzodioxol-5-yl)-1-ethylacetate-acetonitrile from above was charged with 3 N HCl (617.3 mL, 1.85 mol) over 20 min while maintaining an internal temperature < 40 °C. The mixture was then heated to 75°C over 1 h and analyzed by HPLC every 1 - 2 h for % conversion. When a conversion of > 99% was observed (typically after 5 - 6 h), the reaction was cooled to 20 - 25 °C and extracted with MTBE (2 X 525 mL), with sufficient time to allow for complete phase separation during the extractions. The combined organic extracts were washed with 5% NaCl (2 X 375 mL). The solution was then transferred to equipment appropriate for a 1.5 - 2.5 Torr vacuum distillation that was equipped with a cooled receiver flask. The solution was concentrated under vacuum at < 60°C to remove the solvents. (2,2-Difluoro-1,3-benzodioxol-5-yl)-acetonitrile was then distilled from the resulting oil at 125 - 130 °C (oven temperature) and 1.5 - 2.0 Torr. (2,2-Difluoro-1,3-benzodioxol-5-yl)-acetonitrile was isolated as a clear oil in 66% yield from 5-bromo-2,2-difluoro-1,3-benzodioxole (2 steps) and with an HPLC purity of 91.5% AUC (corresponds to a w/w assay of 95%). $^1H$ NMR (500 MHz, DMSO) δ 7.44 (br s, 1H), 7.43 (d, *J*= 8.4 Hz, 1H), 7.22 (dd, *J*= 8.2, 1.8 Hz, 1H), 4.07 (s, 2H).

**Preparation of (2,2-difluoro-1,3-benzodioxol-5-yl)-cyclopropanecarbonitrile.**

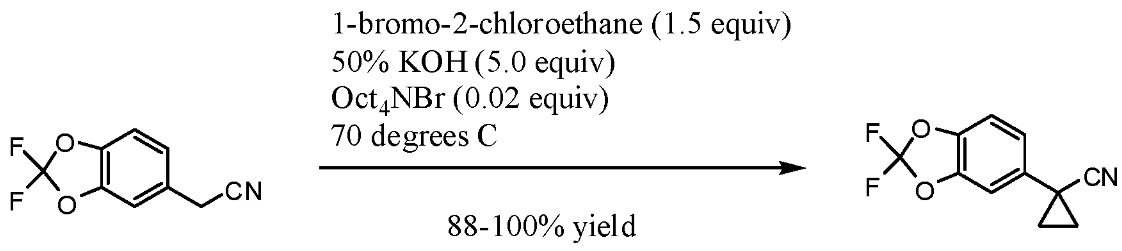


A mixture of (2,2-difluoro-1,3-benzodioxol-5-yl)-acetonitrile (1.0 eq), 50 wt % aqueous KOH (5.0 eq) 1-bromo-2-chloroethane (1.5 eq), and $Oct_4NBr$ (0.02 eq) was heated at 70 °C for 1 h. The reaction mixture was cooled, then worked up with MTBE and water. The organic phase was washed with water and brine. The solvent was removed to afford (2,2-difluoro-1,3-benzodioxol-5-yl)-cyclopropanecarbonitrile.

**Preparation of 1-(2,2-difluoro-1,3-benzodioxol-5-yl)-cyclopropanecarboxylic acid.**

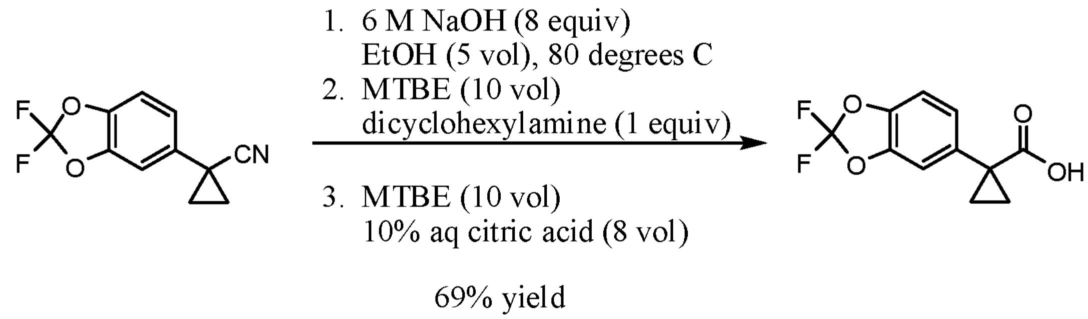


(2,2-difluoro-1,3-benzodioxol-5-yl)-cyclopropanecarbonitrile was hydrolyzed using 6 M NaOH (8 equiv) in ethanol (5 vol) at 80 °C overnight. The mixture was cooled to room temperature and the ethanol was evaporated under vacuum. The residue was taken up in water and MTBE, 1 M HCl was added, and the layers were separated. The MTBE layer was then treated with dicyclohexylamine (DCHA) (0.97 equiv). The slurry was cooled to 0 °C, filtered and washed with heptane to give the corresponding DCHA salt. The salt was taken into MTBE and 10% citric acid and stirred until all the solids had dissolved. The layers were separated and the MTBE layer was washed with water and brine. A solvent swap to heptane followed by filtration gave 1-(2,2-difluoro-1,3-benzodioxol-5-yl)-cyclopropanecarboxylic acid after drying in a vacuum oven at 50 °C overnight.

**Preparation of 1-(2,2-difluoro-1,3-benzodioxol-5-yl)-cyclopropanecarbonyl chloride.**

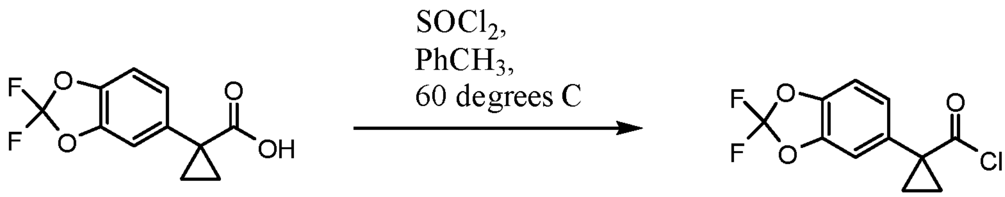


1-(2,2-difluoro-1,3-benzodioxol-5-yl)-cyclopropanecarboxylic acid (1.2 eq) is slurried in toluene (2.5 vol) and the mixture was heated to 60 °C. $SOCl_2$ (1.4 eq) was added via addition funnel. The toluene and $SOCl_2$ were distilled from the reaction mixture after 30 minutes. Additional toluene (2.5 vol) was added and the resulting mixture was distilled again, leaving the product acid chloride as an oil, which was used without further purification.

**Preparation of *tert*-butyl-3-(3-methylpyridin-2-yl)benzoate.**

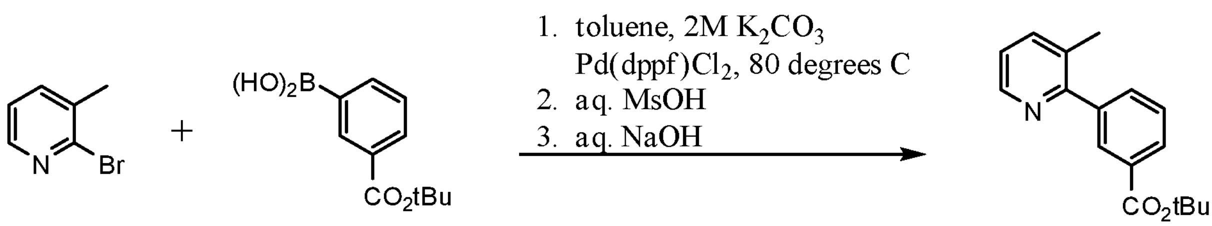


2-Bromo-3-methylpyridine (1.0 eq) was dissolved in toluene (12 vol). $K_2CO_3$ (4.8 eq) was added, followed by water (3.5 vol). The resulting mixture was heated to 65 °C under a stream of $N_2$ for 1 hour. 3-(*t*-Butoxycarbonyl)phenylboronic acid (1.05 eq) and $Pd(dppf)Cl_2 \cdot CH_2Cl_2$ (0.015 eq) were then added and the mixture was heated to 80 °C. After 2 hours, the heat was turned off, water was added (3.5 vol), and the layers were allowed to separate. The organic phase was then washed with water (3.5 vol) and extracted with 10% aqueous methanesulfonic acid (2 eq MsOH, 7.7 vol). The aqueous phase was made basic with 50% aqueous NaOH (2 eq) and extracted with EtOAc (8 vol). The organic layer was concentrated to afford crude *tert*-butyl-3-(3-methylpyridin-2-yl)benzoate (82%) that was used directly in the next step.

**Preparation of 2-(3-(*tert*-butoxycarbonyl)phenyl)-3-methylpyridine-1-oxide.**

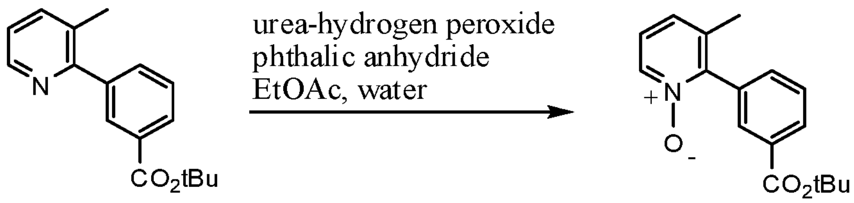


*tert*-Butyl-3-(3-methylpyridin-2-yl)benzoate (1.0 eq) was dissolved in EtOAc (6 vol). Water (0. 3 vol) was added, followed by urea-hydrogen peroxide (3 eq). Phthalic anhydride (3 eq) was then added portionwise to the mixture as a solid at a rate to maintain the temperature in the reactor below 45 °C. After completion of the phthalic anhydride addition, the mixture was heated to 45 °C. After stirring for an additional 4 hours, the heat was turned off. 10% w/w aqueous $Na_2SO_3$ (1.5 eq) was added via addition funnel. After completion of $Na_2SO_3$ addition, the mixture was stirred for an additional 30 min and the layers separated. The organic layer was stirred and 10% wt/wt aqueous. $Na_2CO_3$ (2 eq) was added. After stirring for 30 minutes, the layers were allowed to separate. The organic phase was washed 13% w/v aq NaCl. The organic phase was then filtered and concentrated to afford crude 2-(3-(*tert*-butoxycarbonyl)phenyl)-3-methylpyridine-1-oxide (95%) that was used directly in the next step.

**Preparation of *tert*-butyl-3-(6-amino-3-methylpyridin-2-yl)benzoate.**

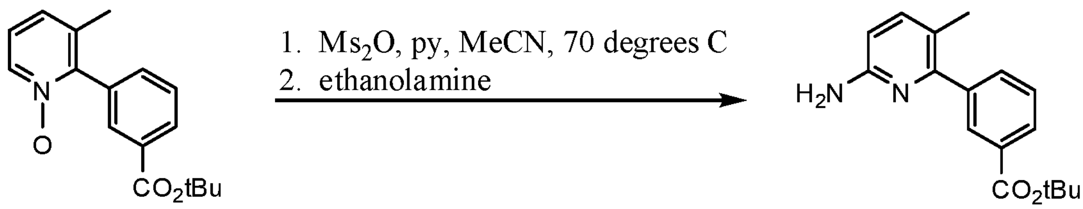


A solution of 2-(3-(*tert*-butoxycarbonyl)phenyl)-3-methylpyridine-1-oxide (1 eq) and pyridine (4 eq) in acetonitrile (8 vol) was heated to 70 °C. A solution of methanesulfonic anhydride (1.5 eq) in MeCN (2 vol) was added over 50 min via addition funnel while maintaining the temperature at less than 75 °C. The mixture was stirred for an additional 0.5 hours after complete addition. The mixture was then allowed to cool to ambient. Ethanolamine (10 eq) was added via addition funnel. After stirring for 2 hours, water (6 vol) was added and the mixture was cooled to 10 °C. After stirring for 3 hours, the solid was collected by filtration and washed with water (3 vol), 2:1 acetonitrile/water (3 vol), and acetonitrile (2 x 1.5 vol). The solid was dried to constant weight (<1% difference) in a vacuum oven at 50 °C with a slight $N_2$ bleed to afford *tert*-butyl-3-(6-amino-3-methylpyridin-2-yl)benzoate as a red-yellow solid (53% yield).

**Preparation of 3-(6-(1-(2,2-difluorobenzo[d][1,3]dioxol-5-yl)-cyclopropanecarboxamido)-3-methylpyridin-2-yl)-t-butylbenzoate.**

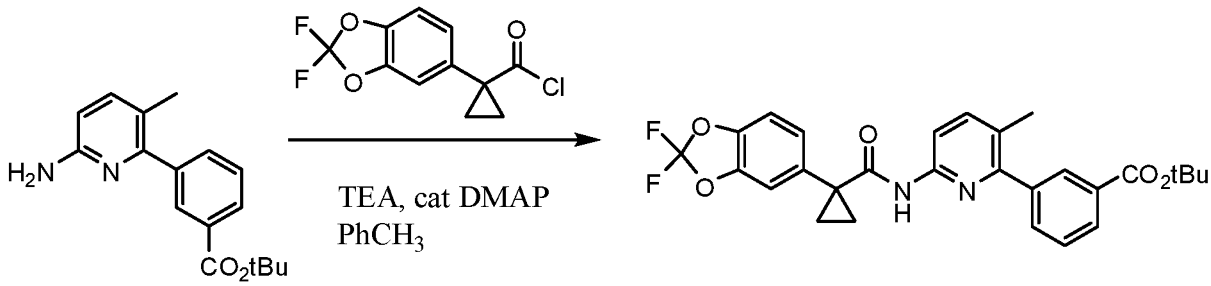

The crude acid chloride described above was dissolved in toluene (2.5 vol based on acid chloride) and added via addition funnel to a mixture of *tert*-butyl-3-(6-amino-3-methylpyridin-2-yl)benzoate (1 eq), DMAP, (0.02 eq), and triethylamine (3.0 eq) in toluene (4 vol based on *tert*-butyl-3-(6-amino-3-methylpyridin-2-yl)benzoate). After 2 hours, water (4 vol based on *tert*-butyl-3-(6-amino-3-methylpyridin-2-yl)benzoate) was added to the reaction mixture. After stirring for 30 minutes, the layers were separated. The organic phase was then filtered and concentrated to afford a thick oil of 3-(6-(1-(2,2-difluorobenzo[d][1,3]dioxol-5-yl) cyclopropanecarboxamido)-3-methylpyridin-2-yl)-t-butylbenzoate (quantitative crude yield). Acetonitrile (3 vol based on crude product) was added and distilled until crystallization occurs. Water (2 vol based on crude product) was added and the mixture stirred for 2 h. The solid was collected by filtration, washed with 1:1 (by volume) acetonitrile/water (2 x 1 volumes based on crude product), and partially dried on the filter under vacuum. The solid was dried to a constant weight (<1% difference) in a vacuum oven at 60 °C with a slight $N_2$ bleed to afford 3-(6-(1-(2,2-difluorobenzo[d][1,3]dioxol-5-yl) cyclopropanecarboxamido)-3-methylpyridin-2-yl)-t-butylbenzoate as a brown solid.

**Preparation of 3-(6-(1-(2,2-difluorobenzo[d][1,3]dioxol-5-yl) cyclopropanecarboxamido)-3-methylpyridin-2-yl)benzoic acid • HCL salt.**

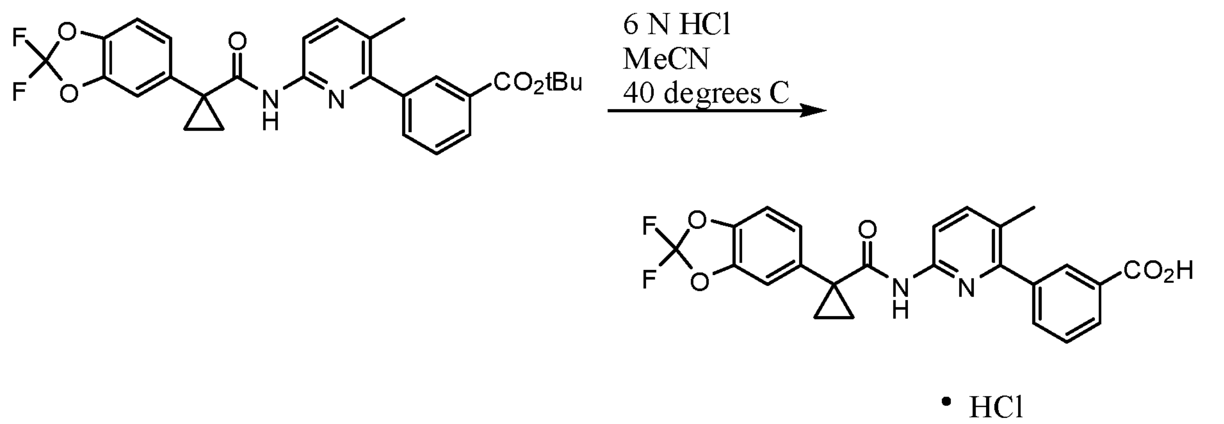


To a slurry of 3-(6-(1-(2,2-difluorobenzo[d][1,3]dioxol-5-yl) cyclopropanecarboxamido)-3-methylpyridin-2-yl)-t-butylbenzoate (1.0 eq) in MeCN (3.0 vol) was added water (0.83 vol) followed by concentrated aqueous HCl (0.83 vol). The mixture was heated to 45 $\pm$ 5 °C. After stirring for 24 to 48 h, the reaction was complete, and the mixture was allowed to cool to ambient. Water (1.33 vol) was added and the mixture stirred. The solid was collected by filtration, washed with water (2 x 0.3 vol), and partially dried on the filter under vacuum. The solid was dried to a constant weight (<1% difference) in a vacuum oven at 60 °C with a slight $N_2$ bleed to afford 3-(6-(1-(2,2-difluorobenzo[d][1,3]dioxol-5-yl) cyclopropanecarboxamido)-3-methylpyridin-2-yl)benzoic acid • HCl as an off-white solid.

An $^1$HNMR spectrum of Compound 1 is shown in Figure 8 and Figure 9 depicts an $^1$HNMR spectrum of Compound 1 as an HCl salt.

Table 2 below recites the $^1$HNMR data for Compound I.

**Table 2.**

| Compound No | LC/MS M + 1 | LC/RT minutes | NMR |
|---|---|---|---|
| 1 | 453.3 | 1.93 | $^1$HNMR (400 MHz, DMSO-d6) 9.14 (s, 1H), 7.99-7.93 (m, 3H), 7.80-7.78 (m,1H), 7.74-7.72 (m,1H), 7.60-7.55 (m,2H), 7.41-7.33 (m, 2H), 2.24 (s, 3H), 1.53-1.51 (m, 2H), 1.19-1.17 (m, 2H). |

**Preparation of Compound 1 Form I**

**Preparation of Compound 1 Form I, Method A.**

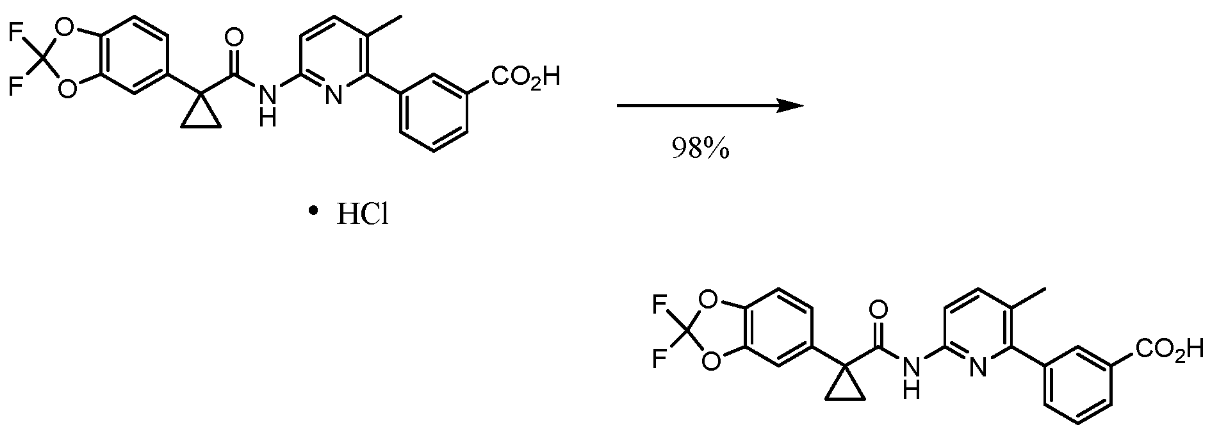


Form I

A slurry of 3-(6-(1-(2,2-difluorobenzo[d][1,3]dioxol-5-yl) cyclopropanecarboxamido)-3-methylpyridin-2-yl)benzoic acid • HCl (1 eq) in water (10 vol) was stirred at ambient temperature. A sample was taken after stirring for 24 h. The sample was filtered and the solid was washed with water (2 times). The solid sample was submitted for DSC analysis. When DSC analysis indicated complete conversion to Form I, the solid was collected by filtration, washed with water (2 x 1.0 vol), and partially dried on a filter under vacuum. The solid was then dried to a constant weight (<1% difference) in a vacuum oven at 60 °C with a slight $N_2$ bleed to afford Compound 1 Form I as an off-white solid (98% yield). $^1H$ NMR (400 MHz, DMSO-d6) 9.14 (s, 1H), 7.99-7.93 (m, 3H), 7.80-7.78 (m, 1H), 7.74-7.72 (m, 1H), 7.60-7.55 (m, 2H), 7.41-7.33 (m, 2H), 2.24 (s, 3H), 1.53-1.51 (m, 2H), 1.19-1.17 (m, 2H).

**Preparation of Compound 1 Form I, Method B.**

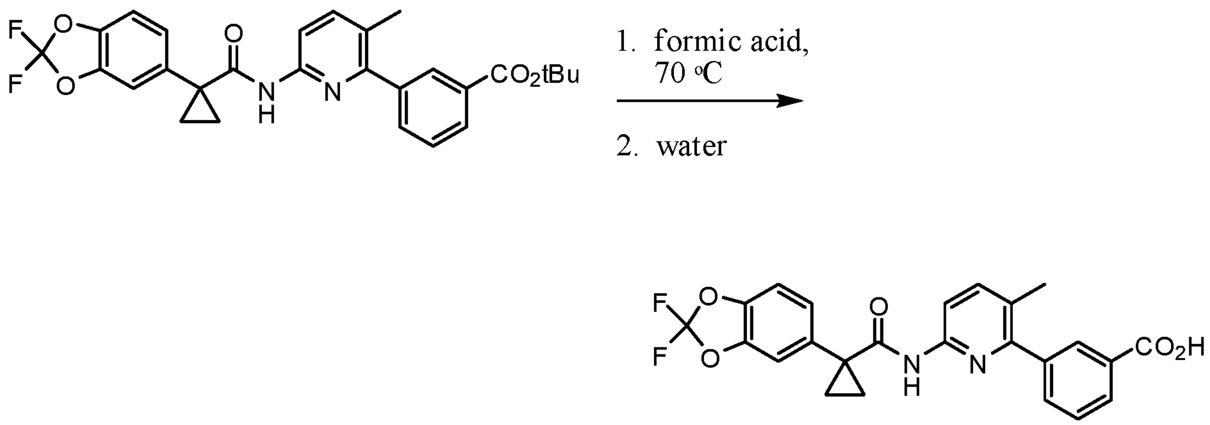


Form I

A solution of 3-(6-(1-(2,2-difluorobenzo[d][1,3]dioxol-5-yl) cyclopropanecarboxamido)-3-methylpyridin-2-yl)-t-butylbenzoate (1.0 eq) in formic acid (3.0 vol) was heated with stirring to 70 ± 10 °C, for 8 h. The reaction was deemed complete when no more than 1.0% AUC by chromatographic methods of 3-(6-(1-(2,2-difluorobenzo[d][1,3]dioxol-5-yl) cyclopropanecarboxamido)-3-methylpyridin-2-yl)-t-butylbenzoate) remained. The mixture was allowed to cool to ambient. The solution was added to water (6 vol), heated at 50 °C, and the mixture was stirred. The mixture was then heated to 70 ± 10 °C until the level of 3-(6-(1-(2,2-difluorobenzo[d][1,3]dioxol-5-yl) cyclopropanecarboxamido)-3-methylpyridin-2-yl)-t-butylbenzoate was no more than 0.8% (AUC). The solid was collected by filtration, washed with water (2 x 3 vol), and partially dried on the filter under vacuum. The solid was dried to a constant weight (<1% difference) in a vacuum oven at 60 °C with a slight $N_2$ bleed to afford Compound 1 Form I as an off-white solid.

The DSC trace of Compound 1 Form I is shown in Figure 10. Melting for Compound 1 Form I occurs at about

204 °C.

An X-ray diffraction pattern was calculated from a single crystal structure of Compound 1 Form I and is shown in Figure 1. Table 3 lists the calculated peaks for Figure 1.

**Table 3.**

| Peak Rank | 2θ Angle [degrees] | Relative Intensity [%] |
|---|---|---|
| 11 | 14.41 | 48.2 |
| 8 | 14.64 | 58.8 |
| 1 | 15.23 | 100.0 |
| 2 | 16.11 | 94.7 |
| 3 | 17.67 | 81.9 |
| 7 | 19.32 | 61.3 |
| 4 | 21.67 | 76.5 |
| 5 | 23.40 | 68.7 |
| 9 | 23.99 | 50.8 |
| 6 | 26.10 | 67.4 |
| 10 | 28.54 | 50.1 |

An actual X-ray powder diffraction pattern of Compound 1 Form I is shown in Figure 2. Table 4 lists the actual peaks for Figure 2.

**Table 4.**

| Peak Rank | 2θ Angle [degrees] | Relative Intensity [%] |
|---|---|---|
| 7 | 7.83 | 37.7 |
| 3 | 14.51 | 74.9 |
| 4 | 14.78 | 73.5 |
| 1 | 15.39 | 100.0 |
| 2 | 16.26 | 75.6 |
| 6 | 16.62 | 42.6 |
| 5 | 17.81 | 70.9 |
| 9 | 21.59 | 36.6 |
| 10 | 23.32 | 34.8 |
| 11 | 24.93 | 26.4 |
| 8 | 25.99 | 36.9 |

Colorless crystals of Compound 1 Form I were obtained by cooling a concentrated 1-butanol solution from 75°C to 10 °C at a rate of 0.2 °C/min. A crystal with dimensions of 0.50 *x* 0.08 *x* 0.03 mm was selected, cleaned with mineral oil, mounted on a MicroMount and centered on a Bruker *APEX* II system. Three batches of 40 frames separated in reciprocal space were obtained to provide an orientation matrix and initial cell parameters. Final cell parameters were obtained and refined based on the full data set.

A diffraction data set of reciprocal space was obtained to a resolution of 0.82 Å using 0.5° steps using 30 s exposure for each frame. Data were collected at 100 (2) K. Integration of intensities and refinement of cell parameters were accomplished using APEXII software. Observation of the crystal after data collection showed no signs of decomposition.

A conformational picture of Compound 1 Form I based on single crystal X-ray analysis is shown in Figure 11. Compound 1 Form I is monoclinic, $P_2 1/n$, with the following unit cell dimensions: a=4.9626(7) Å, b=12.299(2) Å, c=33.075

(4) Å, β=93.938(9)°, V=2014.0 $Å^3$, Z=4. Density of Compound 1 Form I calculated from structural data is 1.492 $g/cm^3$ at 100 K.

**Preparation of Compound 2**

**Synthesis of 4-oxo-1,4-dihydroquinoline-3-carboxylic acid (26)**

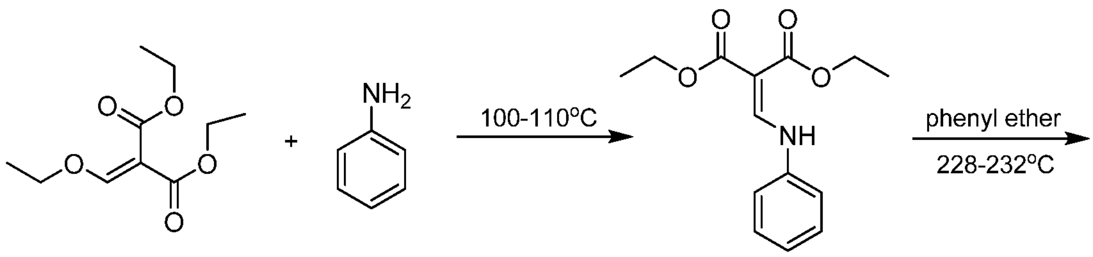


**22** **23** **24**

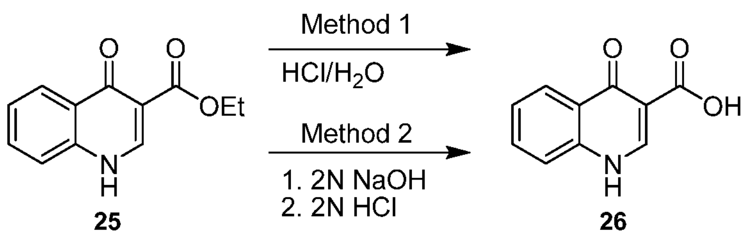


**25** **26**

**Procedure for the preparation of ethyl 4-oxo-1,4-dihydroquinoline-3-carboxylate (25)**

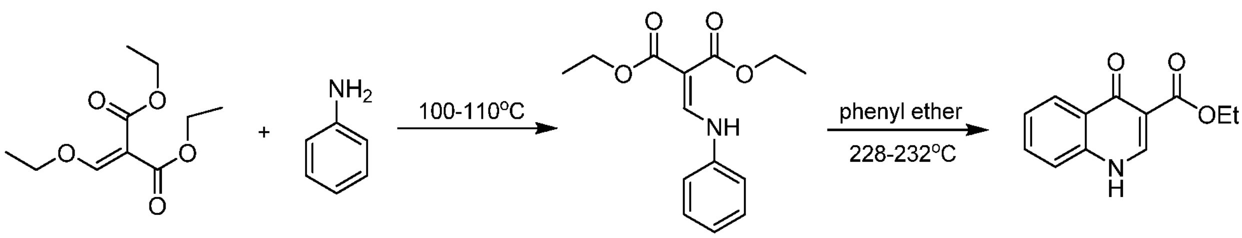


**22** **23** **24** **25**

Compound **23** (4.77 g, 47.7 mmol) was added dropwise to compound **22** (10 g, 46.3 mmol) with subsurface $N_2$ flow to drive out ethanol below 30 °C for 0.5 hours. The solution was then heated to 100-110 °C and stirred for 2.5 hours. After cooling the mixture to below 60 °C, diphenyl ether was added. The resulting solution was added dropwise to diphenyl ether that had been heated to 228-232 °C for 1.5 hours with subsurface $N_2$ flow to drive out ethanol. The mixture was stirred at 228-232 °C for another 2 hours, cooled to below 100 °C and then heptane was added to precipitate the product. The resulting slurry was stirred at 30 °C for 0.5 hours. The solids were then filtrated, and the cake was washed with heptane and dried *in vacuo* to give compound **25** as brown solid. $^1H$ NMR (DMSO-$d_6$; 400 MHz) δ 12.25 (s), δ 8.49 (d), δ 8.10 (m), δ 7.64 (m), δ 7.55 (m), δ 7.34 (m), δ 4.16 (q), δ 1.23 (t).

**Procedure for the preparation of 4-oxo-1,4-dihydroquinoline-3-carboxylic acid (26)**

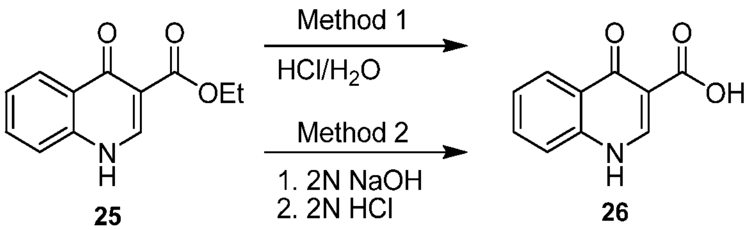


25 26

**Method 1**

Compound **25** (1.0 eq) was suspended in a solution of HCl (10.0 eq) and $H_2O$ (11.6 vol). The slurry was heated to 85 - 90 °C, although alternative temperatures are also suitable for this hydrolysis step. For example, the hydrolysis can alternatively be performed at a temperature of from about 75 to about 100 °C. In some instances, the hydrolysis is performed at a temperature of from about 80 to about 95 °C. In others, the hydrolysis step is performed at a temperature of from about 82 to about 93 °C (e.g., from about 82.5 to about 92.5 °C or from about 86 to about 89 °C). After stirring at 85 - 90 °C for approximately 6.5 hours, the reaction was sampled for reaction completion. Stirring may be performed under any of the temperatures suited for the hydrolysis. The solution was then cooled to 20 - 25 °C and filtered. The reactor/cake was rinsed with $H_2O$ (2 vol x 2). The cake was then washed with 2 vol $H_2O$ until the pH ≥ 3.0. The cake was then dried under vacuum at 60 °C to give compound **26.**

**Method 2**

Compound **25** (11.3 g, 52 mmol) was added to a mixture of 10% NaOH (aq) (10 mL) and ethanol (100 mL). The solution was heated to reflux for 16 hours, cooled to 20-25 °C and then the pH was adjusted to 2-3 with 8% HCl. The mixture was then stirred for 0.5 hours and filtered. The cake was washed with water (50 mL) and then dried *in vacuo* to give compound **26** as a brown solid. $^1H$ NMR (DMSO-$d_6$; 400 MHz) δ 15.33 (s), δ 13.39 (s), δ 8.87 (s), δ 8.26 (m), δ 7.87 (m), δ 7.80 (m), δ 7.56 (m).

**Total synthesis of N-(2,4-di-tert-butyl-5-hydroxyphenyl)-4-oxo-1,4-dihydroquinoline-3-carboxamide (Compound 2)**

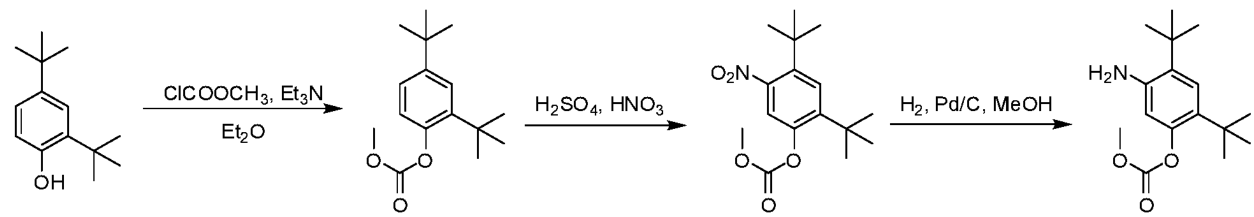


29 30 31 32

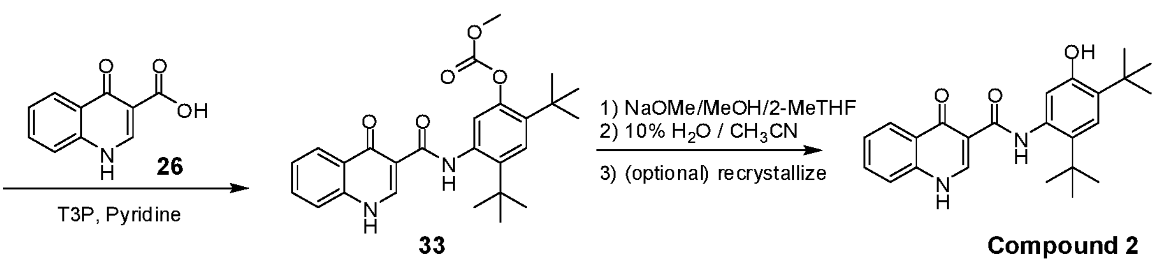


**33** **Compound 2**

**Procedure for the preparation of 2,4-di-tert-butylphenyl methyl carbonate (30)**

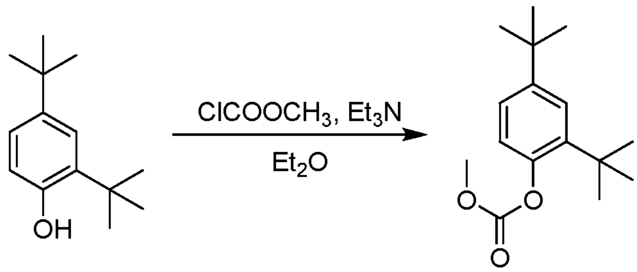


**29** **30**

**Method 1**

To a solution of 2,4-di-*tert*-butyl phenol, **29**, (10 g, 48.5mmol) in diethyl ether (100 mL) and triethylamine (10.1 mL, 72.8 mmol), was added methyl chloroformate (7.46 mL, 97 mmol) dropwise at 0 °C. The mixture was then allowed to warm to room temperature and stir for an additional 2 hours. An additional 5 mL triethylamine and 3.7 mL methyl chloroformate was then added and the reaction stirred overnight. The reaction was then filtered, the filtrate was cooled to 0 °C, and an additional 5 mL triethylamine and 3.7 mL methyl chloroformate was then added and the reaction was allowed to warm to room temperature and then stir for an addition **1** hours. At this stage, the reaction was almost complete and was worked up by filtering, then washing with water (2x), followed by brine. The solution was then concentrated to produce a yellow oil and purified using column chromatography to give compound **30.** $^1$H NMR (400 MHz, DMSO-$d_6$) δ 7.35 (d, *J* = 2.4 Hz, 1H), 7.29 (dd, *J* = 8.4, 2.4 Hz, 1H), 7.06 (d, *J* = 8.4 Hz, 1H), 3.85 (s, 3H), 1.30 (s, 9H), 1.29 (s, 9H).

**Method 2**

To a reactor vessel charged with 4-dimethylaminopyridine (DMAP, 3.16 g, 25.7 mmol) and 2,4-di*tert*-butyl phenol (compound **29,** 103.5 g, 501.6 mmol) was added methylene chloride (415 g, 313 mL) and the solution was agitated until all solids dissolved. Triethylamine (76 g, 751 mmol) was then added and the solution was cooled to 0 - 5 °C. Methyl chloroformate (52 g, 550.3 mmol) was then added dropwise over 2.5 - 4 hours, while keeping the solution temperature between 0 - 5 °C. The reaction mixture was then slowly heated to 23 - 28 °C and stirred for 20 hours. The reaction was then cooled to 10 - 15 °C and charged with 150 mL water. The mixture was stirred at 15 - 20 °C for 35 - 45 minutes and the aqueous layer was then separated and extracted with 150 mL methylene chloride. The organic layers were combined and neutralized with 2.5% HCl (aq) at a temperature of 5 - 20 °C to give a final pH of 5 - 6. The organic layer was then washed with water and concentrated *in vacuo* at a temperature below 20 °C to 150 mL to give compound **30** in methylene chloride.

**Procedure for the preparation of 5-nitro-2,4-di-tert-butylphenyl methyl carbonate (31)**

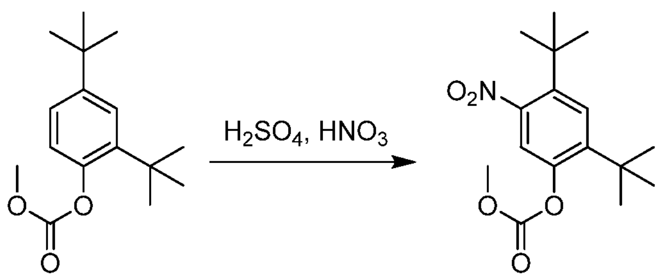


**30** **31**

**Method 1**

To a stirred solution of compound **30** (6.77g, 25.6 mmol) was added 6 mL of a 1:1 mixture of sulfuric acid and nitric acid at 0 °C dropwise. The mixture was allowed to warm to room temperature and stirred for 1 hour. The product was purified using liquid chromatography (ISCO, 120 g, 0-7% EtOAc/Hexanes, 38 min) producing about an 8:1 - 10:1 mixture of regioisomers of compound **31** as a white solid. $^1$H NMR (400 MHz, DMSO-$d_6$) δ 7.63 (s, 1H), 7.56 (s, 1H),

3.87 (s, 3H), 1.36 (s, 9H), 1.32 (s, 9H). HPLC ret. time 3.92 min 10-99% $CH_3CN$, 5 min run; ESI-MS 310 m/z $(MH)^+$.

**Method 2**

To compound **30** (100g, 378 mmol) was added DCM (540 g, 408 mL). The mixture was stirred until all solids dissolved, and then cooled to -5 - 0 °C. Concentrated sulfuric acid (163 g) was then added dropwise, while maintaining the initial temperature of the reaction, and the mixture was stirred for 4.5 hours. Nitric acid (62 g) was then added dropwise over 2-4 hours while maintaining the initial temperature of the reaction, and was then stirred at this temperature for an additional 4.5 hours. The reaction mixture was then slowly added to cold water, maintaining a temperature below 5 °C. The quenched reaction was then heated to 25 °C and the aqueous layer was removed and extracted with methylene chloride. The combined organic layers were washed with water, dried using $Na_2SO_4$, and concentrated to 124 - 155 mL. Hexane (48 g) was added and the resulting mixture was again concentrated to 124 - 155 mL. More hexane (160 g) was subsequently added to the mixture. The mixture was then stirred at 23 - 27 °C for 15.5 hours, and was then filtered. To the filter cake was added hexane (115 g), the resulting mixture was heated to reflux and stirred for 2 - 2.5 hours. The mixture was then cooled to 3 - 7 °C, stirred for an additional 1 - 1.5 hours, and filtered to give compound **31** as a pale yellow solid.

**Procedure for the preparation of 5-amino-2,4-di-tert-butylphenyl methyl carbonate (32)**

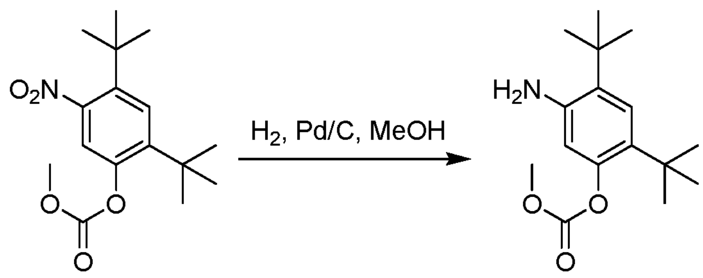


**31** **32**

2,4-Di-tert-butyl-5-nitrophenyl methyl carbonate (1.00 eq) was charged to a suitable hydrogenation reactor, followed by 5% Pd/C (2.50 wt% dry basis, Johnson-Matthey Type 37). MeOH (15.0 vol) was charged to the reactor, and the system was closed. The system was purged with $N_2$ (g), and was then pressurized to 2.0 Bar with $H_2$ (g). The reaction was performed at a reaction temperature of 25 °C +/- 5 °C. When complete, the reaction was filtered, and the reactor/cake was washed with MeOH (4.00 vol). The resulting filtrate was distilled under vacuum at no more than 50 °C to 8.00 vol. Water (2.00 vol) was added at 45 °C +/- 5 °C. The resultant slurry was cooled to 0 °C +/- 5. The slurry was held at 0 °C +/- 5 °C for no less than 1 hour, and filtered. The cake was washed once with 0 °C +/- 5 °C MeOH/$H_2O$ (8:2) (2.00 vol). The cake was dried under vacuum (-0.90 bar and -0.86 bar) at 35 °C - 40 °C to give compound **32.** $^1H$ NMR (400 MHz, DMSO-$d_6$) $\delta$ 7.05 (s, 1H), 6.39 (s, 1H), 4.80 (s, 2H), 3.82 (s, 3H), 1.33 (s, 9H), 1.23 (s, 9H).

Once the reaction was complete, the resulting mixture was diluted with from about 5 to 10 volumes of MeOH (e.g., from about 6 to about 9 volumes of MeOH, from about 7 to about 8.5 volumes of MeOH, from about 7.5 to about 8 volumes of MeOH, or about 7.7 volumes of MeOH), heated to a temperature of about 35 $\pm$ 5 °C, filtered, washed, and dried, as described above.

**Preparation of N-(2,4-di-tert-butyl-5-hydroxyphenyl)-4-oxo-1,4-dihydroquinoline-3-carboxamide (Compound 2).**

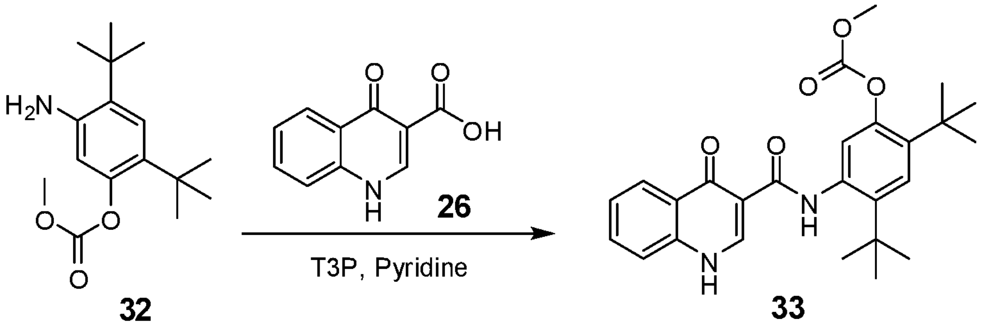


32 26 33

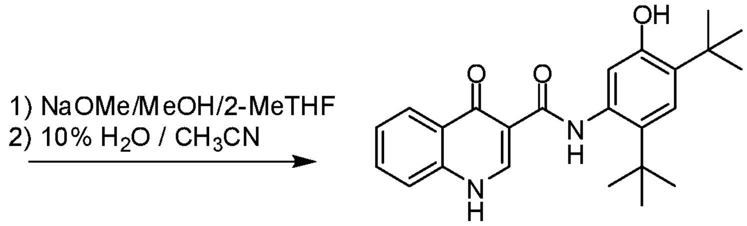


**Compound 2**

4-Oxo-1,4-dihydroquinoline-3-carboxylic acid, **26,** (1.0 eq) and 5-amino-2,4-di-tert-butylphenyl methyl carbonate, **32,** (1.1 eq) were charged to a reactor. 2-MeTHF (4.0 vol, relative to the acid) was added followed by T3P® 50% solution in 2-MeTHF (1.7 eq). The T3P charged vessel was washed with 2-MeTHF (0.6 vol). Pyridine (2.0 eq) was then added, and the resulting suspension was heated to 47.5 +/- 5.0 °C and held at this temperature for 8 hours. A sample was taken and checked for completion by HPLC. Once complete, the resulting mixture was cooled to 25.0 °C +/- 2.5 °C. 2-MeTHF was added (12.5 vol) to dilute the mixture. The reaction mixture was washed with water (10.0 vol) 2 times. 2-MeTHF was added to bring the total volume of reaction to 40.0 vol (~16.5 vol charged). To this solution was added NaOMe/MeOH (1.7 equiv) to perform the methanolysis. The reaction was stirred for no less than 1.0 hour, and checked for completion by HPLC. Once complete, the reaction was quenched with 1 N HCl (10.0 vol), and washed with 0.1 N HCl (10.0 vol). The organic solution was polish filtered to remove any particulates and placed in a second reactor. The filtered solution was concentrated at no more than 35 °C (jacket temperature) and no less than 8.0 °C (internal reaction temperature) under reduced pressure to 20 vol. $CH_3CN$ was added to 40 vol and the solution concentrated at no more than 35 °C (jacket temperature) and no less than 8.0 °C (internal reaction temperature) to 20 vol. The addition of $CH_3CN$ and concentration cycle was repeated 2 more times for a total of 3 additions of $CH_3CN$ and 4 concentrations to 20 vol. After the final concentration to 20 vol, 16.0 vol of $CH_3CN$ was added followed by 4.0 vol of $H_2O$ to make a final concentration of 40 vol of 10% $H_2O/CH_3CN$ relative to the starting acid. This slurry was heated to 78.0 °C +/- 5.0 °C (reflux). The slurry was then stirred for no less than 5 hours. The slurry was cooled to 0.0 °C +/- 5 °C over 5 hours, and filtered. The cake was washed with 0.0 °C +/- 5.0 °C $CH_3CN$ (5 vol) 4 times. The resulting solid (Compound 2) was dried in a vacuum oven at 50.0 °C +/- 5.0 °C. $^1H$ NMR (400 MHz, DMSO-$d_6$) δ 12.8 (s, 1H), 11.8 (s, 1H), 9.2 (s, 1H), 8.9 (s, 1H), 8.3 (s, 1H), 7.2 (s, 1H), 7.9 (t, 1H), 7.8 (d, 1H), 7.5 (t, 1H), 7.1 (s, 1H), 1.4 (s, 9H), 1.4 (s, 9H).

**Alternative Preparation of N-(2,4-di-tert-butyl-5-hydroxyphenyl)-4-oxo-1,4-dihydroquinoline-3-carboxamide (Compound 2).**

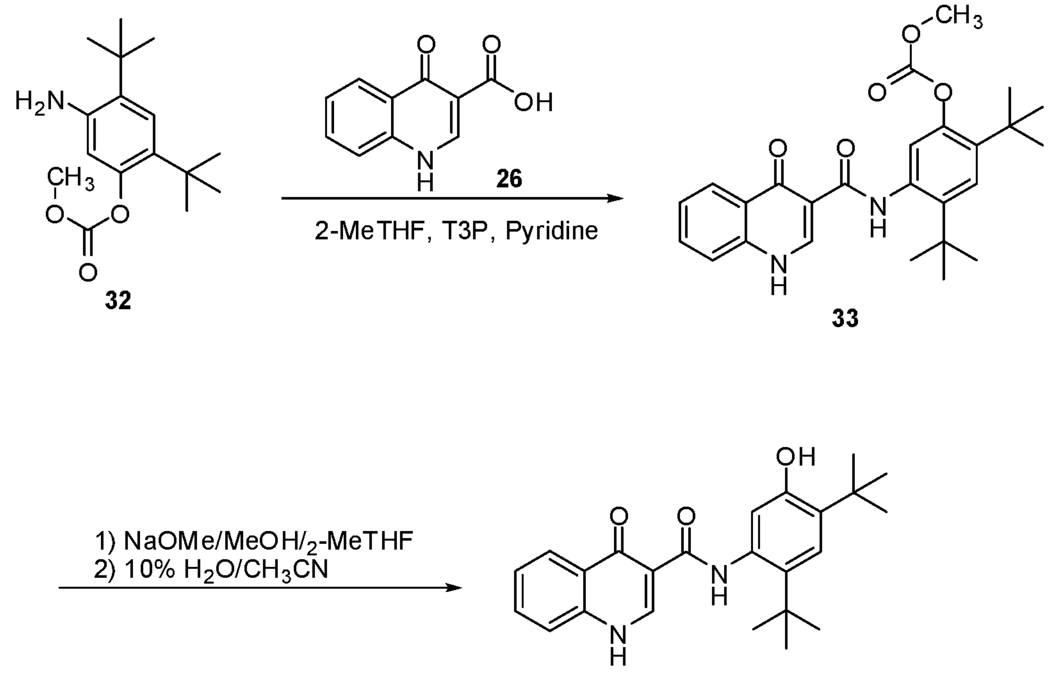


32 26 33

**Compound 2**

4-Oxo-1,4-dihydroquinoline-3-carboxylic acid, **26,** (1.0 eq) and 5-amino-2,4-di-tert-butylphenyl methyl carbonate, **32,** (1.1 eq) were charged to a reactor. 2-MeTHF (4.0 vol, relative to the acid) was added followed by T3P® 50% solution in 2-MeTHF (1.7 eq). The T3P charged vessel was washed with 2-MeTHF (0.6 vol). Pyridine (2.0 eq) was then added, and the resulting suspension was heated to 47.5 +/- 5.0 °C and held at this temperature for 8 hours. A sample was taken and checked for completion by HPLC. Once complete, the resulting mixture was cooled to 20 °C +/- 5 °C. 2-MeTHF was added (12.5 vol) to dilute the mixture. The reaction mixture was washed with water (10.0 vol) 2 times and 2-MeTHF (16.5 vol) was charged to the reactor. This solution was charged with 30% w/w NaOMe/MeOH (1.7 equiv) to perform the methanolysis. The reaction was stirred at 25.0 °C +/- 5.0 °C for no less than 1.0 hour, and checked for completion by HPLC. Once complete, the reaction was quenched with 1.2 N HCl/$H_2O$ (10.0 vol), and washed with 0.1 N HCl/$H_2O$ (10.0 vol). The organic solution was polish filtered to remove any particulates and placed in a second reactor.

The filtered solution was concentrated at no more than 35 °C (jacket temperature) and no less than 8.0 °C (internal reaction temperature) under reduced pressure to 20 vol. $CH_3CN$ was added to 40 vol and the solution concentrated at no more than 35 °C (jacket temperature) and no less than 8.0 °C (internal reaction temperature) to 20 vol. The addition of $CH_3CN$ and concentration cycle was repeated 2 more times for a total of 3 additions of $CH_3CN$ and 4 concentrations to 20 vol. After the final concentration to 20 vol, 16.0 vol of $CH_3CN$ was charged followed by 4.0 vol of $H_2O$ to make a final concentration of 40 vol of 10% $H_2O/CH_3CN$ relative to the starting acid. This slurry was heated to 78.0 °C +/- 5.0 °C (reflux). The slurry was then stirred for no less than 5 hours. The slurry was cooled to 20 to 25 °C over 5 hours, and filtered. The cake was washed with $CH_3CN$ (5 vol) heated to 20 to 25 °C 4 times. The resulting solid (Compound 2) was dried in a vacuum oven at 50.0 °C +/- 5.0 °C. $^1H$ NMR (400 MHz, DMSO-$d_6$) δ 12.8 (s, 1H), 11.8 (s, 1H), 9.2 (s, 1H), 8.9 (s, 1H), 8.3 (s, 1H), 7.2 (s, 1H), 7.9 (t, 1H), 7.8 (d, 1H), 7.5 (t, 1H), 7.1 (s, 1H), 1.4 (s, 9H), 1.4 (s, 9H).

**Procedure for the recrystallization of N-(2,4-di-tert-butyl-5-hydroxyphenyl)-4-oxo-1,4-dihydroquinoline-3-carboxamide (Compound 2)**

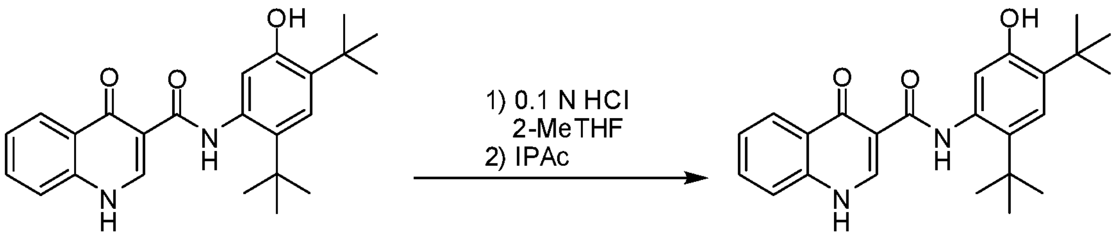


Compound 2 (1.0 eq) was charged to a reactor. 2-MeTHF (20.0 vol) was added followed by 0.1N HCl (5.0 vol). The biphasic solution was stirred and separated and the top organic phase was washed twice more with 0.1N HCl (5.0 vol). The organic solution was polish filtered to remove any particulates and placed in a second reactor. The filtered solution was concentrated at no more than 35 °C (jacket temperature) and no more than 8.0 °C (internal reaction temperature) under reduced pressure to 10 vol. Isopropyl acetate (IPAc) (10 vol) was added and the solution concentrated at no more than 35 °C (jacket temperature) and no more than 8.0 °C (internal reaction temperature) to 10 vol. The addition of IPAc and concentration was repeated 2 more times for a total of 3 additions of IPAc and 4 concentrations to 10 vol. After the final concentration, 10 vol of IPAc was charged and the slurry was heated to reflux and maintained at this temperature for 5 hours. The slurry was cooled to 0.0 °C +/- 5 °C over 5 hours and filtered. The cake was washed with IPAc (5 vol) once. The resulting solid was dried in a vacuum oven at 50.0 °C +/- 5.0 °C.

**Preparation of a Solid Dispersion Comprising Substantially Amorphous Compound 2**

A solvent system of MEK and DI water, formulated according to the ratio 90 wt% MEK / 10 wt% DI water, was heated to a temperature of 20 - 30 °C in a reactor, equipped with a magnetic stirrer and thermal circuit. Into this solvent system, hypromellose acetate succinate polymer (HPMCAS)(HG grade), SLS, and Compound 2 were added according to the ratio 19.5 wt% hypromellose acetate succinate / 0.5 wt% SLS / 80 wt% Compound 2. The resulting mixture contained 10.5 wt% solids. The actual amounts of ingredients and solvents used to generate this mixture are recited in Table 5, below:

**Table 5:** Solid Spray Dispersion Ingredients for Intermediate F.

| | Units | Batch |
|---|---|---|
| Compound 2 | Kg | 70.0 |
| HPMCAS | Kg | 17.1 |
| SLS | Kg | 0.438 |
| **Total Solids** | **Kg** | **87.5** |
| MEK | Kg | 671 |
| Water | Kg | 74.6 |
| **Total Solvents** | **Kg** | **746** |
| **Total Spray Solution Weight** | **Kg** | **833** |

The mixture temperature was adjusted to a range of 20 - 45 °C and mixed until it was substantially homogenous and all components were substantially dissolved.

A spray drier, Niro PSD4 Commercial Spray Dryer, fitted with pressure nozzle (Spray Systems Maximum Passage series SK-MFP having orifice/core size 54/21) equipped with anti-bearding cap, was used under normal spray drying mode, following the dry spray process parameters recited in Table 6, below.

**Table 6:** Dry spray process parameters used to generate Intermediate F.

| **Parameter** | **Value** |
|---|---|
| Feed Pressure | 20 bar |
| Feed Flow Rate | 92 - 100 Kg/hr |
| Inlet Temperature | 93 - 99 °C |

(continued)

| Parameter | Value |
|---|---|
| Outlet Temperature | 53 - 57 °C |
| Vacuum Dryer Temperature | 80 °C for 2 hours then 110 °C (+/-5 °C) |
| Vacuum Drying Time | 20 - 24 hours |

A high efficiency cyclone separated the wet product from the spray gas and solvent vapors. The wet product contained 8.5 - 9.7% MEK and 0.56 - 0.83% Water and had a mean particle size of 17 - 19um and a bulk density of 0.27 - 0.33g/cc. The wet product was transferred to a 4000L stainless steel double cone vacuum dryer for drying to reduce residual solvents to a level of less than about 5000 ppm and to generate dry spray dry dispersion of amorphous Compound 2, containing <0.03% MEK and 0.3% Water.

**Tablet Formation from a Fully Continuous Wet Granulation Process**

Equipment/Process

*Equipment*

Fully Continuous Development and Launch Rig (DLR) or similar type of equipment.

*Screening*

Compound 1 Form I, the solid dispersion comprising substantially amorphous Compound 2, and excipients may be dispensed in separate intermediate bin containers (IBCs). These materials may be screened using a "bin-to-bin" screening operation. Appropriate screen sizes are mesh 20, mesh 40, or mesh 60.

*Blending*

The IBCs containing the screened Compound 1 Form I, the solid dispersion comprising substantially amorphous Compound 2, and excipients may be docked to the a feeder system, which can feed the materials in a controlled manner, e.g. using volumetric or gravimetric loss in weight feeders, into a continuous blender. The feed rates of the individual components is defined by the formulation composition and the overall line rate. The line rate may be 8 kg/hr to 30 kg/hr. The continuous blender can have different blade configurations to allow appropriate blending and the rotational speed of these blades may be between 80 RPM and 300 RPM.

*Wet Granulation*

A granulation solution may be prepared by dissolving 48 g sodium lauryl sulfate and 159 g polyvinylpyrrolidone in 1,626 g water in a stainless steel container, using an overhead stirrer with a stirring speed of 700 RPM. The granulation solution may be placed in a container from which the solution may be pumped into the twin screw granulator using a peristaltic pump with a mass flow meter and control, using a flow rate that is appropriate for the process. The blend may be granulated using a twin screw granulator such as the granulator that is part of the DLR. The blend may be added to the twin screw granulator using a Loss in Weight feeder, such as the K-Tron feeder on the DLR, with a feed rate of 8 kg/hr to 24 kg/hr. The twin screw granulator may be operated with a barrel temperature of 25 degrees Celsius and a screw speed of 200 to 950 RPM. The granulation process may be performed for three minutes for small batch sizes or several hours for large batch sizes.

*Drying*

The wet granules may be fed directly into a fluid bed dryer, such as the segmented fluid bed dryer on the DLR. The drying end-point may be chosen at a product temperature during discharge ranging from 40 to 55 degrees Celsius at which point the water content of the granules may be 2.1 %w/w ("Loss on Drying, LOD") or less. The drying time may be 12 minutes, or shorter or longer, to reach the desired drying endpoint.

*Milling*

The dried granules may be milled to reduce the size of the granules. A cone mill such as the integrated Quadro U10 CoMil may be used for this.

*Blending*

The granules may be blended with extra-granular excipients such as fillers and lubricant using loss in weight feeders and a continuous blender. The blending speed may be 80 - 300 RPM.

*Compression*

The compression blend may be compressed into tablets using a single station or rotary tablet press, such as the Courtoy Modul P press, which is part of the DLR system, using appropriately sized tooling. The weight of the tablets for a dose of 200 mg of Compound 1 Form I and 125 mg of substantially amorphous Compound 2 may be about 500 or 600 mg.

*Film Ceating*

Tablets may be film coated using the innovative Omega film coater, which is part of the DLR system. This coater enables fast film coating of sub-batches of 1 to 4 kg to allow continuous manufacturing.

*Printing*

Film coated tablets may be printed with a monogram on one or both tablet faces with, for example, an Ackley ramp printer.

**Tablet Formation from Twin Screw Wet Granulation Process**

Equipment/Process

*Equipment*

Twin Screw Wet Granulators: ConsiGma-1, ConsiGma-25 or Leistritz nano.

*Screening/Weighing*

Compound 1 Form I, the solid dispersion comprising substantially amorphous Compound 2, and excipients may be screened prior to or after weigh-out. Appropriate screen sizes are mesh 20, mesh 40, or mesh 60. Compound 1 Form I and/or the solid dispersion comprising substantially amorphous Compound 2 may be pre-blended with one or more of the excipients to simplify screening.

*Blending*

Compound 1 Form I, the solid dispersion comprising substantially amorphous Compound 2, and excipients may be added to the blender in different order. The blending may be performed in a Turbula blender, a v-shell blender, or a bin blender. The components may be blended for 10 minutes.

*Wet Granulation*

A granulation solution may be prepared by dissolving 48 g sodium lauryl sulfate and 159 g polyvinylpyrrolidone in 1,626 g water in a stainless steel container, using an overhead stirrer with a stirring speed of 700 RPM. The blend may be granulated using a twin screw granulator such as the ConsiGma-1. The granulation solution may be added to the twin screw granulator using a peristaltic pump, such as the pump on the ConsiGma-1, with a feed rate of 67 g/min. The blend may be added to the twin screw granulator using a Loss in Weight feeder, such as the Brabender feeder on the ConsiGma-1, with a feed rate of 10 kg/hr. The twin screw granulator may be operated with a barrel temperature of 25 degrees Celsius and a screw speed of 400 RPM. The granulation process may be performed for four minutes. The granulation process may be performed for a shorter or longer duration of time to produce a smaller or larger amount of

wet granules.

*Drying*

The wet granules may be fed directly into a fluid bed dryer, such as the drying chamber on the ConsiGma-1 or the segmented fluid bed dryer on the CTL-25. The drying end-point may be chosen at a product temperature of 43 degrees Celsius at which point the water content of the granules may be 1.6%w/w ("Loss on Drying, LOD"). The drying time may be 12 minutes, or shorter or longer, to reach the desired drying endpoint. The drying may be performed with an air flow of 59 $m^3$/min and inlet temperature of 60 degrees Celsius. Alternatively, the wet granules coming from the twin screw granulator may be collected into a bin or container for a certain period of time after which the wet granules are transferred to a separate stand-alone fluid bed dryer, such as the Vector Multi 15.

*Milling*

The dried granules may be milled to reduce the size of the granules. A cone mill such as the Quadro 194 CoMil may be used for this.

*Blending*

The granules may be blended with extra-granular excipients such as fillers and lubricant using a V-shell blender or a bin blender. The blending time may be 5, 3 or 1 minute(s).

*Compression*

The compression blend may be compressed into tablets using a single station or rotary tablet press, such as the Courtoy Modul P press, using 0.55' x 0.33' oval shaped tooling. The weight of the tablets for a dose of 200 mg of Compound 1 Form I and 125 mg of substantially amorphous Compound 2 may be about 500 or 600 mg.

*Film Coating*

Tablets may be film coated using a pan coater, such as, for example a Thomas Engineering Compu-Lab coater. A trace amount of Carnauba wax may be added to improve tablet appearance and process ability.

*Printing*

Film coated tablets may be printed with a monogram on one or both tablet faces with, for example, a Hartnett Delta printer.

**Tablet Formation from Continuous Twin Screw Wet Granulation Process**

Equipment/Process

*Equipment*

Granulator: ConsiGma or Leistritz or Thermo Fisher twin screw granulator.

*Screening/Weighing*

Compound 1 and excipients may be screened prior to or after weigh-out. Possible screen sizes are mesh 20, mesh 40, or mesh 60. Compound 1 may be pre-blended with one or more of the excipients to simplify screening.

*Blending*

Compound 1 and excipients may be added to the blender in different order. The blending may be performed in a Turbula blender, a v-shell blender, a bin blender, or a continuous blender. The components may be blended for 10 minutes for batch blenders or continuously for a continuous blender.

*Granulation Operation*

Granulation Fluid - SLS and binder are added to purified water and mixed until dissolved. A suitable ratio is 2.5% w/w SLS and 10.0% w/w PVP K30 in water.

Granulation - The blend containing Compound 1 and excipients may be dosed into the twin screw granulator using a Loss in Weight feeder at a rate of 10 kg/hr. The granulation fluid may be added using a peristaltic pump at a rate of 3.5 kg/hr. The granulator may be run at a speed of 400 RPM. A notable advantage of the present twin screw wet granulation process is using a granulation fluid that comprises both a surfactant and the binder for better granulation through increased wettability. In one embodiment, the surfactant is SLS. Another notable advantage is that because the process is continuous and at any moment in time only a limited amount of material is processed, the process can be well controlled and results in a high quality product.

*Milling*

The granules may be reduced in size using a screen mill or a cone mill, either before drying or after drying, or both.

*Drying*

The granules may be dried using a vacuum oven, tray dryer, bi-conical dryer, or fluid bed drier.

*Blending*

The granules may be blended with extra-granular excipients. The granules have been blended using a 300 liter bin blender for 60 revolutions.

*Compression*

The compression blend has been compressed into tablets using a Courtoy Modul P rotary press

*Film Coating*

Tablets may be film coated using a pan coater, such as, for example an O'Hara Labcoat.

*Printing*

Film coated tablets may be printed with a monogram on one or both tablet faces with, for example, a Hartnett Delta printer.

**ASSAYS**

**PROTOCOL 1**

**Assays for Detecting and Measuring ΔF508-CFTR Potentiation Properties of Compounds**

Membrane potential optical methods for assaying ΔF508-CFTR modulation properties of compounds

The assay utilizes fluorescent voltage sensing dyes to measure changes in membrane potential using a fluorescent plate reader (e.g., FLIPR III, Molecular Devices, Inc.) as a readout for increase in functional ΔF508-CFTR in NIH 3T3 cells. The driving force for the response is the creation of a chloride ion gradient in conjunction with channel activation by a single liquid addition step after the cells have previously been treated with compounds and subsequently loaded with a voltage sensing dye.

Identification of Potentiator Compounds

To identify potentiators of ΔF508-CFTR, a double-addition HTS assay format was developed. This HTS assay utilizes fluorescent voltage sensing dyes to measure changes in membrane potential on the FLIPR III as a measurement for increase in gating (conductance) of ΔF508 CFTR in temperature-corrected ΔF508 CFTR NIH 3T3 cells. The driving force for the response is a $Cl^-$ ion gradient in conjunction with channel activation with forskolin in a single liquid addition

step using a fluorescent plate reader such as FLIPR III after the cells have previously been treated with potentiator compounds (or DMSO vehicle control) and subsequently loaded with a redistribution dye.

*Solutions*

Bath Solution #1: (in mM) NaCl 160, KCl 4.5, $CaCl_2$ 2, $MgCl_2$ 1, HEPES 10, pH 7.4 with NaOH. Chloride-free bath solution: Chloride salts in Bath Solution #1 (above) are substituted with gluconate salts.

*Cell Culture*

NIH3T3 mouse fibroblasts stably expressing ΔF508-CFTR are used for optical measurements of membrane potential. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For all optical assays, the cells were seeded at ~20,000/well in 384-well matrigel-coated plates and cultured for 2 hrs at 37 °C before culturing at 27 °C for 24 hrs. for the potentiator assay. For the correction assays, the cells are cultured at 27 °C or 37 °C with and without compounds for 16 - 24 hours.

Electrophysiological Assays for assaying ΔF508-CFTR modulation properties of compounds.

*Using Chamber Assay*

Ussing chamber experiments were performed on polarized airway epithelial cells expressing ΔF508-CFTR to further characterize the ΔF508-CFTR augmenters or inducers identified in the optical assays. Non-CF and CF airway epithelia were isolated from bronchial tissue, cultured as previously described (Galietta, L.J.V., Lantero, S., Gazzolo, A., Sacco, O., Romano, L., Rossi, G.A., & Zegarra-Moran, O. (1998) In Vitro Cell. Dev. Biol. 34, 478-481), and plated onto Costar® Snapwell™ filters that were precoated with NIH3T3-conditioned media. After four days the apical media was removed and the cells were grown at an air liquid interface for >14 days prior to use. This resulted in a monolayer of fully differentiated columnar cells that were ciliated, features that are characteristic of airway epithelia. Non-CF HBE were isolated from non-smokers that did not have any known lung disease. CF-HBE were isolated from patients homozygous for ΔF508.

HBE grown on Costar® Snapwell™ cell culture inserts were mounted in an Using chamber (Physiologic Instruments, Inc., San Diego, CA), and the transepithelial resistance and short-circuit current in the presence of a basolateral to apical $Cl^-$ gradient ($I_{SC}$) were measured using a voltage-clamp system (Department of Bioengineering, University of Iowa, IA). Briefly, HBE were examined under voltage-clamp recording conditions ($V_{hold}$ = 0 mV) at 37 °C. The basolateral solution contained (in mM) 145 NaCl, 0.83 $K_2HPO_4$, 3.3 $KH_2PO_4$, 1.2 $MgCl_2$, 1.2 $CaCl_2$, 10 Glucose, 10 HEPES (pH adjusted to 7.35 with NaOH) and the apical solution contained (in mM) 145 NaGluconate, 1.2 $MgCl_2$, 1.2 $CaCl_2$, 10 glucose, 10 HEPES (pH adjusted to 7.35 with NaOH).

*Identification of Potentiator Compounds*

Typical protocol utilized a basolateral to apical membrane $Cl^-$ concentration gradient. To set up this gradient, normal ringers was used on the basolateral membrane, whereas apical NaCl was replaced by equimolar sodium gluconate (titrated to pH 7.4 with NaOH) to give a large $Cl^-$ concentration gradient across the epithelium. Forskolin (10 μM) and all test compounds were added to the apical side of the cell culture inserts. The efficacy of the putative ΔF508-CFTR potentiators was compared to that of the known potentiator, genistein.

*Patch-clamp Recordings*

Total $Cl^-$ current in ΔF508-NIH3T3 cells was monitored using the perforated-patch recording configuration as previously described (Rae, J., Cooper, K., Gates, P., & Watsky, M. (1991) J. Neurosci. Methods 37, 15-26). Voltage-clamp recordings were performed at 22 °C using an Axopatch 200B patch-clamp amplifier (Axon Instruments Inc., Foster City, CA). The pipette solution contained (in mM) 150 *N*-methyl-D-glucamine (NMDG)-Cl, 2 $MgCl_2$, 2 $CaCl_2$, 10 EGTA, 10 HEPES, and 240 μg/mL amphotericin-B (pH adjusted to 7.35 with HCl). The extracellular medium contained (in mM) 150 NMDG-Cl, 2 $MgCl_2$, 2 $CaCl_2$, 10 HEPES (pH adjusted to 7.35 with HCl). Pulse generation, data acquisition, and analysis were performed using a PC equipped with a Digidata 1320 A/D interface in conjunction with Clampex 8 (Axon Instruments Inc.). To activate ΔF508-CFTR, 10 μM forskolin and 20 μM genistein were added to the bath and the current-voltage relation was monitored every 30 sec.

*Identification of Potentiator Compounds*

The ability of ΔF508-CFTR potentiators to increase the macroscopic ΔF508-CFTR $Cl^-$ current ($I_{\Delta F508}$) in NIH3T3 cells stably expressing ΔF508-CFTR was also investigated using perforated-patch-recording techniques. The potentiators identified from the optical assays evoked a dose-dependent increase in $I\Delta_{F508}$ with similar potency and efficacy observed in the optical assays. In all cells examined, the reversal potential before and during potentiator application was around -30 mV, which is the calculated $E_{Cl}$ (-28 mV).

*Cell Culture*

NIH3T3 mouse fibroblasts stably expressing ΔF508-CFTR are used for whole-cell recordings. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For whole-cell recordings, 2,500 - 5,000 cells were seeded on poly-L-lysine-coated glass coverslips and cultured for 24 - 48 hrs at 27 °C before use to test the activity of potentiators; and incubated with or without the correction compound at 37 °C for measuring the activity of correctors.

*Single-channel recordings*

Gating activity of wt-CFTR and temperature-corrected ΔF508-CFTR expressed in NIH3T3 cells was observed using excised inside-out membrane patch recordings as previously described (Dalemans, W., Barbry, P., Champigny, G., Jallat, S., Dott, K., Dreyer, D., Crystal, R.G., Pavirani, A., Lecocq, J-P., Lazdunski, M. (1991) Nature 354, 526 - 528) using an Axopatch 200B patch-clamp amplifier (Axon Instruments Inc.). The pipette contained (in mM): 150 NMDG, 150 aspartic acid, 5 $CaCl_2$, 2 $MgCl_2$, and 10 HEPES (pH adjusted to 7.35 with Tris base). The bath contained (in mM): 150 NMDG-Cl, 2 $MgCl_2$, 5 EGTA, 10 TES, and 14 Tris base (pH adjusted to 7.35 with HCl). After excision, both wt- and ΔF508-CFTR were activated by adding 1 mM Mg-ATP, 75 nM of the catalytic subunit of cAMP-dependent protein kinase (PKA; Promega Corp. Madison, WI), and 10 mM NaF to inhibit protein phosphatases, which prevented current rundown. The pipette potential was maintained at 80 mV. Channel activity was analyzed from membrane patches containing ≤ 2 active channels. The maximum number of simultaneous openings determined the number of active channels during the course of an experiment. To determine the single-channel current amplitude, the data recorded from 120 sec of ΔF508-CFTR activity was filtered "off-line" at 100 Hz and then used to construct all-point amplitude histograms that were fitted with multigaussian functions using Bio-Patch Analysis software (Bio-Logic Comp. France). The total microscopic current and open probability ($P_o$) were determined from 120 sec of channel activity. The $P_o$ was determined using the Bio-Patch software or from the relationship $P_o$ = I/i(N), where I = mean current, i = single-channel current amplitude, and N = number of active channels in patch.

*Cell Culture*

NIH3T3 mouse fibroblasts stably expressing ΔF508-CFTR are used for excised-membrane patch-clamp recordings. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For single channel recordings, 2,500 - 5,000 cells were seeded on poly-L-lysine-coated glass coverslips and cultured for 24 - 48 hrs at 27 °C before use.

**PROTOCOL 2**

**Assays for Detecting and Measuring ΔF508-CFTR Correction Properties of Compounds**

Membrane potential optical methods for assaying ΔF508-CFTR modulation properties of compounds.

The optical membrane potential assay utilized voltage-sensitive FRET sensors described by Gonzalez and Tsien (*See* Gonzalez, J. E. and R. Y. Tsien (1995) "Voltage sensing by fluorescence resonance energy transfer in single cells" Biophys J 69(4): 1272-80, and Gonzalez, J. E. and R. Y. Tsien (1997) "Improved indicators of cell membrane potential that use fluorescence resonance energy transfer" Chem Biol 4(4): 269-77) in combination with instrumentation for measuring fluorescence changes such as the Voltage/Ion Probe Reader (VIPR) (See, Gonzalez, J. E., K. Oades, et al. (1999) "Cell-based assays and instrumentation for screening ion-channel targets" Drug Discov Today 4(9): 431-439).

These voltage sensitive assays are based on the change in fluorescence resonant energy transfer (FRET) between the membrane-soluble, voltage-sensitive dye, $DiSBAC_2(3)$, and a fluorescent phospholipid, CC2-DMPE, which is attached to the outer leaflet of the plasma membrane and acts as a FRET donor. Changes in membrane potential

($V_m$) cause the negatively charged $DiSBAC_2(3)$ to redistribute across the plasma membrane and the amount of energy transfer from CC2-DMPE changes accordingly. The changes in fluorescence emission were monitored using VIPR™ II, which is an integrated liquid handler and fluorescent detector designed to conduct cell-based screens in 96- or 384-well microtiter plates.

Identification of Correction Compounds

To identify small molecules that correct the trafficking defect associated with ΔF508-CFTR; a single-addition HTS assay format was developed. The cells were incubated in serum-free medium for 16 hrs at 37 °C in the presence or absence (negative control) of test compound. As a positive control, cells plated in 384-well plates were incubated for 16 hrs at 27 °C to "temperature-correct" ΔF508-CFTR. The cells were subsequently rinsed 3X with Krebs Ringers solution and loaded with the voltage-sensitive dyes. To activate ΔF508-CFTR, 10 μM forskolin and the CFTR potentiator, genistein (20 μM), were added along with $Cl^-$-free medium to each well. The addition of $Cl^-$-free medium promoted $Cl^-$ efflux in response to ΔF508-CFTR activation and the resulting membrane depolarization was optically monitored using the FRET-based voltage-sensor dyes.

Identification of Potentiator Compounds

To identify potentiators of ΔF508-CFTR, a double-addition HTS assay format was developed. During the first addition, a $Cl^-$-free medium with or without test compound was added to each well. After 22 sec, a second addition of $Cl^-$-free medium containing 2 - 10 μM forskolin was added to activate ΔF508-CFTR. The extracellular $Cl^-$ concentration following both additions was 28 mM, which promoted $Cl^-$ efflux in response to ΔF508-CFTR activation and the resulting membrane depolarization was optically monitored using the FRET-based voltage-sensor dyes.

Solutions

| | |
|---|---|
| Bath Solution **#1**: | (in mM) NaCl 160, KCl 4.5, $CaCl_2$ 2, $MgCl_2$ 1, HEPES 10, pH 7.4 with NaOH. |
| Chloride-free bath solution: | Chloride salts in Bath Solution #1 (above) are substituted with gluconate salts. |
| CC2-DMPE: | Prepared as a 10 mM stock solution in DMSO and stored at -20°C. |
| $DiSBAC_2(3)$: | Prepared as a 10 mM stock in DMSO and stored at -20°C. |

Cell Culture

NIH3T3 mouse fibroblasts stably expressing ΔF508-CFTR are used for optical measurements of membrane potential. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For all optical assays, the cells were seeded at 30,000/well in 384-well matrigel-coated plates and cultured for 2 hrs at 37 °C before culturing at 27 °C for 24 hrs for the potentiator assay. For the correction assays, the cells are cultured at 27 °C or 37 °C with and without compounds for 16 - 24 hours.

Electrophysiological Assays for assaying ΔF508-CFTR modulation properties of compounds

Using Chamber Assay

Using chamber experiments were performed on polarized epithelial cells expressing ΔF508-CFTR to further characterize the ΔF508-CFTR augmenters or inducers identified in the optical assays. $FRT^{\Delta F508\text{-}CFTR}$ epithelial cells grown on Costar Snapwell cell culture inserts were mounted in an Ussing chamber (Physiologic Instruments, Inc., San Diego, CA), and the monolayers were continuously short-circuited using a Voltage-clamp System (Department of Bioengineering, University of Iowa, IA, and, Physiologic Instruments, Inc., San Diego, CA). Transepithelial resistance was measured by applying a 2-mV pulse. Under these conditions, the FRT epithelia demonstrated resistances of 4 KΩ/ $cm^2$ or more. The solutions were maintained at 27 °C and bubbled with air. The electrode offset potential and fluid resistance were corrected using a cell-free insert. Under these conditions, the current reflects the flow of $Cl^-$ through ΔF508-CFTR expressed in the apical membrane. The $I_{SC}$ was digitally acquired using an MP100A-CE interface and AcqKnowledge software (*v*3.2.6; BIOPAC Systems, Santa Barbara, CA).

Identification of Correction Compounds

Typical protocol utilized a basolateral to apical membrane $Cl^-$ concentration gradient. To set up this gradient, normal ringer was used on the basolateral membrane, whereas apical NaCl was replaced by equimolar sodium gluconate (titrated to pH 7.4 with NaOH) to give a large $Cl^-$ concentration gradient across the epithelium. All experiments were performed with intact monolayers. To fully activate ΔF508-CFTR, forskolin (10 μM) and the PDE inhibitor, IBMX (100 μM), were applied followed by the addition of the CFTR potentiator, genistein (50 μM).

As observed in other cell types, incubation at low temperatures of FRT cells stably expressing ΔF508-CFTR increases the functional density of CFTR in the plasma membrane. To determine the activity of correction compounds, the cells were incubated with 10 μM of the test compound for 24 hours at 37°C and were subsequently washed 3X prior to recording. The cAMP- and genistein-mediated $I_{SC}$ in compound-treated cells was normalized to the 27°C and 37°C controls and expressed as percentage activity. Preincubation of the cells with the correction compound significantly increased the cAMP- and genistein-mediated $I_{SC}$ compared to the 37°C controls.

Identification of Potentiator Compounds

Typical protocol utilized a basolateral to apical membrane $Cl^-$ concentration gradient. To set up this gradient, normal ringers was used on the basolateral membrane and was permeabilized with nystatin (360 μg/ml), whereas apical NaCl was replaced by equimolar sodium gluconate (titrated to pH 7.4 with NaOH) to give a large $Cl^-$ concentration gradient across the epithelium. All experiments were performed 30 min after nystatin permeabilization. Forskolin (10 μM) and all test compounds were added to both sides of the cell culture inserts. The efficacy of the putative ΔF508-CFTR potentiators was compared to that of the known potentiator, genistein.

Solutions

| | |
|---|---|
| Basolateral solution (in mM): | NaCl (135), $CaCl_2$ (1.2), $MgCl_2$ (1.2), $K_2HPO_4$ (2.4), $KHPO_4$ (0.6), N-2-hydroxyethylpiperazine-N'-2-ethanesulfonic acid (HEPES) (10), and dextrose (10). The solution was titrated to pH 7.4 with NaOH. |
| Apical solution (in mM): | Same as basolateral solution with NaCl replaced with Na Gluconate (135). |

Cell Culture

Fisher rat epithelial (FRT) cells expressing ΔF508-CFTR ($FRT^{\Delta F508\text{-}CFTR}$) were used for Ussing chamber experiments for the putative ΔF508-CFTR augmenters or inducers identified from our optical assays. The cells were cultured on Costar Snapwell cell culture inserts and cultured for five days at 37 °C and 5% $CO_2$ in Coon's modified Ham's F-12 medium supplemented with 5% fetal calf serum, 100 U/ml penicillin, and 100 μg/ml streptomycin. Prior to use for characterizing the potentiator activity of compounds, the cells were incubated at 27 °C for 16 - 48 hrs to correct for the ΔF508-CFTR. To determine the activity of corrections compounds, the cells were incubated at 27 °C or 37 °C with and without the compounds for 24 hours.

Whole-cell recordings

The macroscopic ΔF508-CFTR current ($I_{\Delta F508}$) in temperature- and test compound-corrected NIH3T3 cells stably expressing ΔF508-CFTR were monitored using the perforated-patch, whole-cell recording. Briefly, voltage-clamp recordings of $I_{\Delta F508}$ were performed at room temperature using an Axopatch 200B patch-clamp amplifier (Axon Instruments Inc., Foster City, CA). All recordings were acquired at a sampling frequency of 10 kHz and low-pass filtered at 1 kHz. Pipettes had a resistance of 5 - 6 MΩ when filled with the intracellular solution. Under these recording conditions, the calculated reversal potential for $Cl^-$ ($E_{Cl}$) at room temperature was -28 mV. All recordings had a seal resistance > 20 GΩ and a series resistance < 15 MΩ. Pulse generation, data acquisition, and analysis were performed using a PC equipped with a Digidata 1320 A/D interface in conjunction with Clampex 8 (Axon Instruments Inc.). The bath contained < 250 μl of saline and was continuously perifused at a rate of 2 ml/min using a gravity-driven perfusion system.

Identification of Correction Compounds

To determine the activity of correction compounds for increasing the density of functional ΔF508-CFTR in the plasma membrane, we used the above-described perforated-patch-recording techniques to measure the current density following 24-hr treatment with the correction compounds. To fully activate ΔF508-CFTR, 10 μM forskolin and 20 μM genistein were added to the cells. Under our recording conditions, the current density following 24-hr incubation at 27°C was higher than that observed following 24-hr incubation at 37 °C. These results are consistent with the known effects of low-temperature incubation on the density of ΔF508-CFTR in the plasma membrane. To determine the effects of correction compounds on CFTR current density, the cells were incubated with 10 μM of the test compound for 24 hours at 37°C and the current density was compared to the 27°C and 37°C controls (% activity). Prior to recording, the cells were washed 3X with extracellular recording medium to remove any remaining test compound. Preincubation with 10 μM of correction compounds significantly increased the cAMP- and genistein-dependent current compared to the 37°C controls.

Identification of Potentiator Compounds

The ability of ΔF508-CFTR potentiators to increase the macroscopic ΔF508-CFTR $Cl^-$ current ($I_{\Delta F508}$) in NIH3T3 cells stably expressing ΔF508-CFTR was also investigated using perforated-patch-recording techniques. The potentiators identified from the optical assays evoked a dose-dependent increase in $I_{\Delta F508}$ with similar potency and efficacy observed in the optical assays. In all cells examined, the reversal potential before and during potentiator application was around -30 mV, which is the calculated $E_{Cl}$ (-28 mV).

Solutions

| | |
|---|---|
| Intracellular solution (in mM): | Cs-aspartate (90), CsCl (50), $MgCl_2$ (1), HEPES (10), and 240 μg/ml amphotericin-B (pH adjusted to 7.35 with CsOH). |
| Extracellular solution (in mM): | N-methyl-D-glucamine (NMDG)-Cl (150), $MgCl_2$ (2), $CaCl_2$ (2), HEPES (10) (pH adjusted to 7.35 with HCl). |

Cell Culture

NIH3T3 mouse fibroblasts stably expressing ΔF508-CFTR are used for whole-cell recordings. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For whole-cell recordings, 2,500 - 5,000 cells were seeded on poly-L-lysine-coated glass coverslips and cultured for 24 - 48 hrs at 27 °C before use to test the activity of potentiators; and incubated with or without the correction compound at 37 °C for measuring the activity of correctors.

Single-channel recordings

The single-channel activities of temperature-corrected ΔF508-CFTR stably expressed in NIH3T3 cells and activities of potentiator compounds were observed using excised inside-out membrane patch. Briefly, voltage-clamp recordings of single-channel activity were performed at room temperature with an Axopatch 200B patch-clamp amplifier (Axon Instruments Inc.). All recordings were acquired at a sampling frequency of 10 kHz and low-pass filtered at 400 Hz. Patch pipettes were fabricated from Corning Kovar Sealing #7052 glass (World Precision Instruments, Inc., Sarasota, FL) and had a resistance of 5 - 8 MΩ when filled with the extracellular solution. The ΔF508-CFTR was activated after excision, by adding 1 mM Mg-ATP, and 75 nM of the cAMP-dependent protein kinase, catalytic subunit (PKA; Promega Corp. Madison, WI). After channel activity stabilized, the patch was perifused using a gravity-driven microperfusion system. The inflow was placed adjacent to the patch, resulting in complete solution exchange within 1 - 2 sec. To maintain ΔF508-CFTR activity during the rapid perifusion, the nonspecific phosphatase inhibitor $F^-$ (10 mM NaF) was added to the bath solution. Under these recording conditions, channel activity remained constant throughout the duration of the patch recording (up to 60 min). Currents produced by positive charge moving from the intra-to extracellular solutions (anions moving in the opposite direction) are shown as positive currents. The pipette potential ($V_p$) was maintained at 80 mV.

Channel activity was analyzed from membrane patches containing ≤ 2 active channels. The maximum number

of simultaneous openings determined the number of active channels during the course of an experiment. To determine the single-channel current amplitude, the data recorded from 120 sec of ΔF508-CFTR activity was filtered "off-line" at 100 Hz and then used to construct all-point amplitude histograms that were fitted with multigaussian functions using Bio-Patch Analysis software (Bio-Logic Comp. France). The total microscopic current and open probability ($P_o$) were determined from 120 sec of channel activity. The $P_o$ was determined using the Bio-Patch software or from the relationship $P_o$ = I/i(N), where I = mean current, i = single-channel current amplitude, and N = number of active channels in patch.

Solutions

| | |
|---|---|
| Extracellular solution (in mM): | NMDG (150), aspartic acid (150), $CaCl_2$ (5), $MgCl_2$ (2), and HEPES (10) (pH adjusted to 7.35 with Tris base). |
| Intracellular solution (in mM): | NMDG-Cl (150), $MgCl_2$ (2), EGTA (5), TES (10), and Tris base (14) (pH adjusted to 7.35 with HCl). |

Cell Culture

NIH3T3 mouse fibroblasts stably expressing ΔF508-CFTR are used for excised-membrane patch-clamp recordings. The cells are maintained at 37 °C in 5% $CO_2$ and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 $cm^2$ culture flasks. For single channel recordings, 2,500 - 5,000 cells were seeded on poly-L-lysine-coated glass coverslips and cultured for 24 - 48 hrs at 27 °C before use.

Compound 1 and Compound 2 of the invention are useful as augmenters or inducers of CFTR activity. Table 5 below illustrates the EC50 and relative efficacy of Compound 1 and Compound 2. In Table 5 below, the following meanings apply. EC50: "+++" means <10 uM; "++" means between 10uM to 25 uM; "+" means between 25 uM to 60uM. % Efficacy: "+" means < 25%; "++" means between 25% to 100%; "+++" means > 100%..

Table 5.

| Cmpd. No. | EC50 (μM) | % Activity |
|---|---|---|
| 1 | +++ | +++ |
| 2 | +++ | ++ |

## OTHER EMBODIMENTS

The foregoing discussion discloses and describes merely exemplary embodiments of the invention, which is defined in the appended claims.

## Claims

**1.** A solid oral pharmaceutical composition comprising:

200 mg of 3-(6-(1-(2,2-difluorobenzo[d][1,3]dioxol-5-yl) cyclopropanecarboxamido)-3-methylpyridin-2-yl)benzoic acid (Compound 1) Form I and
a solid dispersion comprising substantially amorphous N-(5-hydroxy-2,4-ditert-butylphenyl)-4-oxo-1H-quinoline-3-carboxamide (Compound 2) and a polymer,
wherein the substantially amorphous Compound 2 is present in the solid oral pharmaceutical composition in an amount of 125 mg,
wherein Compound 1 Form I is **characterized by** one or more peaks at 15.4, 16.3, and 14.5 degrees in an X-ray powder diffraction pattern,
wherein substantially amorphous Compound 2 has less than 15% crystallinity, and
wherein the solid oral pharmaceutical composition is a tablet comprising 25 to 50 percent by weight Compound 1 Form I, and 15 to 35 percent weight of a solid dispersion comprising Compound 2.

**2.** The solid oral pharmaceutical composition of claim 1 further comprising:

a. a filler;
b. a disintegrant;
c. a surfactant; and
d. a binder;

referred to as PC-I.

**3.** The solid oral pharmaceutical composition of claim 2, further comprising:
e. a lubricant
referred to as **PC-III.**

**4.** The solid oral pharmaceutical composition of claim 1, wherein the solid oral pharmaceutical composition further comprises:

a filler which is microcystalline cellulose in an amount from 20 to 30 percent by weight,
a disintegrant which is croscarmelose sodium in an amount from 3 to 10 percent by weight,
a surfactant which is sodium lauryl sulfate in an amount from 0.5 to 2 percent by weight,
a binder which is polyvinylpyrrolidone in an amount from 0 to 5 percent by weight, and
a lubricant which is magnesium stearate, in an amount from 0.5 to 2 percent by weight.

**5.** The solid oral pharmaceutical composition of claim 1, comprising 30 to 50 percent by weight Compound 1 Form I.

**6.** The solid oral pharmaceutical composition of any of claims 1-4 having the following formulation:

| | % by wgt. |
|---|---|
| Compound 1 Form I | 25-50 |
| A solid dispersion comprising substantially amorphous Compound 2 | 15-35 |
| Microcrystalline cellulose | 20-30 |
| Croscarmellose sodium | 3-10 |
| Sodium lauryl sulfate | 0.5-2 |
| Polyvinylpyrrolidone | 0-5 |
| Magnesium stearate | 0.5-2 |

referred to as **PC-IV**.

**7.** The solid oral pharmaceutical composition of any of claims 1-6, further comprising a colorant and a wax.

**8.** The tablet of any of claims 1-7 having the following formulation:

| | % by wgt. |
|---|---|
| Compound 1 Form I | 35 |
| Solid dispersion comprising substantially amorphous Compound 2 | 28 |
| Microcrystalline cellulose | 26 |
| Croscarmellose sodium | 6 |
| Sodium lauryl sulfate | 1 |
| Polyvinylpyrrolidone | 3 |
| Magnesium stearate | 1 |

referred to as **PC-VIII**, or:

| | % by wgt. |
|---|---|
| Compound 1 Form I | 31 |
| Solid dispersion comprising substantially amorphous Compound 2 | 32 |
| Microcrystalline cellulose | 26 |
| Croscarmellose sodium | 6 |
| Sodium lauryl sulfate | 1 |
| Polyvinylpyrrolidone | 3 |
| Magnesium stearate | 1 |

referred to as **PC-IX,**
or:

| | mg |
|---|---|
| Compound 1 Form I | 200 |
| Solid dispersion comprising substantially amorphous Compound 2 | 156 |
| Microcrystalline cellulose | 150 |
| Croscarmellose sodium | 34 |
| Sodium lauryl sulfate | 4 |
| Polyvinylpyrrolidone | 15 |
| Magnesium stearate | 6 |

referred to as **PC-XI,** or:

| | % by wgt. |
|---|---|
| Compound 1 Form I | 34 |
| Solid dispersion comprising substantially amorphous Compound 2 | 27 |
| Microcrystalline cellulose | 25 |
| Croscarmellose sodium | 6 |
| Sodium lauryl sulfate | 1 |
| Polyvinylpyrrolidone | 3 |
| Magnesium stearate | 1 |
| Colorant | 3 |

referred to as **PC-XIV,** or:

| | % by wgt. |
|---|---|
| Compound 1 Form I | 30 |
| Solid dispersion comprising substantially amorphous Compound 2 | 31 |
| Microcrystalline cellulose | 25 |
| Croscarmellose sodium | 6 |
| Sodium lauryl sulfate | 1 |

(continued)

| | % by wgt. |
|---|---|
| Polyvinylpyrrolidone | 3 |
| Magnesium stearate | 1 |
| Colorant | 3 |

referred to as **PC-XV,** or:

| | mg |
|---|---|
| Compound 1 Form I | 200 |
| Solid dispersion comprising substantially amorphous Compound 2 | 156 |
| Microcrystalline cellulose | 150 |
| Croscarmellose sodium | 34 |
| Sodium lauryl sulfate | 4 |
| Polyvinylpyrrolidone | 15 |
| Magnesium stearate | 6 |
| Colorant | 17 |

referred to as **PC-XVII.**

**9.** A solid oral pharmaceutical composition comprising:

100 mg of 3-(6-(1-(2,2-difluorobenzo[d][1,3]dioxol-5-yl) cyclopropanecarboxamido)-3-methylpyridin-2-yl)benzoic acid (Compound 1) Form I and
a solid dispersion comprising substantially amorphous N-(5-hydroxy-2,4-ditert-butylphenyl)-4-oxo-1H-quinoline-3-carboxamide (Compound 2) and a polymer,
wherein the substantially amorphous Compound 2 is present in the solid oral pharmaceutical composition in an amount of 125 mg,
wherein Compound 1 Form I is **characterized by** one or more peaks at 15.4, 16.3, and 14.5 degrees in an X-ray powder diffraction pattern,
wherein substantially amorphous Compound 2 has less than 15% crystallinity,
and wherein the solid oral pharmaceutical composition is a tablet having the following formulation:

| Component | mg/Tablet |
|---|---|
| Compound 1 Form I | 100 |
| Solid dispersion comprising substantially amorphous Compound 2 | 156 |
| Microcrystalline cellulose | 55 |
| Croscarmellose sodium | 7 |
| Polyvinylpyrrolidone | 11 |
| Sodium lauryl sulfate | 3 |
| Total Granules | 332 |
| Croscarmellose sodium | 18 |
| Microcrystalline cellulose | 53 |
| Magnesium stearate | 4 |
| Total Tablet | 407 |

referred to as **PC-XXIII.**

**10.** The tablet of any of claims 1-7 having the following formulation:

| | mg |
|---|---|
| Compound 1 Form I | 200 |
| Substantially amorphous Compound 2 | 125 |
| Microcrystalline cellulose | 150 |
| Croscarmellose sodium | 34 |
| Sodium lauryl sulfate | 4 |
| Polyvinylpyrrolidone | 15 |
| Magnesium stearate | 6 |
| Colorant | 17 |

referred to as **PC-XVIII.**

**11.** A method of preparing a tablet according to any one of claims 1-9, wherein the method comprises:

a) wet granulating the following components to produce a granule:

a. Compound 1 Form I;
b. a solid dispersion comprising substantially amorphous Compound 2;
c. a filler;
d. a disintegrant;
e. a surfactant; and
f. a binder;

and
b) compressing the following components to produce a tablet:

i. a plurality of granules from step a);
ii) a disintegrant;
iii) a filler; and
iv) a lubricant,

wherein Compound 1 Form I is **characterized by** one or more peaks at 15.4, 16.3, and 14.5 degrees in an X-ray powder diffraction pattern, and
wherein substantially amorphous Compound 2 has less than 15% crystallinity.

**12.** A kit comprising the solid oral pharmaceutical composition of claim 1 and a separate therapeutic agent.

**13.** The kit of claim 12, wherein the solid oral pharmaceutical composition and the therapeutic agent are in separate containers.

**14.** The kit of claim 13, wherein the containers are bottles, vials, or blister packs, or combination thereof.

**Patentansprüche**

**1.** Feste orale pharmazeutische Zusammensetzung, umfassend:

200 mg 3-(6-(1-(2,2-Difluorbenzo[d] [1,3]dioxol-5-yl)cyclopropancarboxamido)-3-methylpyridin-2-yl)benzoesäure (Verbindung 1) Form I und

eine feste Dispersion, umfassend im Wesentlichen amorphes N-(5-Hydroxy-2,4-di-tert.-butylphenyl)-4-oxo-1H-chinolin-3-carbonsäureamid (Verbindung 2) und ein Polymer,
wobei die im Wesentlichen amorphe Verbindung 2 in der festen oralen pharmazeutischen Zusammensetzung in einer Menge von 125 mg vorliegt,
wobei Verbindung 1 Form I durch einen oder mehrere Peaks bei 15,4, 16,3 und 14,5 Grad in einem Röntgenpulverbeugungsmuster gekennzeichnet ist,
wobei im Wesentlichen amorphe Verbindung 2 eine Kristallinität von weniger als 15% aufweist und
wobei es sich bei der festen oralen pharmazeutischen Zusammensetzung um eine Tablette handelt, die 25 bis 50 Gewichtsprozent Verbindung 1 Form I und 15 bis 35 Gewichtsprozent einer festen Dispersion, die Verbindung 2 umfasst, umfasst.

**2.** Feste orale pharmazeutische Zusammensetzung nach Anspruch 1, weiterhin umfassend:

a. einen Füllstoff,
b. ein Sprengmittel,
c. ein Tensid und
d. ein Bindemittel,

bezeichnet als **PC-I.**

**3.** Feste orale pharmazeutische Zusammensetzung nach Anspruch 2, weiterhin umfassend:
e. ein Schmiermittel,
bezeichnet als **PC-III.**

**4.** Feste orale pharmazeutische Zusammensetzung nach Anspruch 1, wobei die feste orale pharmazeutische Zusammensetzung weiterhin Folgendes umfasst:

einen Füllstoff, bei dem es sich um mikrokristalline Cellulose handelt, in einer Menge von 20 bis 30 Gewichtsprozent,
ein Sprengmittel, bei dem es sich um Croscarmelose-Natrium handelt, in einer Menge von 3 bis 10 Gewichtsprozent,
ein Tensid, bei dem es sich um Natriumlaurylsulfat handelt, in einer Menge von 0,5 bis 2 Gewichtsprozent,
ein Bindemittel, bei dem es sich um Polyvinylpyrrolidon handelt, in einer Menge von 0 bis 5 Gewichtsprozent und
ein Schmiermittel, bei dem es sich um Magnesiumstearat handelt, in einer Menge von 0,5 bis 2 Gewichtsprozent.

**5.** Feste orale pharmazeutische Zusammensetzung nach Anspruch 1, umfassend 30 bis 50 Gewichtsprozent Verbindung 1 Form I.

**6.** Feste orale pharmazeutische Zusammensetzung nach einem der Ansprüche 1-4 mit der folgenden Formulierung:

| | Gew.-% |
|---|---|
| Verbindung 1 Form I | 25-50 |
| Feste Dispersion, umfassend im Wesentlichen amorphe Verbindung 2 | 15-35 |
| Mikrokristalline Cellulose | 20-30 |
| Croscarmellose-Natrium | 3-10 |
| Natriumlaurylsulfat | 0, 5-2 |
| Polyvinylpyrrolidon | 0-5 |
| Magnesiumstearat | 0, 5-2 |

bezeichnet als **PC-IV.**

**7.** Feste orale pharmazeutische Zusammensetzung nach einem der Ansprüche 1-6, welche weiterhin einen Farbstoff und ein Wachs umfasst.

**8.** Tablette nach einem der Ansprüche 1-7 mit der folgenden Formulierung:

| | Gew.-% |
|---|---|
| Verbindung 1 Form I | 35 |
| Feste Dispersion, umfassend im Wesentlichen amorphe Verbindung 2 | 28 |
| Mikrokristalline Cellulose | 26 |
| Croscarmellose-Natrium | 6 |
| Natriumlaurylsulfat | 1 |
| Polyvinylpyrrolidon | 3 |
| Magnesiumstearat | 1 |

bezeichnet als **PC-VIII,** oder:

| | Gew.-% |
|---|---|
| Verbindung 1 Form I | 31 |
| Feste Dispersion, umfassend im Wesentlichen amorphe Verbindung 2 | 32 |
| Mikrokristalline Cellulose | 26 |
| Croscarmellose-Natrium | 6 |
| Natriumlaurylsulfat | 1 |
| Polyvinylpyrrolidon | 3 |
| Magnesiumstearat | 1 |

bezeichnet als **PC-IX,** oder:

| | mg |
|---|---|
| Verbindung 1 Form I | 200 |
| Feste Dispersion, umfassend im Wesentlichen amorphe Verbindung 2 | 156 |
| Mikrokristalline Cellulose | 150 |
| Croscarmellose-Natrium | 34 |
| Natriumlaurylsulfat | 4 |
| Polyvinylpyrrolidon | 15 |
| Magnesiumstearat | 6 |

bezeichnet als **PC-XI,** oder:

| | Gew.-% |
|---|---|
| Verbindung 1 Form I | 34 |
| Feste Dispersion, umfassend im Wesentlichen amorphe Verbindung 2 | 27 |
| Mikrokristalline Cellulose | 25 |
| Croscarmellose-Natrium | 6 |
| Natriumlaurylsulfat | 1 |
| Polyvinylpyrrolidon | 3 |

(fortgesetzt)

| | Gew.-% |
|---|---|
| Magnesiumstearat | 1 |
| Farbstoff | 3 |

bezeichnet als **PC-XIV,** oder:

| | Gew.-% |
|---|---|
| Verbindung 1 Form I | 30 |
| Feste Dispersion, umfassend im Wesentlichen amorphe Verbindung 2 | 31 |
| Mikrokristalline Cellulose | 25 |
| Croscarmellose-Natrium | 6 |
| Natriumlaurylsulfat | 1 |
| Polyvinylpyrrolidon | 3 |
| Magnesiumstearat | 1 |
| Farbstoff | 3 |

bezeichnet als **PC-XV,** oder:

| | mg |
|---|---|
| Verbindung 1 Form I | 200 |
| Feste Dispersion, umfassend im Wesentlichen amorphe Verbindung 2 | 156 |
| Mikrokristalline Cellulose | 150 |
| Croscarmellose-Natrium | 34 |
| Natriumlaurylsulfat | 4 |
| Polyvinylpyrrolidon | 15 |
| Magnesiumstearat | 6 |
| Farbstoff | 17 |

bezeichnet als **PC-XVII.**

**9.** Feste orale pharmazeutische Zusammensetzung, umfassend:

100 mg 3-(6-(1-(2,2-Difluorbenzo[d] [1,3]dioxol-5-yl)cyclopropancarboxamido)-3-methylpyridin-2-yl)benzoesäure (Verbindung 1) Form I und
eine feste Dispersion, umfassend im Wesentlichen amorphes N-(5-Hydroxy-2,4-di-tert.-butylphenyl)-4-oxo-1H-chinolin-3-carbonsäureamid (Verbindung 2) und ein Polymer,
wobei die im Wesentlichen amorphe Verbindung 2 in der festen oralen pharmazeutischen Zusammensetzung in einer Menge von 125 mg vorliegt,
wobei Verbindung 1 Form I durch einen oder mehrere Peaks bei 15,4, 16,3 und 14,5 Grad in einem Röntgenpulverbeugungsmuster gekennzeichnet ist,
wobei im Wesentlichen amorphe Verbindung 2 eine Kristallinität von weniger als 15% aufweist und
wobei es sich bei der festen oralen pharmazeutischen Zusammensetzung um eine Tablette mit der folgenden Formulierung handelt:

| Komponente | mg/Tablette |
|---|---|
| Verbindung 1 Form I | 100 |
| Feste Dispersion, umfassend im Wesentlichen amorphe Verbindung 2 | 156 |
| Mikrokristalline Cellulose | 55 |
| Croscarmellose-Natrium | 7 |
| Polyvinylpyrrolidon | 11 |
| Natriumlaurylsulfat | 3 |
| Granulat gesamt | 332 |
| Croscarmellose-Natrium | 18 |
| Mikrokristalline Cellulose | 53 |
| Magnesiumstearat | 4 |
| Tablette gesamt | 407 |

bezeichnet als **PC-XXIII.**

**10.** Tablette nach einem der Ansprüche 1-7 mit der folgenden Formulierung:

| | mg |
|---|---|
| Verbindung 1 Form I | 200 |
| Im Wesentlichen amorphe Verbindung 2 | 125 |
| Mikrokristalline Cellulose | 150 |
| Croscarmellose-Natrium | 34 |
| Natriumlaurylsulfat | 4 |
| Polyvinylpyrrolidon | 15 |
| Magnesiumstearat | 6 |
| Farbstoff | 17 |

,
bezeichnet als **PC-XVIII.**

**11.** Verfahren zur Herstellung einer Tablette nach einem der Ansprüche 1-9, wobei das Verfahren Folgendes umfasst:

a) Feuchtgranulation der folgenden Komponenten unter Herstellung eines Granulats:

a. Verbindung 1 Form I,
b. eine feste Dispersion, welche im Wesentlichen amorphe Verbindung 2 umfasst,
c. einen Füllstoff,
d. ein Sprengmittel,
e. ein Tensid und
f. ein Bindemittel

und
b) Komprimieren der folgenden Komponenten unter Herstellung einer Tablette:

i. Granulat aus Schritt a),
ii) ein Sprengmittel,

iii) einen Füllstoff und
iv) ein Schmiermittel,

wobei Verbindung 1 Form I durch einen oder mehrere Peaks bei 15,4, 16,3 und 14,5 Grad in einem Röntgenpulverbeugungsmuster gekennzeichnet ist, und
wobei im Wesentlichen amorphe Verbindung 2 eine Kristallinität von weniger als 15% aufweist.

**12.** Kit, umfassend die feste orale pharmazeutische Zusammensetzung nach Anspruch 1 und ein separates Therapeutikum.

**13.** Kit nach Anspruch 12, wobei sich die feste orale pharmazeutische Zusammensetzung und das Therapeutikum in getrennten Behältnissen befinden.

**14.** Kit nach Anspruch 13, wobei es sich bei den Behältnissen um Flaschen, Ampullen oder Durchdrückpackungen oder eine Kombination davon handelt.

**Revendications**

**1.** Composition pharmaceutique orale solide comprenant :

200 mg de forme I de l'acide 3-(6-(1-(2,2-difluorobenzo[*d*][1,3]dioxol-5-yl)cyclopropanecarboxamido)-3-méthylpyridin-2-yl)benzoïque (composé 1) et
une dispersion solide comprenant du *N*-(5-hydroxy-2,4-di-*tert*-butylphényl)-4-oxo-1*H*-quinoléine-3-carboxamide (composé 2) pratiquement amorphe et un polymère,
le composé 2 pratiquement amorphe étant présent dans la composition pharmaceutique orale solide en une quantité de 125 mg,
la forme I du composé 1 étant **caractérisée par** un ou plusieurs pics à 15,4, 16,3 et 14,5 degrés dans un diagramme de diffraction des rayons X sur poudre,
le composé 2 pratiquement amorphe ayant une cristallinité inférieure à 15 %, et
la composition pharmaceutique orale solide étant un comprimé comprenant 25 à 50 pour cent en poids de forme I du composé 1, et 15 à 35 pour cent en poids d'une dispersion solide comprenant le composé 2.

**2.** Composition pharmaceutique orale solide selon la revendication 1 comprenant en outre :

a. une charge ;
b. un délitant ;
c. un tensioactif ; et
d. un liant ;

appelée PC-I.

**3.** Composition pharmaceutique orale solide selon la revendication 2, comprenant en outre :
e. un lubrifiant
appelée PC-III.

**4.** Composition pharmaceutique orale solide selon la revendication 1, la composition pharmaceutique orale solide comprenant en outre :

une charge qui est de la cellulose microcristalline en une quantité de 20 à 30 pour cent en poids,
un délitant qui est de la croscarmellose sodique en une quantité de 3 à 10 pour cent en poids,
un tensioactif qui est du laurylsulfate de sodium en une quantité de 0,5 à 2 pour cent en poids,
un liant qui est de la polyvinylpyrrolidone en une quantité de 0 à 5 pour cent en poids, et
un lubrifiant qui est du stéarate de magnésium, en une quantité de 0,5 à 2 pour cent en poids.

**5.** Composition pharmaceutique orale solide selon la revendication 1, comprenant 30 à 50 pour cent en poids de forme I du composé 1.

**6.** Composition pharmaceutique orale solide selon l'une quelconque des revendications 1-4 ayant la formulation suivante :

| | % en poids |
|---|---|
| Forme I du composé 1 | 25-50 |
| Une dispersion solide comprenant du composé 2 pratiquement amorphe | 15-35 |
| Cellulose microcristalline | 20-30 |
| Croscarmellose sodique | 3-10 |
| Laurylsulfate de sodium | 0, 5-2 |
| Polyvinylpyrrolidone | 0-5 |
| Stéarate de magnésium | 0, 5-2 |

appelée PC-IV.

**7.** Composition pharmaceutique orale solide selon l'une quelconque des revendications 1-6, comprenant en outre un colorant et une cire.

**8.** Comprimé selon l'une quelconque des revendications 1 à 7 ayant la formulation suivante :

| | % en poids |
|---|---|
| Forme I du composé 1 | 35 |
| Dispersion solide comprenant du composé 2 pratiquement amorphe | 28 |
| Cellulose microcristalline | 26 |
| Croscarmellose sodique | 6 |
| Laurylsulfate de sodium | 1 |
| Polyvinylpyrrolidone | 3 |
| Stéarate de magnésium | 1 |

appelée PC-VIII, ou :

| | % en poids |
|---|---|
| Forme I du composé 1 | 31 |
| Dispersion solide comprenant du composé 2 pratiquement amorphe | 32 |
| Cellulose microcristalline | 26 |
| Croscarmellose sodique | 6 |
| Laurylsulfate de sodium | 1 |
| Polyvinylpyrrolidone | 3 |
| Stéarate de magnésium | 1 |

Appelée PC-IX,
ou :

| | mg |
|---|---|
| Forme I du composé 1 | 200 |
| Dispersion solide comprenant du composé 2 pratiquement amorphe | 156 |

(suite)

| | mg |
|---|---|
| Cellulose microcristalline | 150 |
| Croscarmellose sodique | 34 |
| Laurylsulfate de sodium | 4 |
| Polyvinylpyrrolidone | 15 |
| Stéarate de magnésium | 6 |

appelée PC-XI, ou :

| | % en poids |
|---|---|
| Forme I du composé 1 | 34 |
| Dispersion solide comprenant du composé 2 pratiquement amorphe | 27 |
| Cellulose microcristalline | 25 |
| Croscarmellose sodique | 6 |
| Laurylsulfate de sodium | 1 |
| Polyvinylpyrrolidone | 3 |
| Stéarate de magnésium | 1 |
| Colorant | 3 |

appelée PC-XIV, ou :

| | % en poids |
|---|---|
| Forme I du composé 1 | 30 |
| Dispersion solide comprenant du composé 2 pratiquement amorphe | 31 |
| Cellulose microcristalline | 25 |
| Croscarmellose sodique | 6 |
| Laurylsulfate de sodium | 1 |
| Polyvinylpyrrolidone | 3 |
| Stéarate de magnésium | 1 |
| Colorant | 3 |

appelée PC-XV, ou :

| | mg |
|---|---|
| Forme I du composé 1 | 200 |
| Dispersion solide comprenant du composé 2 pratiquement amorphe | 156 |
| Cellulose microcristalline | 150 |
| Croscarmellose sodique | 34 |
| Laurylsulfate de sodium | 4 |
| Polyvinylpyrrolidone | 15 |
| Stéarate de magnésium | 6 |

(suite)

| | mg |
|---|---|
| Colorant | 17 |

appelée PC-XVII.

**9.** Composition pharmaceutique orale solide comprenant :

100 mg de forme I de l'acide 3-(6-(1-(2,2-difluorobenzo[*d*][1,3]dioxol-5-yl)cyclopropanecarboxamido)-3-méthylpyridin-2-yl)benzoïque (composé 1) et
une dispersion solide comprenant du *N*-(5-hydroxy-2,4-di-*tert*-butylphényl)-4-oxo-1*H*-quinoléine-3-carboxamide (composé 2) pratiquement amorphe et un polymère,
le composé 2 pratiquement amorphe étant présent dans la composition pharmaceutique orale solide en une quantité de 125 mg,
la forme I du composé 1 étant **caractérisée par** un ou plusieurs pics à 15,4, 16,3 et 14,5 degrés dans un diagramme de diffraction des rayons X sur poudre,
le composé 2 pratiquement amorphe ayant une cristallinité inférieure à 15 %,
et la composition pharmaceutique orale solide étant un comprimé ayant la formulation suivante :

| Composant | mg/comprimé |
|---|---|
| Forme I du composé 1 | 100 |
| Dispersion solide comprenant du composé 2 pratiquement amorphe | 156 |
| Cellulose microcristalline | 55 |
| Croscarmellose sodique | 7 |
| Polyvinylpyrrolidone | 11 |
| Laurylsulfate de sodium | 3 |
| Granulés totaux | 332 |
| Croscarmellose sodique | 18 |
| Cellulose microcristalline | 53 |
| Stéarate de magnésium | 4 |
| Comprimé total | 407 |

appelée PC-XXIII.

**10.** Comprimé selon l'une quelconque des revendications 1 à 7 ayant la formulation suivante :

| | mg |
|---|---|
| Forme I du composé 1 | 200 |
| Composé 2 pratiquement amorphe | 125 |
| Cellulose microcristalline | 150 |
| Croscarmellose sodique | 34 |
| Laurylsulfate de sodium | 4 |
| Polyvinylpyrrolidone | 15 |
| Stéarate de magnésium | 6 |
| Colorant | 17 |

appelée PC-XVIII.

**11.** Procédé de préparation d'un comprimé selon l'une quelconque des revendications 1 à 9, le procédé comprenant :

a) la granulation en voie humide des composants suivants pour produire un granulé :

a. la forme I du composé 1 ;
b. une dispersion solide comprenant du composé 2 pratiquement amorphe ;
c. une charge ;
d. un délitant ;
e. un tensioactif ; et
f. un liant ;

et
b) la compression des composants suivants pour produire un comprimé :

i. une pluralité de granulés provenant de l'étape a) ;
ii) un délitant ;
iii) une charge ; et
iv) un lubrifiant,

dans lequel la forme I du composé 1 est **caractérisée par** un ou plusieurs pics à 15,4, 16,3 et 14,5 degrés dans un diagramme de diffraction des rayons X sur poudre, et dans lequel le composé 2 pratiquement amorphe a une cristallinité inférieure à 15 %.

**12.** Kit comprenant la composition pharmaceutique orale solide selon la revendication 1 et un agent thérapeutique séparé.

**13.** Kit selon la revendication 12, dans lequel la composition pharmaceutique orale solide et l'agent thérapeutique sont dans des contenants séparés.

**14.** Kit selon la revendication 13, dans lequel les contenants sont des bouteilles, des flacons ou des plaquettes ou une combinaison de ceux-ci.

**Figure 1**

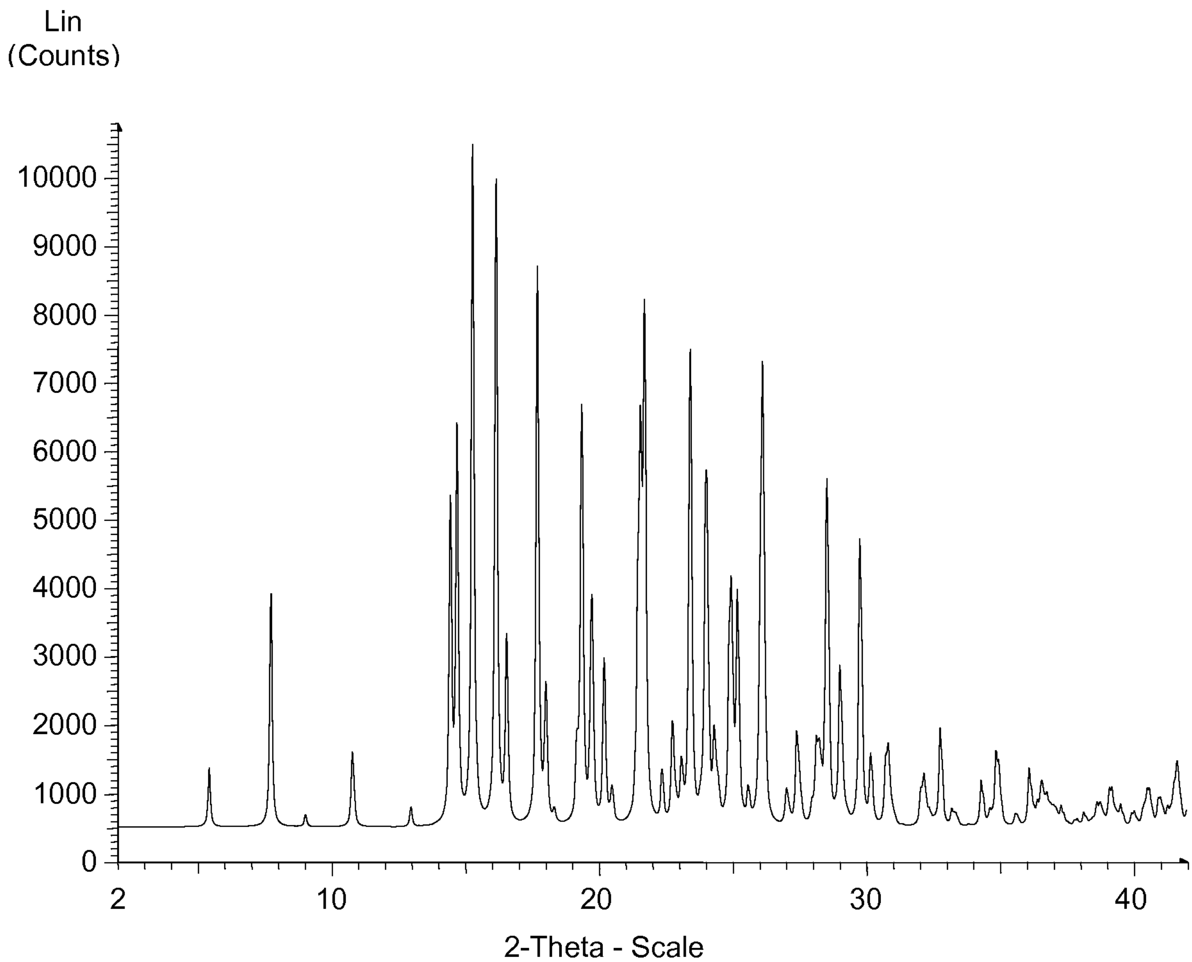
Lin
(Counts)
10000
9000
8000
7000
6000
5000
4000
3000
2000
1000
0
2
10
20
30
40
2-Theta - Scale

Figure 2

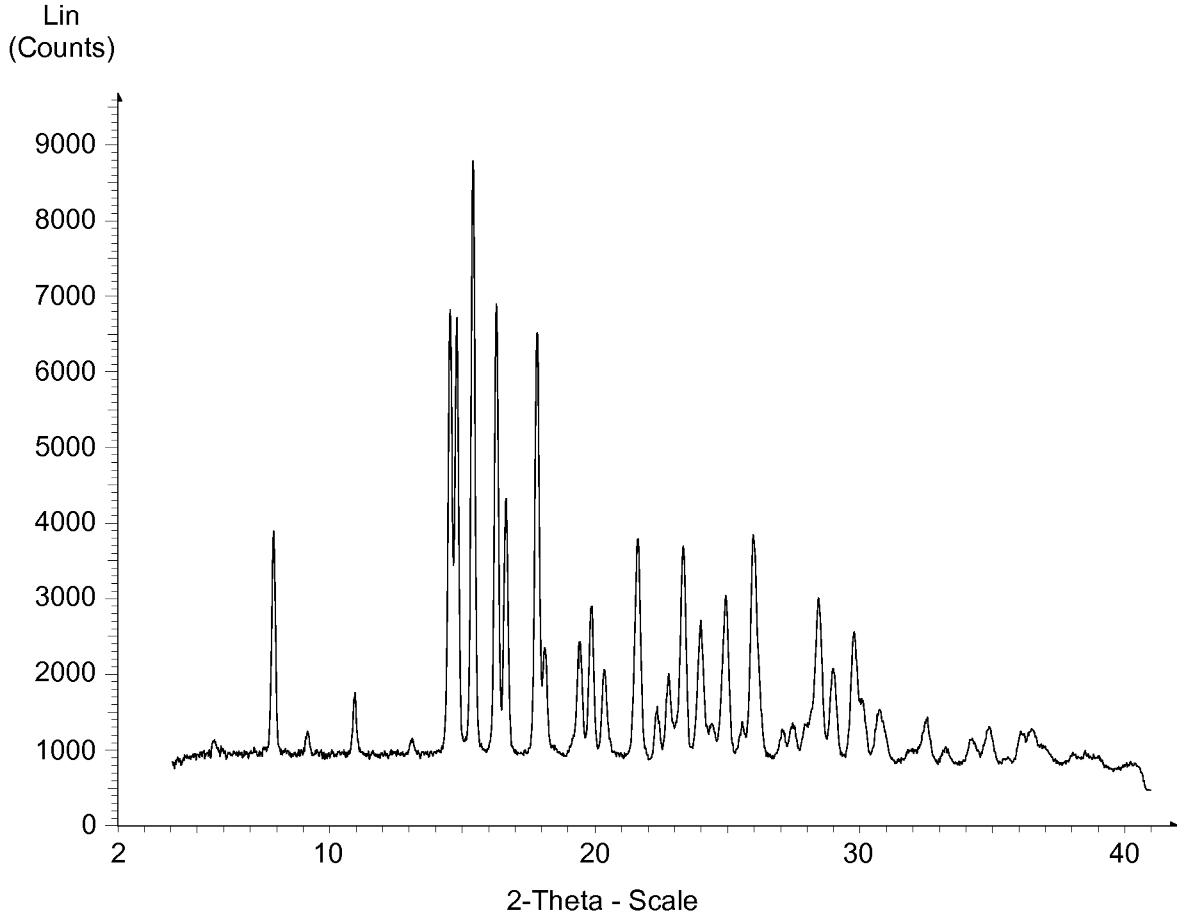
Lin
(Counts)
9000
8000
7000
6000
5000
4000
3000
2000
1000
0
2
10
20
30
40
2-Theta - Scale

Figure 3

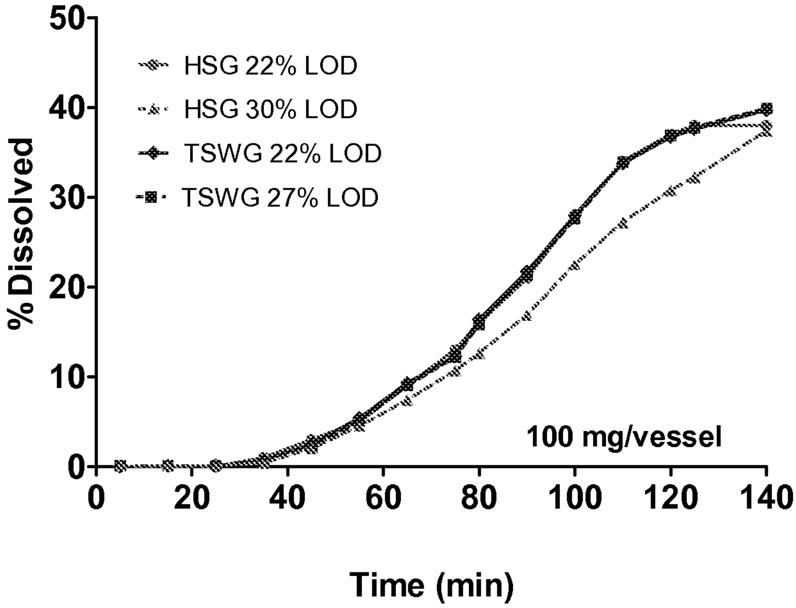
50
40
30
20
10
0
% Dissolved
HSG 22% LOD
HSG 30% LOD
TSWG 22% LOD
TSWG 27% LOD
100 mg/vessel
0
20
40
60
80
100
120
140
Time (min)

**Figure 4**

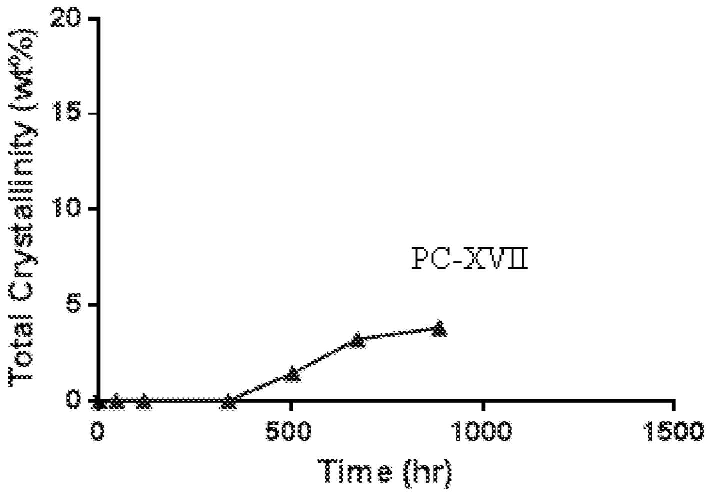
Total Crystallinity (wt%)
20
15
10
5
0
0
500
1000
1500
Time (hr)
PC-XVII

Figure 5

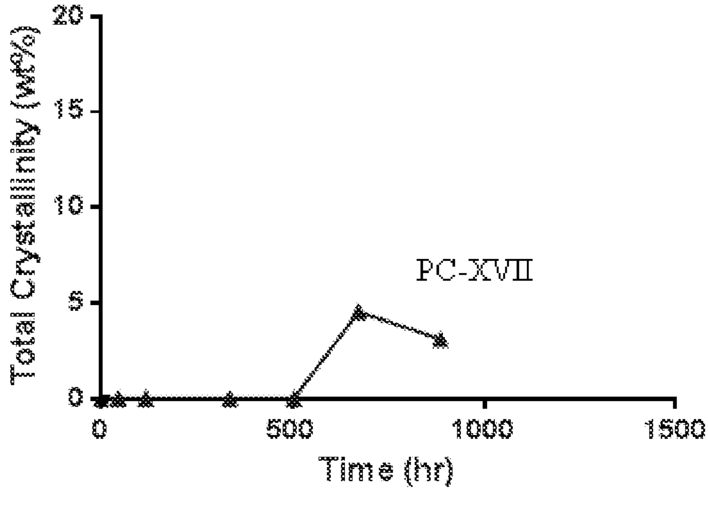
20
15
10
5
0
Total Crystallinity (wt%)
0
500
1000
1500
Time (hr)
PC-XVII

Figure 6

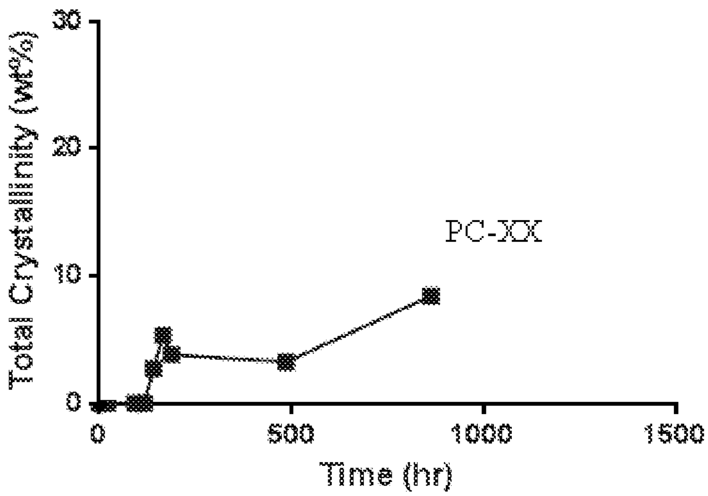

Figure 7

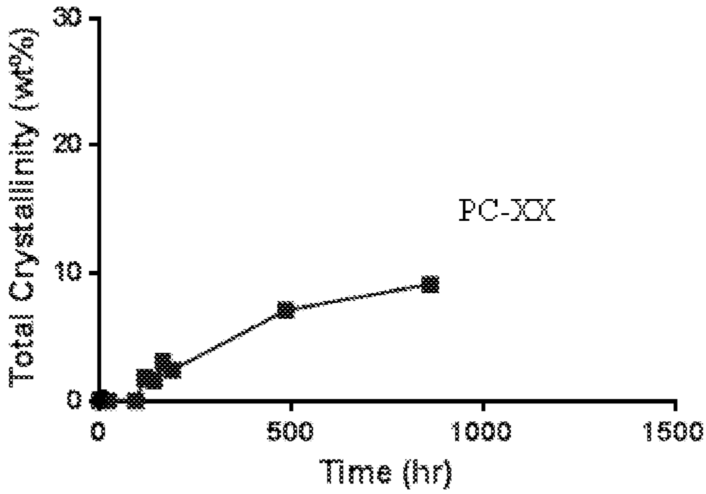

Figure 8

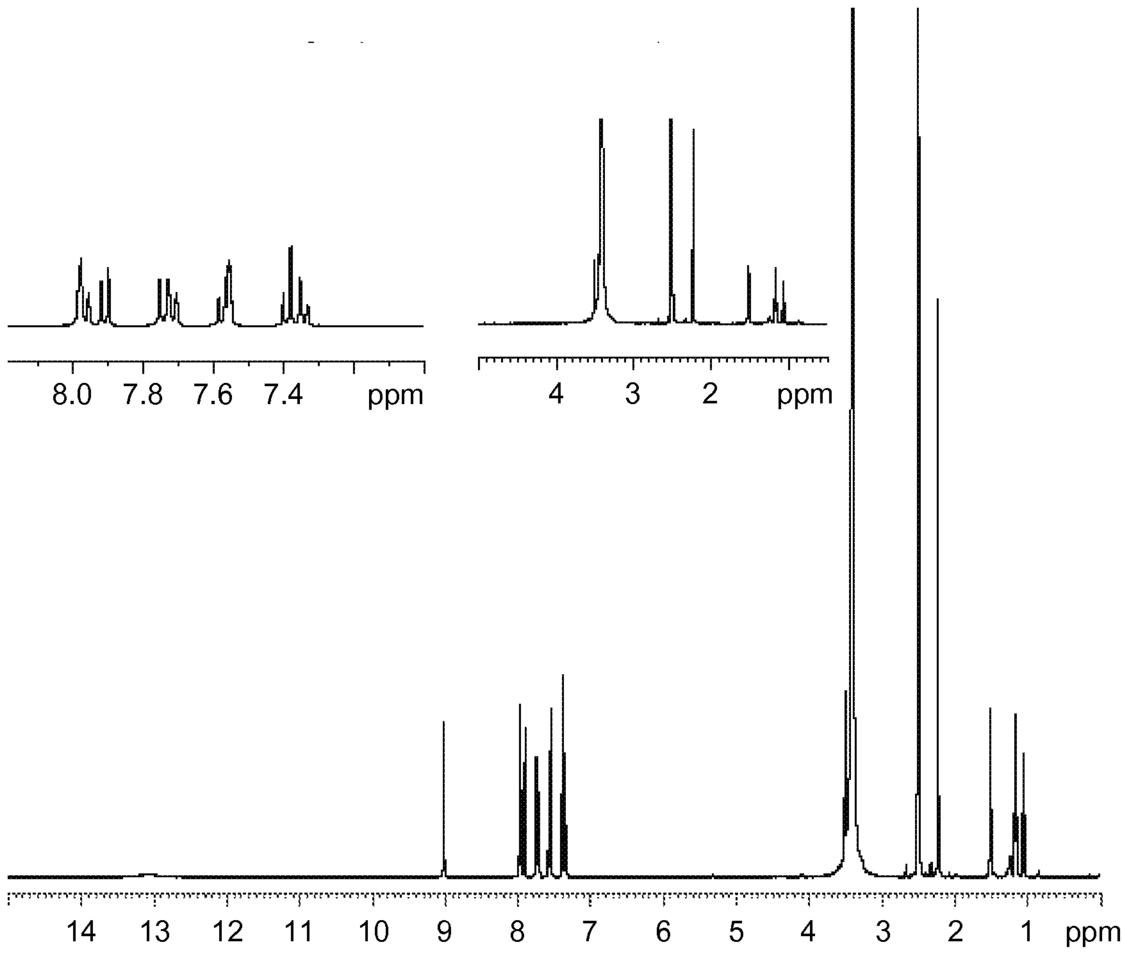

Figure 9

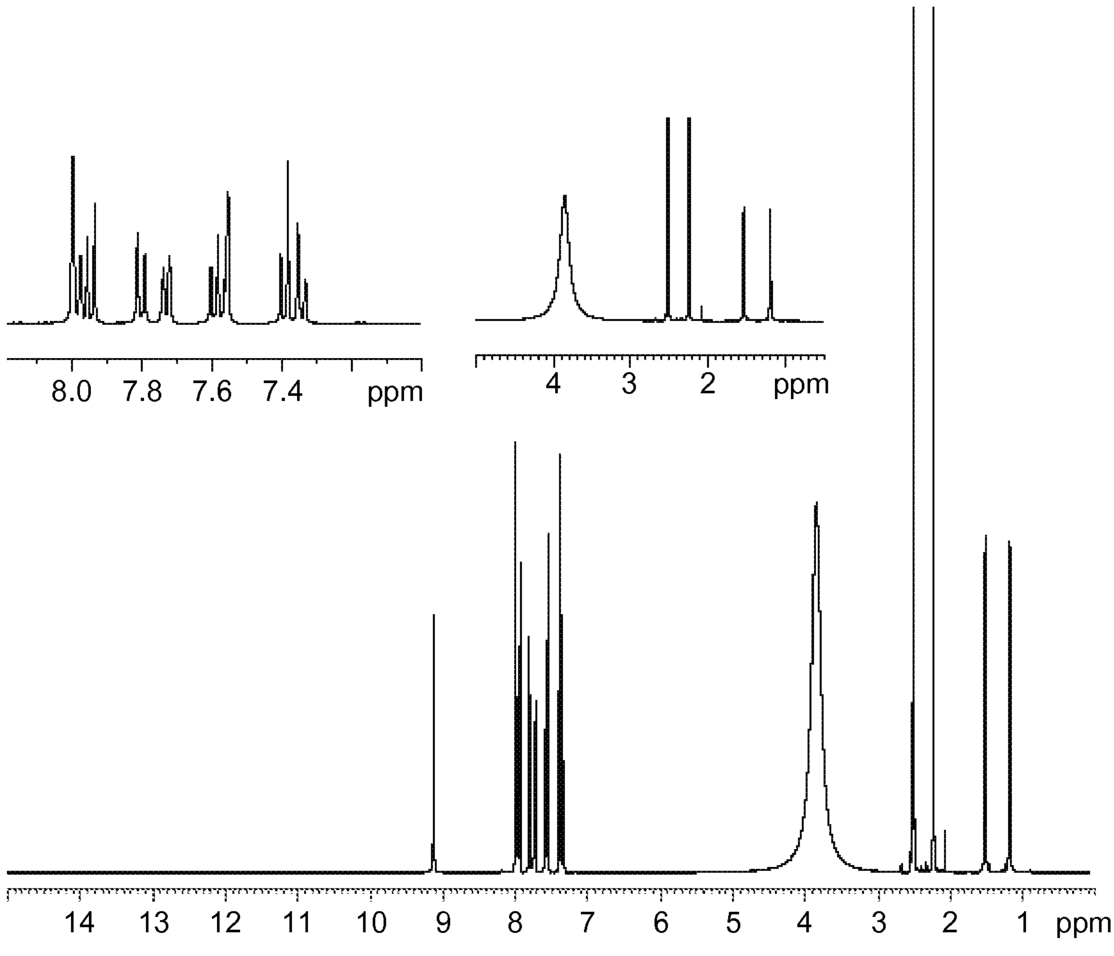

**Figure 10**

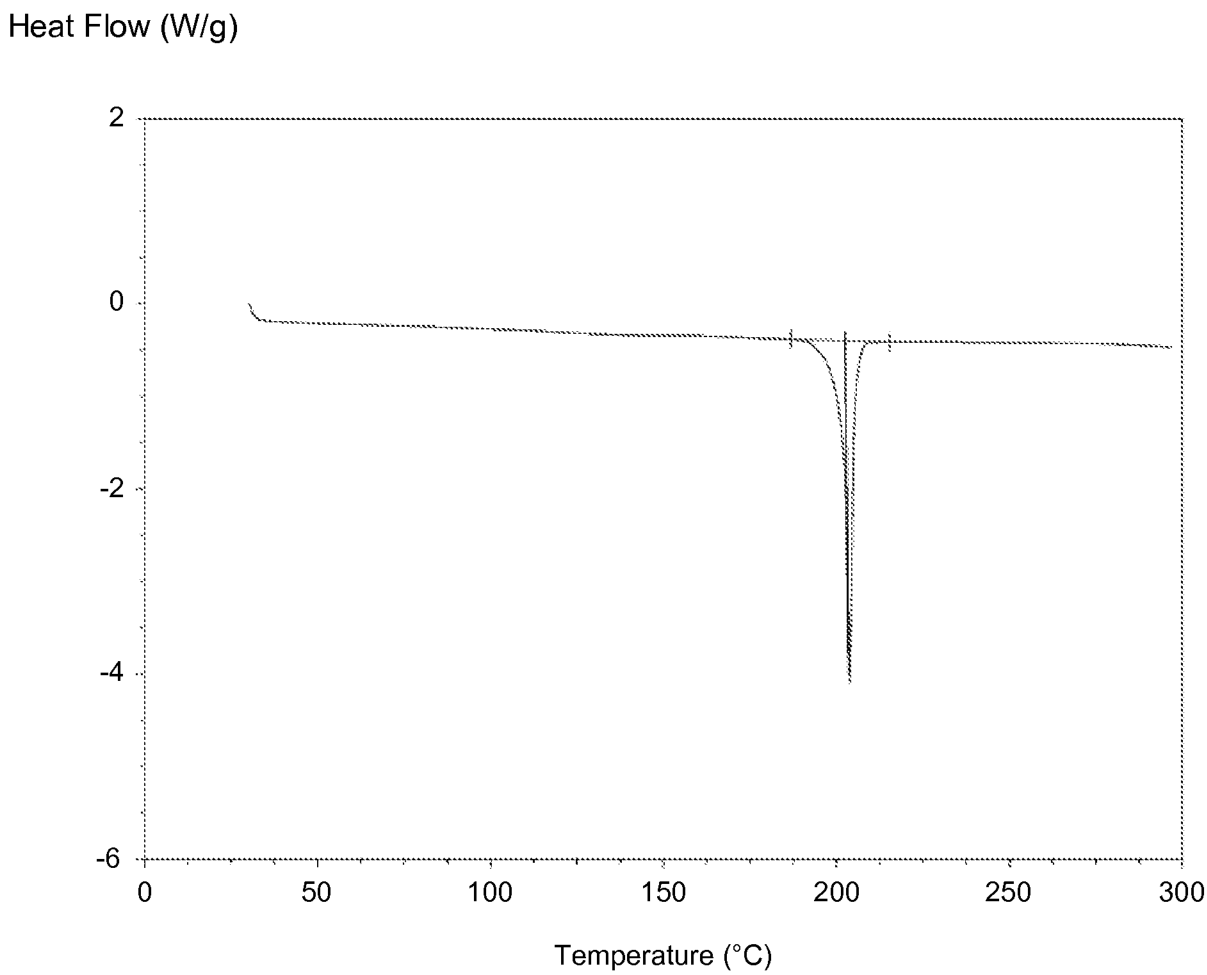

Figure 11

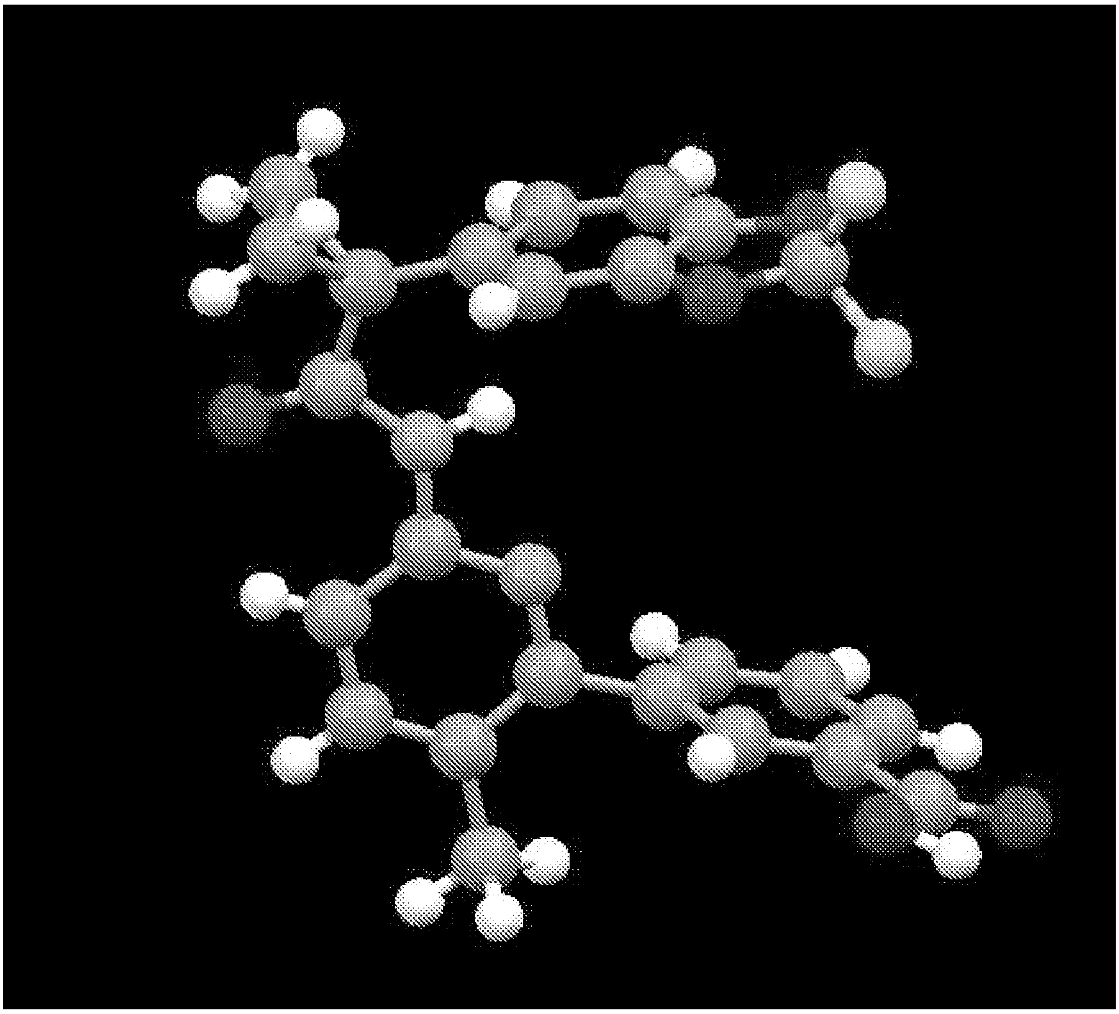

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007056341 A
- US 7741321 B
- WO 2009073757 A
- US 8507534 B
- WO 2006002421 A
- US 7495103 B
- WO 2010019239 A
- US 20100074949 A
- WO 2010019239 A2
- WO 2009073757 A1
- US 20040105820 A
- US 20030144257 A
- US 7407976 B
- US 7645789 B
- US 7659268 B
- US 7671221 B
- US 7691902 B
- US 7754739 B
- US 7776905 B
- US 7973169 B
- US 7977322 B
- US 7999113 B
- US 8227615 B
- US 8299099 B
- US 20060052358 A
- US 20090143381 A
- US 20090170905 A
- US 20090253736 A
- US 20110263654 A
- US 20110251253 A
- WO 2008141119 A
- US 11047361 B
- US 20130116238 A
- US 20050113423 A
- US 20050059687 A
- US 7598412 B
- US 8354427 B
- US 20070105833 A
- US 8242149 B
- US 8314256 B
- US 8399479 B
- US 8188283 B
- US 20100249180 A
- US 20110008259 A
- US 8367660 B
- US 8247436 B
- WO 2011113894 A
- WO 2012035158 A
- WO 2009074575 A
- WO 2011028740 A
- WO 2009150137 A
- WO 2011079087 A
- WO 2008135557 A
- WO 2004028480 A
- WO 2004110352 A
- WO 2005094374 A
- WO 2005120497 A
- WO 2006101740 A
- US 7202262 B
- US 6992096 B
- US 20060148864 A
- US 20060148863 A
- US 20060035943 A
- US 20050164973 A
- WO 2006110483 A
- WO 2006044456 A
- WO 2006044682 A
- WO 2006044505 A
- WO 2006044503 A
- WO 2006044502 A
- WO 2004091502 A
- WO 2004080972 A
- WO 2004111014 A
- WO 2005035514 A
- WO 2005049018 A
- WO 2006099256 A
- WO 2006127588 A
- WO 2007044560 A


### Non-patent literature cited in the description


- **CUTTING, G. R. et al.** *Nature,* 1990, vol. 346, 366-369
- **DEAN, M et al.** *Cell,* 1990, vol. 61 (863), 870
- **KEREM, B-S. et al.** *Science,* 1989, vol. 245, 1073-1080
- **KEREM, B-S et al.** *Proc. Natl. Acad. Sci. USA,* 1990, vol. 87, 8447-8451
- **QUINTON, P. M.** *FASEB J,* 1990, vol. 4, 2709-2727
- **DALEMANS et al.** *Nature Lond,* 1991, vol. 354, 526-528

- **PASYK ; FOSKETT.** *J. Cell. Biochem.,* 1995, vol. 270, 12347-50
- Remington: The Science and Practice of Pharmacy. Lippincott Williams & Wilkins, 2003
- The United States Pharmacopeia. 1995, 1843-1844
- Perry's Chemical Engineering Handbook. McGraw-Hill, 1984
- Atomization and Spray-Drying. **MARSHALL.** Chem. Eng. Prog. Monogr. Series. 1954, vol. 2
- **ANSEL et al.** Pharmaceutical Dosage Forms And Drug Delivery Systems. Lippincott Williams & Wilkins, 1999
- The Handbook of Pharmaceutical Excipients. American Pharmaceuticals Association, 2003
- **GIBSON.** Pharmaceutical Preformulation And Formulation. CRC Press, 2001
- Dissolution. United States Pharmacopoeia. United States Pharmacopeial Convention, Inc, 2005
- **SHELDRICK, G.M.** *Acta Cryst.,* 2008, vol. A64, 112-122
- **GALIETTA, L.J.V ; LANTERO, S. ; GAZZOLO, A. ; SACCO, O ; ROMANO, L. ; ROSSI, G.A ; ZEGARRA-MORAN, O.** *In Vitro Cell. Dev. Biol.,* 1998, vol. 34, 478-481
- **RAE, J. ; COOPER, K. ; GATES, P. ; WATSKY, M.** *J. Neurosci. Methods,* 1991, vol. 37, 15-26
- **DALEMANS, W. ; BARBRY, P ; CHAMPIGNY, G. ; JALLAT, S ; DOTT, K. ; DREYER, D. ; CRYSTAL, R.G. ; PAVIRANI, A ; LECOCQ, J-P. ; LAZDUNSKI, M.** *Nature,* 1991, vol. 354, 526-528
- **GONZALEZ, J. E ; R. Y. TSIEN.** Voltage sensing by fluorescence resonance energy transfer in single cells. *Biophys J,* 1995, vol. 69 (4), 1272-80
- **GONZALEZ, J. E. ; R. Y. TSIEN.** Improved indicators of cell membrane potential that use fluorescence resonance energy transfer. *Chem Biol,* 1997, vol. 4 (4), 269-77
- **GONZALEZ, J. E. ; K. OADES et al.** Cell-based assays and instrumentation for screening ion-channel targets. *Drug Discov Today,* 1999, vol. 4 (9), 431-439